# EUROPEAN PATENT APPLICATION

(11) **EP 1 752 040 A1**
(43) Date of publication of application: **14.02.2007**
(21) Application number: 05017651.0
(22) Date of filing: 12.08.2005
(51) Int. Cl.: A01K 67/027, C07K 14/47, C12N 15/85

(54) **Isolation of the t-complex distorters and applications thereof**

(71) Applicant: Max-Planck-Gesellschaft zur Förderung der Wissenschaften e.V., Berlin (DE)
(72) Inventor: Herrmann, Bernhard, 14163 Berlin (DE); Bauer, Hermann, 14195 Berlin (DE)
(74) Representative: Vossius & Partner

(57) **Abstract**

The present invention relates to a method for producing a transgenic non human male animal, preferably a mammal, fish, bird or insect, wherein the transgene(s) confer(s) a change in the transmission ratio of (a) genetic trait(s) to the offspring of said non human male animal, preferably mammal, fish, bird or insect to a non-Mendelian ratio, said method comprising introducing (a) a first nucleic acid molecule encoding an expression product with a Responder function into a chromosome of a non-human germ cell, (fertilized) egg cell, embryonic cell or a cell derived therefrom, of the same species as the transgenic male to be prepared, said chromosome containing or conferring said genetic trait(s), thereby linking on said chromosome said Responder function to the genetic trait(s); and (b) at least one second nucleic acid molecule encoding an expression product with a Distorter function into (a) chromosome(s) of a non-human germ cell, (fertilized) egg cell, embryonic cell or a cell derived therefrom, of the same species as the transgenic male to be prepared, wherein said expression product with a Distorter function is a factor involved in G protein signaling, wherein said first nucleic acid molecule encoding an expression product with Responder function and said at least one second nucleic acid molecule encoding an expression product with a Distorter function are introduced into the same or different chromosomes.

## Description

The present invention relates to a method for producing a transgenic non human male animal, preferably a mammal, fish, bird or insect, wherein the transgene(s) confer(s) a change in the transmission ratio of (a) genetic trait(s) to the offspring of said non human male animal, preferably mammal, fish, bird or insect to a non-Mendelian ratio, said method comprising introducing (a) a first nucleic acid molecule encoding an expression product with a Responder function into a chromosome of a non-human germ cell, (fertilized) egg cell, embryonic cell or a cell derived therefrom, of the same species as the transgenic male to be prepared, said chromosome containing or conferring said genetic trait(s), thereby linking on said chromosome said Responder function to the genetic trait(s); and (b) at least one second nucleic acid molecule encoding an expression product with a Distorter function into (a) chromosome(s) of a non-human germ cell, (fertilized) egg cell, embryonic cell or a cell derived therefrom, of the same species as the transgenic male to be prepared, wherein said expression product with a Distorter function is a factor involved in G protein signaling, wherein said first nucleic acid molecule encoding an expression product with Responder function and said at least one second nucleic acid molecule encoding an expression product with a Distorter function are introduced into the same or different chromosomes.

Furthermore, the invention relates to a method for producing a transgenic non human male animal, preferably a mammal, fish, bird or insect, wherein the transgene(s) confer(s) a change in the transmission ratio of (a) genetic trait(s) to the offspring of said non human male animal, preferably mammal, fish, bird or insect to a non-Mendelian ratio, said method comprising introducing (a) a first nucleic acid molecule encoding an expression product with a Responder function into a chromosome of a non-human germ cell, (fertilized) egg cell, embryonic cell or a cell derived therefrom, of the same species as the transgenic male to be prepared, said chromosome containing or conferring said genetic trait(s), thereby linking on said chromosome said Responder function to the genetic trait(s); and (b) at least one second nucleic acid molecule encoding an expression product directed against the Distorter function into (a) chromosome(s) of a non-human germ cell, (fertilized) egg cell, embryonic cell or a cell derived therefrom, of the same species as the transgenic male to be prepared, wherein said Distorter function is an expression product which is encoded by a nucleic acid molecule encoding an expression product with a Distorter function, wherein said expression product with a Distorter function is a factor involved in G protein signaling; and/or (c) a second nucleic acid molecule for inactivation of the Distorter function by homologous recombination, wherein said second nucleic acid molecule for inactivation of the Distorter function by homologous recombination is at least partially identical to said nucleic acid molecule encoding an expression product with a Distorter function, wherein said first nucleic acid molecule encoding an expression product with a Responder function and said at least one second nucleic acid molecule encoding an expression product directed against the Distorter function and/or said second nucleic acid molecule for inactivation of the Distorter function by homologous recombination are introduced into the same or different chromosomes, thereby partially or completely inactivating the Distorter function.

In this specification, a number of documents are cited. The disclosure content of these documents including manufacturers' manuals is herewith incorporated by reference in its entirety.

The mouse t-complex, a region of approximately 12 cM genetic distance on the proximal part of chromosome 17, contains several loci acting in concert to produce a phenomenon called transmission ratio distortion (TRD). The latter designation indicates the fact that the so-called t-haplotype form of this chromosomal region has a selective advantage over the wild type form in that it is transmitted to the offspring at non-Mendelian ratios of up to 99%. This transmission at non-Mendelian ratio is achieved by the concerted action of at least five loci, the *t complex Distorters* Tcd1a and Tcd1b (D1a, D1b), Tcd2 (D2) and Tcd3 (D3), and the *t complex responder, Tcr* (R^{t})(Lyon 1984, Lyon et al 2000). More Distorters have been postulated (Silver and Remis 1987).

According to Lyon's model (Lyon 1986) which formally explains the genetic interactions of these loci, D1, D2 and D3 act strongly and harmfully on the wild type allele of the Responder and weakly on the t form of the Responder (R^{t}), leading to distortion in favor of R^{t}. R^{t} might protect sperm carrying it from this harmful action of the Distorters. The Distorters act in trans while the Responder acts in cis. This means that the chromosome, which contains R^{t} is transmitted at non-Mendelian ratio to the offspring. If D2 or all the Distorters are present, the chromosome containing R^{t} is transmitted at a frequency of more than 50% up to 99% to the offspring. If no Distorter or only D1 or D3 are present, however, the chromosome containing R^{t} is transmitted at less than 50% to the offspring (as low as 12%, "low" phenotype). The Distorters are only transmitted at ratios over 50% if they are tightly linked to R^{t}. The trans-acting and cis-acting properties of the Distorters and the Responder, respectively, have been demonstrated by the transmission ratio properties of so-called partial t-haplotypes, which carry only a subset of the above named loci.

Genetic mapping of molecular markers on partial t-haplotypes allowed a rough localization of D 1 a, D 1 b, D2, D3 and R^{t} to subregions of the T/t-complex and relative to these molecular markers (Lyon 1984; (Fox, Martin et al. 1985); (Herrmann, Bucan et al. 1986); (Silver and Remis 1987); (Bullard, Ticknor et al. 1992); Lyon et al 2000). Only one locus, R^{t} could be mapped fairly precisely to a region of appr. 200 kb, the so-called T66B region (renamed later Leh66B ; Fox, Martin et al. 1985; (Schimenti, Vold et al. 1987); (Nadeau, Varnum et al. 1989); (Rosen, Bullard et al. 1990); (Bullard, Ticknor et al. 1992)). The genomic region T66B has been cloned molecularly and analyzed. A partial restriction map covering approximately 145kb of it has been published ((Schimenti, Void et al. 1987); (Rosen, Bullard et al. 1990); (Bullard, Ticknor et al. 1992)).
An extensive and careful search of this region for genes expressed during spermatogenesis led to the identification of a fusion gene expressed during spermiogenesis, the haploid phase of sperm development. Molecular and genetic analyses showed that the fusion gene encoding a mutant form of a novel protein kinase, Smok, represents Tcr (Herrmann, Koschorz et al. 1999)). Transgene analyses demonstrated that Smok^{Tcr}, in combination with Tcd loci, distorts the transmission ratio of itself and preferably closely linked genetic traits. Co-segregation of a transgene construct encoding Smok^{Tcr} with the Y-chromosome resulted in sex ratio distortion (Herrmann, Koschorz et al. 1999).
T complex Distorters could only be mapped very roughly to large chromosomal subregions of several megabase each in size due to suppression of meiotic recombination between the t-haplotype and the wild type chromosome. Rare recombinants have occurred between these chromosomes allowing separation of the different loci, but molecular access to the Distorter loci is extremely difficult. Several attempts to isolate t-Distorters have been reported, though none of the candidates has been verified by genetic means (for review see (Schimenti 2000) (Lyon 2003), Systematic approaches using deletion mapping in the Tcd1 region and candidate gene isolation also has failed to identify a Distorter at the molecular level (Planchart, You et al. 2000) (Lyon, Schimenti et al. 2000).
The combined teachings of the prior art thus did not provide any clue how the genetic elements responsible for the Distorter phenotype might be identified and, hence, did not disclose any means of applicability related to said genetic entity. A preferred goal would be the targeted transmission ratio distortion using, as a basis, the molecular entity of the Distorter(s). The technical problem underlying the present invention therefore was to overcome these long standing prior art difficulties and to provide such means.

The solution to said technical problem is achieved by providing the embodiments characterized in the claims

The present invention relates to a method for producing a transgenic non human male animal, preferably a mammal, fish, bird or insect, wherein the transgene(s) confer(s) a change in the transmission ratio of (a) genetic trait(s) to the offspring of said non human male animal, preferably mammal, fish, bird or insect to a non-Mendelian ratio, said method comprising introducing (a) a first nucleic acid molecule encoding an expression product with a Responder function into a chromosome of a non-human germ cell, (fertilized) egg cell, embryonic cell or a cell derived therefrom, of the same species as the transgenic male to be prepared, said chromosome containing or conferring said genetic trait(s), thereby linking on said chromosome said Responder function to the genetic trait(s); and (b) at least one second nucleic acid molecule encoding an expression product with a Distorter function into (a) chromosome(s) of a non-human germ cell, (fertilized) egg cell, embryonic cell or a cell derived therefrom, of the same species as the transgenic male to be prepared, wherein said expression product with a Distorter function is a factor involved in G protein signaling, wherein said first nucleic acid molecule encoding an expression product with Responder function and said at least one second nucleic acid molecule encoding an expression product with a Distorter function are introduced into the same or different chromosomes.

Furthermore, the invention relates to a method for producing a transgenic non human male animal, preferably a mammal, fish, bird or insect, wherein the transgene(s) confer(s) a change in the transmission ratio of (a) genetic trait(s) to the offspring of said non human male animal, preferably mammal, fish, bird or insect to a non-Mendelian ratio, said method comprising introducing (a) a first nucleic acid molecule encoding an expression product with a Responder function into a chromosome of a non-human germ cell, (fertilized) egg cell, embryonic cell or a cell derived therefrom, of the same species as the transgenic male to be prepared, said chromosome containing or conferring said genetic trait(s), thereby linking on said chromosome said Responder function to the genetic trait(s); and (b) at least one second nucleic acid molecule encoding an expression product directed against the Distorter function into (a) chromosome(s) of a non-human germ cell, (fertilized) egg cell, embryonic cell or a cell derived therefrom, of the same species as the transgenic male to be prepared, wherein said Distorter function is an expression product which is encoded by a nucleic acid molecule encoding an expression product with a Distorter function, wherein said expression product with a Distorter function is a factor involved in G protein signaling; and/or (c) a second nucleic acid molecule for inactivation of the Distorter function by homologous recombination, wherein said second nucleic acid molecule for inactivation of the Distorter function by homologous recombination is at least partially identical to said nucleic acid molecule encoding an expression product with a Distorter function, wherein said first nucleic acid molecule encoding an expression product with a Responder function and said at least one second nucleic acid molecule encoding an expression product directed against the Distorter function and/or said second nucleic acid molecule for inactivation of the Distorter function by homologous recombination are introduced into the same or different chromosomes, thereby partially or completely inactivating the Distorter function.

The term "genetic trait" relates to a heritable feature or characteristic of an organism encoded by (a) nucleic acid molecule(s) contained in its genome, comprising naturally occurring characteristics as well as (a) feature(s) encoded by (a) nucleic acid molecule(s) engineered in vitro, which has/have been introduced into its genome.

The term "confer a change in the transmission ratio of a genetic trait(s) to the offspring of said non human male animal, preferably mammal, fish, bird or insect to a non-Mendelian ratio" as used in accordance with the present invention refers to changing the transmission ratio of (a) genetic trait(s) from the parents to their offspring to ratios markedly deviating from the expected Mendelian ratio of 50% (equal transmission). "Markedly deviating" in connection with the present invention means that the ratios might be less than 50 percent, preferably less than 40%, more preferably less than 30%, even more preferably less than 20% and most preferably less than 10% (reduced transmission) or might be at least 60%, more preferably at least 70 %, even more preferably at least 80% and most preferably at least 90 % (enhanced transmission).

The term "said chromosome containing said genetic trait(s)" as used in connection with the present invention means that the genetic trait is part of the chromosome and will then be transmitted together with said chromosome through the germline.

The term "said chromosome conferring said genetic trait(s)" as used in connection with the present invention means that the entire chromosome is to be considered as the genetic trait therefore the transmission ratio of said genetic trait is also changed when the entire chromosome will be transmitted through the germline.

The term "Responder function" as used in connection with the present invention refers to the unique property of a Responder to distort the transmission ratio of itself and (a) closely linked genetic trait(s) to non-Mendelian ratios, i.e. a marked deviation from 50%.

The term "Distorter function" as used in connection with the present invention refers to the potency of (a) Distorter(s) to enhance or reduce the transmission ratio of a Responder and (a) closely linked genetic trait(s), wherein the transmission ratio of the Responder and (a) closely linked genetic trait(s) markedly deviates from the Mendelian ratio.

The term "nucleic acid molecule encoding an expression product with Distorter function/with Responder function" relates to nucleic acid molecules wherein the deduction of the amino acid sequence of the nucleic acid molecules used in connection with the method of the present invention allows the conclusion that the (poly)peptide is the expression product that contributes to the Distorter/Responder function. However, it is not excluded that the mRNA contributes to or triggers said Distorter/Responder function. Also, it is envisaged in accordance with the present invention that the expression level, stage of expression during spermatogenesis or the copy number of said nucleic acid molecule results in or contributes to the Distorter/Responder function. Therefore, in a preferred embodiment of the nucleic acid molecule used in connection with the method of the invention said expression product is an RNA or a (poly)peptide.

The term "(poly)peptide" as used in the present invention describes a group of molecules which comprise the group of peptides, as well as the group of polypeptides. The group of peptides is consisting of molecules with up to 30 amino acids, the group of polypeptides is consisting of molecules with more than 30 amino acids. Furthermore, the term "protein" as used in connection with the present invention is to be considered identical with the term "(poly)peptide".

The terms "Distorter" or "Responder" therefore as used in connection with the present invention are to be considered in their broadest sense. Preferably, the terms refer to the (poly)peptide with Distorter or Responder function encoded by the corresponding nucleic acid molecules. Also, as mentioned above, said term might refer to the corresponding mRNAs or the nucleic acid molecule encoding the (poly)peptide with Distorter or Responder function.

Preferably, the nucleic acid molecule encoding a Distorter function is selected from the group consisting of SEQ ID NOs 1 and 2 (mouse wildtype Tagap1); SEQ ID NOs 3 to 6 (mouse Tagap^{t1}; Tagap^{t2}; Tagap^{t3}; Tagap^{t4} (Tcd1a)); SEQ ID NOs 7 and 8 (homop sapiens and Rattus Tagap); SEQ ID NOs 9 to 12 (mouse wildtype Fgd2); SEQ ID NO 12 (mouse Fgd2^{t6/w5;} (Tcd2)) and SEQ ID NOs 13 and 14 (mouse and human Tiam2 (Tcd 1 b)).

It is also preferred that the nucleic acid molecule encoding a Responder function is as shown in SEQ ID NO 15 or 16 (Smok^{Tcr}, Tcr, R^{t}).

The term "factors involved in G protein signaling" as used in connection with the present invention refers to any factor and preferably any protein that is a part of a G protein signaling cascade and in particular to members of the GTPase superfamily. For example, said members comprise trimeric G proteins and monomeric GTPases, and (poly)peptides triggering, controlling, modifying (a) signal pathway(s) involving small GTPases or regulated by (a) signal pathway(s) involving small GTPases.

The term "homologous recombination" refers to gene targeting of a nuclear gene locus of interest by integration of a nucleic acid molecule construct containing (a) genomic fragment(s) of said gene thereby altering the DNA sequence of said nuclear gene locus. It is preferred that by introducing said nucleic acid molecule construct into the nuclear gene locus the gene activity of the Distorter is down-regulated or abolished.

Within the meaning of the present invention, the term "directed against the Distorter function" means that the nucleic acid molecule or the expression product of said nucleic acid molecule or the antibody directed against the Distorter reduces or interferes with the activity of the expression product(s).

The term "thereby partially or completely inactivating the Distorter function" as used in connection with the present invention refers to interfering with the gene activity of the Distorter by destruction of the mRNA, inhibition of translation of the mRNA, inhibition of the protein or enzymatic activity, or by other mechanisms allowing down-regulation or abolishment of the gene or protein activity of the Distorter. For example, in any of the above interferences partial inactivation means inactivation of at least 50%, preferably of at least 60%, more preferably of at least 70%, even more preferably of at least 80%, even more preferably of at least 90%, even more preferably of at least 95% and most preferably 100% (complete inactivation) A number of methods and assays which are known to the person skilled in the art allowing measuring down-regulation of transcript or protein levels, inhibition of protein translation or down-regulation of protein activity (Sambrook J. 1989). The skilled person can devise an assay wherein for example the amount of Distorter transcripts in cells containing said Distorter and expressing a nucleic acid molecule directed against said Distorter is compared to cells containing said Distorter but not said nucleic acid molecule directed against said Distorter. Likewise, the protein expression level of cells expressing said Distorter can be compared to cells expressing in addition a nucleic acid molecule allowing down-regulation or abolishment of the gene or protein activity of the Distorter for instance by western blot analysis using an antibody binding to the protein product of said Distorter. The protein activity of the expression product of a Distorter allele which has been altered in vitro in order to interfere with the protein activity of said wild type Distorter product can be compared to the activity of said wild type Distorter protein using in vitro activity assays such as for example those known in the art devised for assaying the activity of GAP or GEF proteins on target GTPases, such as for example, the one shown herein below, the method comprising expression of the Distorter protein in vitro or in bacterial cells. Expression products derived from dominant negative alleles can be assayed in mixing experiments in the presence of the wild type protein for its ability to interfere with the activity of the wild type protein. Furthermore, the activity of constitutively active proteins can be compared to the activity of the wild type protein comprising relating the activity of either protein to the protein amounts used in the assay.

The term "partially identical", as used herein, means in a first alternative that the genomic fragments used for integrating the nucleic acid molecule construct by homologous recombination are completely identical with the target nucleic acid molecule encoding an expression product with Distorter function. In this alternative the overall construct is partially identical because the target nucleic acid molecule encoding an expression product with Distorter function is not identical with the sequence in the construct used for the inactivation. Alternatively, even said genomic fragments may not be completely identical with the target nucleic acid molecule encoding an expression product with Distorter function but are sufficiently identical to allow recombination.
As outlined above and in other terms, the invention solves the recited technical problem by providing a reproducible method for changing the transmission ratio of genetic traits in non-human mammals, birds, fish or insects. In particular, as mentioned above, prior art methods failed to identify a Distorter which, however, is needed for carrying out the method of the present invention.
In the prior art, all t-Distorter candidate genes which have been reported had been identified by the criteria that they were a) located within the t-complex region and b) play a role in sperm specific functions or are primarily expressed in sperm cells, such as Tctex1, Tctex2, Tcte2 and Tcp11 (Fraser and Dudley, 1999). This obvious assumption that t-Distorters are likely involved in sperm specific functions was used as an aid for preselection of likely candidates from the hundreds of genes located in the t-complex region, but turned out to be false. Additionally, it was known in the prior art that transmission ratio distortion relates to sperm motility and that the Responder relates to a Smok kinase (Herrmann et al, 1999), from which a person skilled in the art might have derived that factors involved in calcium signaling or cAMP signaling might be involved in the Distorter phenotype. However, the prior art did not give any clue whatsoever that factors involved in G protein signaling might function as a Distorter. The method of the present invention therefore for the first time makes use of nucleic acid molecules encoding such factors which are involved in G protein signaling for the above-indicated purpose.

The method of the present invention is based on the fact that in mouse the t-haplotype chromosome is transmitted at non-Mendelian ratio (significantly higher or lower than 50%) to the offspring. This phenomenon involves Tcr (Responder) and several Tcd (Distorter) loci, wherein the Tcd loci in the t-haplotype, that is the mutant forms, enhance the transmission ratio of Tcr. In the wild-type form, on the other hand, the Tcd+ loci reduce the transmission ratio of Tcr (the "low" phenotype). In the prior art, Tcd loci could not be localized precisely by chromosomal mapping due to recombination suppression between the t-haplotype and the wild type chromosome. Thus the coarse localization of Tcd loci to regions of several megabases in size each prevented the identification of t-Distorters. Even deletion mapping of Tcd1 did not allow identification of this factor (Lyon et al 2000; Schimenti et al 2000).

Thus, these prior art difficulties had to be overcome in order to solve the ignorance of the molecular nature of a t-Distorter. The present invention not only makes use of Distorters which are factors involved in G protein signaling, but on top of this solved the problem which was in the prior art that the isolation of a Distorter could not be achieved. In the course of isolating a nucleic acid molecule which encodes one of the Distorters which can be used in connection with the present invention, in particular Tagap1, the inventors isolated a specific fusion gene, which showed similarity to FGF receptor oncogene partner (Fop) and is highly expressed in testis of wild type mice, but not of mice carrying the t-haplotype, suggesting that it is related to transmission ratio distortion. However, the inventors could demonstrate by gene targeting and genetic testing that this fusion gene did not show any Distorter activity. Nevertheless, the inventors did not turn to another candidate, but continued the study of this locus. Southern blot analysis indicated that this gene or a part thereof was present in a second locus on the chromosome, but there was no indication that that second gene or gene fragment was expressed. Rapid amplification of cDNA end (RACE) technology was attempted to complete the 5'-region of the second gene transcript, but these experiments produced the 5'end sequence of the known fusion gene instead because this gene is highly expressed in testis. Only after several months of unsuccessful trials eventually a different 5'- end was identified, which then led to the isolation of Tagap1. The transcript of the missing locus, Tagap1, per se was only very weakly expressed in testis and did not hint to a Distorter function either. Only by completing the entire gene the inventors could recognize the missing (second) gene as a gene involved in G protein signalling. Gene targeting and genetic testing demonstrated that Tagap1 is able to alter the transmission ratio of a t-haplotype carrying Tcr. However, the targeted allele reduced the transmission ratio, in contrast to the teachings of the prior art, since Lyon had shown that a deletion of Tcd1 on the wild type chromosome enhanced the transmission ratio (Lyon 1992). Large efforts involving extensive genetic analyses and the production and analyses of transgenic lines had to be undertaken by the inventors to finally show that the t-loci of Tagap1 constitute a Distorter which enhances the transmission ratio of a t-haplotype and which must be different from the t-Distorter identified by Lyon by genetic means using the deletion chromosome T^{22H}.

From the above, the inventors hypothesized that further factors involved in G protein signaling might be involved in Distorter function as for example shown in Example 3 and could identify further factors in the cascade showing Distorter function. Hence, the present invention for the first time links G protein signalling to the phenomenon of transmission ratio distortion.

The method of the present invention comprises deriving an adult animal from said non human germ cell, fertilized egg cell, embryonic cell or cell derived therefrom containing the nucleic acid molecules as defined in the present invention. The fertilized egg cell or an embryo into which said embryonic cell has been introduced (thereby forming a chimera), or the fertilized egg or zygote derived from introducing the nucleus of the cell containing the nucleic acid molecules as defined in the present invention into an egg cell or zygote whose genome has been removed (thereby forming an embryo containing said the nucleic acid molecules as defined in the present invention in its genome), is/are transferred into a foster mother and the embryo let develop to term.

The offspring of the foster mother are then determined for the integration of the nucleic acid molecules as described in the present invention and the sex is determined by methods known to the person skilled in the art. Such methods comprise for example genotyping by PCR and further methods as also described below.

The male offspring can further be characterized by visual inspection of outer genitalia and also by detecting male specific markers. Said techniques are also known to the person skilled in the art.

All methods employed for deriving an adult animal are known in the prior art and further described, where applicable, in the specification of the present invention.

The transgenic non-human male, fish, bird or insect produced by the method of the present invention can be prepared in at least two alternative ways. The nucleic acid molecules as defined in the present invention can be introduced into the same non-human germ cell, (fertilized) egg cell, embryonic cell or a cell derived therefrom and the adult organism let develop as described above. Alternatively, the nucleic acid molecules as defined in the present invention can be introduced into different cells thereby producing two different adult organisms with the same methods as described above. The male and female of said two organisms are then crossed and the offspring is analyzed for the nucleic acid molecule as described in the present invention and the male is characterized as described above.

As mentioned, the techniques involved in animal breeding and animal crossing and the techniques involved in transgenesis are known to the person skilled in the art and, where applicable, are described in the present specification.

Alternatively, in the case that the investigator wishes to start at a different stage of accomplishing the invention the following alternative embodiments are set up.

According to one of these embodiments the invention relates to a method for producing a transgenic non human animal, preferably mammal, fish, bird or insect, said method comprising introducing (a) a first nucleic acid molecule encoding an expression product with a Responder function into a chromosome of a non-human germ cell, (fertilized) egg cell, embryonic cell or a cell derived therefrom, of the same species as the transgenic animal to be prepared, said chromosome containing or conferring said genetic trait(s), thereby linking on said chromosome said Responder function to the genetic trait(s); and (b) at least one second nucleic acid molecule encoding an expression product with a Distorter function into (a) chromosome(s) of a non-human germ cell, (fertilized) egg cell, embryonic cell or a cell derived therefrom, of the same species as the transgenic female to be prepared, wherein said expression product with a Distorter function is a factor involved in G protein signaling, wherein said first nucleic acid molecule encoding an expression product with Responder function and said at least one second nucleic acid molecule encoding an expression product with a Distorter function are introduced into the same or different chromosomes.

Alternatively, the invention envisages a method for producing a transgenic non human animal, preferably mammal, fish, bird or insect, said method comprising introducing (a) a first nucleic acid molecule encoding an expression product with a Responder function into a chromosome of a non-human germ cell, (fertilized) egg cell, embryonic cell or a cell derived therefrom, of the same species as the transgenic animal to be prepared, said chromosome containing or conferring said genetic trait(s), thereby linking on said chromosome said Responder function to the genetic trait(s); and (b) at least one second nucleic acid molecule encoding an expression product directed against the Distorter function into (a) chromosome(s) of a non-human germ cell, (fertilized) egg cell, embryonic cell or a cell derived therefrom, of the same species as the transgenic female to be prepared, wherein said Distorter function is an expression product which is encoded by a nucleic acid molecule encoding an expression product with a Distorter function, wherein said expression product with a Distorter function is a factor involved in G protein signaling; and/or (c) a second nucleic acid molecule for inactivation of the Distorter function by homologous recombination, wherein said second nucleic acid molecule for inactivation of the Distorter function by homologous recombination is at least partially identical to said nucleic acid molecule encoding an expression product with a Distorter function, wherein said first nucleic acid molecule encoding an expression product with a Responder function and said at least one second nucleic acid molecule encoding an expression product directed against the Distorter function and/or said second nucleic acid molecule for inactivation of the Distorter function by homologous recombination are introduced into the same or different chromosomes, thereby partially or completely inactivating the Distorter function.

The above animal may be male or female. In further embodiments, the invention envisages methods for the production of transgenic animals wherein only step (b) is carried out. Again, said animals are preferably mammals, birds, fish or insects.

From these animals the male animal of the main embodiments (if the outcome is not a male anyhow) can be generated by the above embodiments.

Methods for the generation of transgenic mammal, fish, bird or insects are well known in the art and are described (for example in (DePamphilis 1993), (Chapman, Lawson et al. 2005).
Other methods comprise the use of retroviral, in particular lentiviral particles carrying constructs engineered in vitro for the infection of cells, preferably egg cells, zygotes or early embryos, the integration of recombinant DNA constructs into embryonic stem cells and production of stem cell/embryo chimera, or the generation of egg cells or sperm cells from embryonic stem cells having integrated the recombinant DNA construct, by differentiation of said cells in vitro (Lever et al., 2004; Hubner et al., 2003; Geijsen et al., 2004).

A further method comprises recombinase mediated cassette exchange (RMCE) whereby a construct which is flanked by non-identical target sites, such as IoxP and lox2272 sites, recognized by a site specific recombinase, such as Cre is exchanged by homologous recombination mediated by the recombinase for a fragment which is contained in a chromosome and which is flanked by said sites (such as loxP and lox2272 in this example), thereby integrating the construct into said chromosome (Pirottin, Grobet et al. 2005).

The method of the invention also comprises embodiments related to the cloning of transgenic animals. These embodiments include the steps of introducing the nucleic acid molecule as defined in the present invention, recombinant DNA molecule or vector comprising said nucleic acid molecule into the nucleus of a cell, preferably an embryonic cell, replacing the nucleus of an oocyte, a zygote or an early embryo with said nucleus comprising said nucleic acid molecule, recombinant DNA molecule or vector, transferring either said oocyte, zygote or early embryo into a foster mother or first in vitro or in vivo culturing said oocyte, zygote or early embryo and subsequently transferring the resulting embryo into a foster mother and allowing the embryo to develop to term; see, for example, (Wilmut, Schnieke et al. 1997).
A method for the production of a transgenic non-human animal, for example transgenic mouse, comprises introduction of a nucleic acid molecule or targeting vector into a germ cell, an embryonic cell, stem cell or an egg or a cell derived therefrom. Production of transgenic embryos and screening of those can be performed, e.g., as described (Joyner 1993). The DNA of the embryonal membranes of embryos can be analyzed using, e.g., Southern blots with an appropriate probe. A general method for making transgenic non-human animals is described in the art, see for example WO 94/24274. For making transgenic non-human organisms (which include homologously targeted non-human animals), embryonal stem cells (ES cells) are preferred. Murine ES cells, such as AB-1 line grown on mitotically inactive SNL76/7 cell feeder layers (McMahon and Bradley, Cell 62: 1073-1085 (1990)) essentially as described (Robertson, E. J. (1987) in Teratocarcinomas and Embryonic Stem Cells: A Practical Approach. E. J. Robertson, ed. (Oxford: IRL Press), p. 71-112) may be used for homologous gene targeting. Other suitable ES lines include, but are not limited to, the E14 line (Hooper et al., Nature 326: 292-295 (1987)), the D3 line (Doetschman et al., J. Embryol. Exp. Morph. 87: 27-45 (1985)), the CCE line (Robertson et al., Nature 323: 445-448 (1986)), the AK-7 line (Zhuang et al., Cell 77: 875-884 (1994) which is incorporated by reference herein). The success of generating a mouse line from ES cells bearing a specific targeted mutation depends on the pluripotence of the ES cells (i. e., their ability, once injected into a host developing embryo, such as a blastocyst or morula, to participate in embryogenesis and contribute to the germ cells of the resulting animal). The blastocysts containing the injected ES cells are allowed to develop in the uteri of pseudopregnant nonhuman females and are born as chimeric mice. The resultant transgenic mice are chimeric for cells having either the recombinase or reporter loci and are backcrossed and screened for the presence of the correctly targeted transgene (s) by PCR or Southern blot analysis on tail biopsy DNA of offspring so as to identify transgenic mice heterozygous for either the recombinase or reporter locus/loci.
Methods for producing transgenic flies, such as Drosophila melanogaster are also described in the art, see for example US-A-4,670,388, Brand & Perrimon, Development (1993) 118: 401-415; and Phelps & Brand, Methods (April 1998) 14: 367-379.

In a preferred embodiment of the method of the present invention said mammal is selected from the group consisting of Mus, Rattus, Bos, Sus and Ovis.

It is more preferred that Mus is Mus musculus, Rattus is Rattus norvegicus, Bos is Bos taurus, Sus is Sus scrofa f. domestica.

In another preferred embodiment of the method of the present invention said genetic trait is sex.

In further preferred embodiment of the method of the present invention said chromosome is an X or Y chromosome or a corresponding sex chromosome in birds (W, Z), fish or insect. Changing the transmission ratio in insects will have an important impact on the fight against insect pests. For example, by mixing a number of transgenic male Anopheles prepared in accordance with this invention with a naturally occurring Anopheles population, responsible for the spreading of malaria, the production of e.g. predominantly male offspring of the transgenic Anopheles is expected (see also explanation herein below). These male Anopheles will change the overall frequency of males in the population and thus lead to an overall mating problem in the Anopheles population which eventually will lead to an overall reduced amount of Anopheles. A corresponding strategy may be employed with, for example, locusts.

In a still further preferred embodiment of the method of the present invention said chromosome is an autosome.

In still a further preferred embodiment of the method of the present invention the factor involved in G protein signalling is a factor involved in Rho signalling.
The term "a factor involved in Rho signaling" as used in connection with the present invention refers to small G proteins of the Rho subfamily, as well as to molecules acting upstream or downstream of G proteins in terms of signal transduction. Such molecules comprise in particular members of the families of GEFs (guanine nucleotide exchange factors), which enhance the activity of small G proteins, GAPs (GTPase activating proteins) which act as negative regulators and GDls (guanine nucleotide dissociation inhibitors) which also attenuate small G protein signaling (Schmidt and Hall, 2002; Donovan et al., 2002; DerMardirossian and Bokoch, 2005). Finally, the term refers to target molecules, influenced by small G proteins (Bishop and Hall, 2000).

In a further preferred embodiment of the method of the present invention said first nucleic acid molecule encoding an expression product with Responder function is selected from the group consisting of (a) a nucleic acid molecule comprising or consisting of the nucleic acid molecule as shown in SEQ ID No:15 or 16 or a fragment thereof; (b) a nucleic acid molecule being an allelic variant or a homologue or orthologue of the nucleic acid molecule of (a); (c) a nucleic acid molecule hybridizing to a nucleic acid molecule complementary to the nucleic acid molecule of (a) or (b); and (d) a nucleic acid molecule which is related to the nucleic acid molecule of (a), (b) or (c) by the degeneration of the genetic code.

In further preferred embodiment of the method of the present invention said (at least one) second nucleic acid molecule encoding an expression product with a Distorter function is/are selected from the group consisting of (a) a nucleic acid molecule comprising or consisting of the nucleic acid molecule as shown in any one of SEQ ID NOs: 1 to 14 or a fragment thereof, (b) a nucleic acid molecule being an allelic variant or a homologue or orthologue of the nucleic acid molecules of (a); (c) a nucleic acid molecule hybridizing to a nucleic acid molecule complementary to the nucleic acid molecule of (a) or (b); and (d) a nucleic acid molecule which is related to the nucleic acid molecule of (a), (b) or (c) by the degeneration of the genetic code.

The term "an allelic variant or homologue" as used in connection with the present invention refers to different wild type forms and t-alleles of the nucleic acid molecues.
The nucleic acid molecules can be further manipulated *in vitro* in order to achieve an optimized transmission ratio distortion effect and/or to adapt it to the specific requirements of the breeding scheme employed, thus further improving the selectability of genetic traits as further described below. A number of standard manipulations known in the field are taken into consideration, such as those resulting in the exchange of amino acids in the catalytic domain(s) which is the GAP domain in case of the GTPase activating proteins and the DH (Dbl-homology) domain in case of the guanine nucleotide exchange factors, overexpression or knock out mutagenesis of said nucleic acid molecules, construction of hypomorphic or hypermorphic (poly)peptides by mutagenesis, deletion or alteration of candidate modification sites on said (poly)peptide, deletion or alteration of binding sites for other (poly)peptides involved in the G protein signaling cascade (see for example Dvorsky and Ahmadian, 2004), synthesis of antisense RNA, siRNA, shRNA, N-terminal or C-terminal truncations, introduction of frame shifts, which alter part of the amino acid sequence of the protein, etc., resulting either in null, hypomorphic, constitutively active, antimorphic or dominant negative alleles. It is also envisaged that a distortion of the transmission ratio can be achieved with several, if not all, manipulated forms of the nucleic acid molecules described above. Thus, a manipulated allele affecting the transmission ratio most effectively will have to be identified empirically for each gene by employing activity assays in vitro and in cell culture systems such as NIH-3T3 cells and transgenic animal systems.

The term "orthologue" as used in connection with the present invention refers to genes present in different organisms and which have the same function.

The term "fragment" as used in connection with the method of the present invention relates to the fact that said fragment retains the Responder/Distorter function.

If fragments, allelic variants, homologues or orthologues of a specifically identified sequence conferring responder or Distorter function are referred to throughout this specification, it is understood that these fragments etc. retain or essentially retain the Responder or Distorter function. "Essentially retain" means in accordance with these embodiments that at least 70% of the function are retained, preferably at least 80% such as at least 90%.

The term "hybridizing" as used in connection with the present invention and as used in the description of the present invention, preferably refers to "hybridizing under stringent conditions", and is well known to the skilled artisan and corresponds to conditions of high stringency. Appropriate stringent hybridization conditions for each nucleic acid sequence may be established by a person skilled in the art on well-known parameters such as temperature, composition of the nucleic acid molecules, salt conditions etc.; see, for example, (Sambrook J. 1989) (Hames 1985), see in particular the chapter "Hybridization Strategy" by Britten & Davidson, 3 to 15. Stringent hybridization conditions are, for example, conditions comprising overnight incubation at 42° C in a solution comprising: 50% formamide, 5x SSC (750 mM NaCl, 75 mM trisodium citrate), 50 mM sodium phosphate (pH 7.6), 5x Denhardt's solution, 10% dextran sulfate, and 20 micrograms/ml denatured, sheared salmon sperm DNA, followed by washing the filters in 0.1 x SSC at about 65°. Other stringent hybridization conditions are for example 0.2 x SSC (0.03 M NaCl, 0.003Msodium citrate, pH 7) at 65°C. In addition, to achieve even higher stringency, washes performed following stringent hybridization can be done at higher salt concentrations (e.g. 5X SSC). Note that variations in the above conditions may be accomplished through the inclusion and/or substitution of alternate blocking reagents used to suppress background in hybridization experiments. Typical blocking reagents include, but are not limited to, Denhardt's reagent, BLOTTO, heparin, denatured salmon sperm DNA, and commercially available proprietary formulations. The inclusion of specific blocking reagents may require modification of the hybridization conditions described above, due to problems with compatibility. Also contemplated are nucleic acid molecules encoding an interaction partner of a biomolecule, wherein the interaction partner is capable of modulating the activity said biomolecule and wherein the nucleic acid molecules hybridize to the nucleic acid molecule encoding the biomolecule at even lower stringency hybridization conditions. Changes in the stringency of hybridization and signal detection are, for example, accomplished through the manipulation of formamide concentration (lower percentages of formamide result in lowered stringency); salt conditions, or temperature. For example, lower stringency conditions include an overnight incubation at 37 degree C in a solution comprising 6X SSPE (20X SSPE = 3M NaCl; 0.2M NaH₂PO₄; 0.02M EDTA, pH 7.4), 0.5% SDS, 30% formamide, 100 µg/ml salmon sperm blocking DNA; followed by washes at 50 degree C with 1XSSPE, 0.1% SDS. In addition, to achieve even lower stringency, washes performed following stringent hybridization can be done at higher salt concentrations (e.g. 5X SSC). Variations in the above conditions may be accomplished through the inclusion and/or substitution of alternate blocking reagents used to suppress background in hybridization experiments. Typical blocking reagents include, but are not limited to, Denhardt's reagent, BLOTTO, heparin, denatured salmon sperm DNA, and commercially available proprietary formulations. The inclusion of specific blocking reagents may require modification of the hybridization conditions described above, due to problems with compatibility.

As shown above, it is preferred that the method of the present invention be carried out by using the nucleic acid molecules encoding a Distorter function described in SEQ ID NOs: 1 to 14. These sequences relate to wildtype Tagap1 (SEQID NO: 1 and 2), the t-alleles of Tagap1, Tagap1 ^{t1 to t4} (SEQ ID NOs: 3 to 6), the homo sapiens Tagap (SEQ ID NO: 7), the Rattus Tagap (SEQ ID NO: 8), the mouse wildtype Fgd2 (SEQ ID NO: 9), two isoforms thereof (SEQ ID NOs: 10 and 11), a t-allele of Fgd2 (SEQ ID NO: 12), the mouse and homo sapiens Tiam 2 (SEQ ID NOs 13 and 14).

In another preferred embodiment of the method of the present invention said at least one second nucleic acid molecule encoding an expression product with a Distorter function is/are selected from the group consisting of (a) a nucleic acid molecule comprising or consisting of the nucleic acid molecule as shown in any one of SEQ ID Nos 1 to 12 or a fragment thereof (b) a nucleic acid molecule being an allelic variant or a homologue or orthologue of the nucleic acid molecule of (a); (c) a nucleic acid molecule hybridizing to a nucleic acid molecule complementary to the nucleic acid molecule of (a) or (b); and (d) a nucleic acid molecule which is related to the nucleic acid molecule of (a), (b) or (c) by the degeneration of the genetic code, thereby enhancing said transmission ratio of said genetic trait(s).

In a further preferred embodiment of the method of the present invention said at least one second nucleic acid molecule encoding an expression product with a Distorter function is/are selected from the group consisting of (a) a nucleic acid molecule comprising or consisting of the nucleic acid molecule as shown in of SEQ ID NO: 13 or 14 or a fragment thereof; (b) a nucleic acid molecule being an allelic variant or a homologue or orthologue of the nucleic acid molecule of (a); (c) a nucleic acid molecule hybridizing to a nucleic acid molecule complementary to the nucleic acid molecule of (a) or (b); and (d) a nucleic acid molecule which is related to the nucleic acid molecule of (a), (b) or (c) by the degeneration of the genetic code, thereby reducing said transmission ratio of said genetic trait(s).

In another preferred embodiment of the method of the present invention said nucleic acid molecule encoding an expression product with a Distorter function is selected from the group consisting of (a) a nucleic acid molecule comprising or consisting of the nucleic acid molecule as shown in any one of SEQ ID NOs: 1 to 12 or a fragment thereof; (b) a nucleic acid molecule being an allelic variant or a homologue or orthologue of the nucleic acid molecule of (a); (c) a nucleic acid molecule hybridizing to a nucleic acid molecule complementary to the nucleic acid molecule of (a) or (b); and (d) a nucleic acid molecule which is related to the nucleic acid molecule of (a), (b) or (c) by the degeneration of the genetic code, thereby reducing said transmission ratio of said genetic trait(s).

In still another preferred embodiment of the method of the present invention said nucleic acid molecule encoding an expression product with a Distorter function is selected from the group consisting of (a) a nucleic acid molecule comprising or consisting of the nucleic acid molecule as shown in SEQ ID Nos 13 or 14 or a fragment thereof; (b) a nucleic acid molecule being an allelic variant or a homologue or orthologue of the nucleic acid molecule of (a); (c) a nucleic acid molecule hybridizing to a nucleic acid molecule complementary to the nucleic acid molecule of (a) or (b); and (d) a nucleic acid molecule which is related to the nucleic acid molecule of (a), (b) or (c) by the degeneration of the genetic code, thereby enhancing said transmission ratio of said genetic trait(s).

In further preferred embodiment the method of the present invention further comprises crossing the transgenic non human male mammal, fish, bird or insect obtained by the method of the present invention with a non human female mammal, fish, bird or insect and analyzing the offspring of said cross for transmission of said genetic trait(s).

The above preferred embodiments of the method of the present invention are of particular interest for the applicability of the present invention. The genetic trait(s) of interest can be transmitted to the offspring either at an enhanced or at a reduced ratio with respect to the Mendelian ratio. In particular it is envisaged to use the methods of the present invention in the field of farm animal breeding as a tool for manipulating the transmission ratio of genetic traits. The most interesting trait in this respect is sex. The method of the present invention making use of the Distorter now allows an enhancement of the effect of the Responder, for example it will be possible to obtain strong selection for or against sperm carrying the Y chromosome. It is therefore envisaged that a transgene construct expressing the nucleic acid molecule encoding the Responder function and expressing at least one other nucleic acid molecule encoding (a) Distorter function(s) and/or products directed against the Distorter function be integrated on the Y-chromosome of the farm animal species. In one embodiment of the present invention action of the Distorter(s) would impair the sperm cells carrying the Responder, which would result in a preferential or exclusive transmission of the X-chromosome and thus generation of female offspring. In another embodiment of the present invention the action of the Distorter(s) and/or the product(s) directed against the Distorter function would impair all sperm cells, while the sperm cells carrying and expressing the Responder would be rescued. In that latter embodiment the Y chromosome would be preferentially or exclusively transmitted to the offspring resulting in the production of male offspring. Likewise, the construct expressing the Responder function and the construct(s) expressing at least one Distorter function and/or product(s) directed against the Distorter function could be integrated on the X chromosome and allow the generation of males preferentially or exclusively transmitting the Y chromosome or, in the latter example, wherein a high transmission ratio of the Responder construct is achieved, the X chromosome. It will depend on the design of the construct(s) (as taught above) expressing the Distorter function and/or product(s) directed against the Distorter function whether enhanced transmission or reduced transmission of the chromosome carrying the Responder construct will be achieved. For example, in the present invention it could be shown that inactivation of wild type Tagap1 and inactivation of wild type Fgd2 both resulted in a reduced transmission of Tcr, while the respective t-Distorters Tagap1^{Tcd1a} and Fgd2^{Tcd2} enhance the transmission of Tcr. In contrast, it is envisaged that loss of Tiam2 function enhances the transmission of Tcr, while a hypermorphic allele is envisaged to reduce the transmission ratio of Tcr. Thus, the teachings of the t-Distorters and of the mutations in the wild type gene provided by the present invention provide the knowledge how Distorter alleles need to be engineered to achieve enhancement or reduction of the transmission ratio of the chromosome carrying the Responder construct and the genetic trait(s) linked to it. Importantly, Distorters act additively or synergistically, thus the combinatorial use of several nucleic acids encoding Distorter function(s) and/or products directed against the Distorter function(s) is preferred in order to achieve an optimal effect. For example, it is envisaged that the combination of nucleic acid molecules encoding products directed against the function of Tagap1 and of Fgd2 and of a construct overexpressing Tiam2 achieves a strong effect with respect to selection against sperm carrying and expressing the Responder construct, since all three expression products singly should reduce the transmission ratio of the Responder.
It is furthermore envisaged that the constructs expressing the Distorter(s) and/or the constructs expressing an expression product directed against the Distorter function and/or the constructs for inactivation of the Distorter function can be integrated independently of the Responder construct on the same or on different chromosomes.

Such a tool for preselection of sex in farm animals is most desirable for Bos taurus, a species for which specialized strains for milk or meat production have been derived. Female offspring is needed for milk production whereas for meat production male offspring is preferred in this species. In most other farm animal species female offspring is most desired. Thus, preselection of sex is of general importance in farm animal breeding.

In another preferred embodiment of the method of the present invention said Responder function and/or said Distorter function is the mouse-t-complex Responder/Distorter function.

Although it is possible without undue burden to identify mutated or wild-type Distorters in animals other than the mouse on the basis of the genetic structure of the Distorter that is provided in accordance with the present invention, it is envisaged that the mouse t-complex Distorter may find applications, for example in breeding, also when introduced into other animals. Specific applications of the Distorter function are addressed herein below.

In still another preferred embodiment of the method of the present invention said expression product directed against the Distorter function is an aptamer, a siRNA or shRNA or miRNA, a ribozyme, or an antisense nucleic acid molecule specifically hybridizing to said nucleic acid molecules encoding a factor involved in G protein signaling, or is an antibody, an antibody fragment or derivative thereof specific for the Distorter (poly)peptides as used in connection with the present invention.

It is envisaged that shRNA (small hairpin RNA) molecules expressed from a construct integrated into the genome can be used for degradation of RNA molecules (known as RNA interference) transcribed from (a) endogenous gene(s) encoding (a) Distorter(s) thereby partially or completely down-regulating the function of said Distorter(s). Likewise vectors comprising nucleic acid molecules encoding a miRNA (microRNA) can be utilized for inhibition of translation of the RNA encoding said Distorter(s) (Kim 2005). Constructs expressing aptamers can be utilized to inhibit protein-protein interaction such as between a Distorter protein and another (poly)peptide of the Distorter/Responder signaling cascade in order to interfere with the propagation of the signal thereby inhibiting said signal pathway. The person skilled in the art is able to design shRNA or miRNA constructs on the basis of the sequence of the mRNA of the gene the function of which shall be down-regulated. The efficacy of the constructs can easily be tested in cellular systems. Aptamers able to inhibit protein-protein interaction can be selected in vitro or in cellular system such as the yeast the method comprising assaying inhibition of protein-protein interaction as measured in the yeast two-hybrid assay (Schmidt, Diriong et al. 2002) (Kurtz, Esposito et al. 2003) (Cassiday and Maher 2003).

The term "antibody fragment or derivative thereof" relates to single chain antibodies, or fragments thereof, synthetic antibodies, antibody fragments, such as Fab, a F(ab₂)', Fv fragments, single domain antibodies etc., or a chemically modified derivative of any of these. Derivatives include scFvs. Antibodies to be employed in accordance with the invention or their corresponding immunoglobulin chain(s) can be further modified using conventional techniques known in the art, for example, by using amino acid deletion(s), insertion(s), substitution(s), addition(s), and/or recombination(s) and/or any other modification(s) (e.g. posttranslational and chemical modifications, such as glycosylation and phosphorylation) known in the art either alone or in combination. Methods for introducing such modifications in the DNA sequence underlying the amino acid sequence of an immunoglobulin chain are well known to the person skilled in the art; see, e.g., Sambrook et al.; Molecular Cloning: A Laboratory Manual; Cold Spring Harbor Laboratory Press, 2nd edition 1989 and 3rd edition 2001.

The term "antibody fragment or derivative thereof" particularly relates to (poly)peptide constructs comprising at least one CDR such as two, three and preferably all six CDRs of an antibody, e.g. in the scFv format. Framework regions of the antibody may also be replaced by unspecific non-antibody-related sequences.
Fragments or derivatives of the recited antibody molecules may also define (poly)peptides which are parts of the above antibody molecules and/or which are modified by chemical/biochemical or molecular biological methods. Corresponding methods are known in the art and described inter alia in laboratory manuals (see Sambrook et al., loc cit.; Gerhardt et al.; Methods for General and Molecular Bacteriology; ASM Press, 1994; Lefkovits; Immunology Methods Manual: The Comprehensive Sourcebook of Techniques; Academic Press, 1997; Golemis; Protein-Protein Interactions: A Molecular Cloning Manual; Cold Spring Harbor Laboratory Press, 2002; Antibodies, A Laboratory Manual, Ed Harlow and David Lane, Cold Spring Harbor Laboratory, 1988).

In a preferred embodiment of the method of the present invention said at least one second nucleic acid molecule encoding the expression product with a Distorter function is modified, thereby further reducing or further enhancing the Distorter function activity.
The term "further reducing or further enhancing the Distorter function activity" as used in connection with the method of the present invention refers to the fact that the method of the present invention can be further optimized by genetically manipulating the nucleic acid molecules encoding the Distorters. For example, dominant active or dominant negative alleles of (a) Distorter(s) may be designed and assayed in vitro for their ability to enhance or interfere with the activity of the wild type allele of said Distorter, followed by genetic testing in vivo in transgenic animals of the allele(s) which improve the activity of said Distorter or enhance down-regulation of said Distorter in vitro. Other alterations of the nucleic acid sequence resulting in changes of the polypeptide encoded by said Distorter gene may be introduced in vitro by exchanging nucleic acid molecules or by synthesizing genes or gene parts in vitro or by random mutagenesis, and (high-throughput) in vitro assays can be designed to measure the activity of the altered proteins or their ability to enhance or inhibit or interfere with (a) component(s) of the Distorter/Responder signal cascade.

In further preferred embodiment of the method of the present invention said first nucleic acid molecule encoding an expression product with a Responder function and said at least one second nucleic acid molecule encoding an expression product with Distorter function and/or said at least one second nucleic acid molecule encoding an expression product directed against the Distorter function and/or said second nucleic acid molecule for inactivation of the Distorter function by homologous recombination and a promoter controlling expression in spermatogenesis and/or spermiogenesis and/or a stop cassette are integrated in said X or Y chromosome or corresponding sex chromosome or in one of said autosomes in a reversible inactive state of expressibility. Preferably, said promoter is a heterologous promoter.

The term "reversibe inactive state of expressibility" as used in connection with the method of the present invention refers to the possibilty to keep the above nucleic acid molecules in an inactive state of expressibility which can by genetic means be activated, as further described below.

In particular, it is envisaged that the construct(s) expressing the Distorter(s) and/or products directed against the Distorter function(s) reduce the transmission of the nucleic acid molecule encoding a Responder function to such a low ratio that the transmission of the Responder construct is almost or completely excluded. This would mean that one important component of the functional principle of the present invention is not passed on to the next generation by natural breeding. To circumvent this problem it is envisaged to introduce a construct containing the nucleic acid molecule encoding the Responder in an inactive state, for instance by inserting a transcription stop cassette between the promoter controlling expression of the Responder gene and the nucleic acid encoding the Responder, comprising flanking the stop cassette by loxP sites in same orientation. This construct would be inactive with respect to expression of the Responder and thus could be transmitted at Mendelian ratio to the offspring. Males producing sperm allowing preselection of (a) trait(s) are envisaged to be produced by breeding the male or female carrying the inactive Responder construct to a female or male carrying a construct expressing Cre recombinase prior to spermiogenesis. Activation of the Responder construct would then occur by excision of the transcription Stop cassette due to the action of the Cre recombinase during embryonic development or in germ cells. Other combinations of recombinase and specific recognition sites for the recombinase, or the use of other nucleic acid molecules instead of a stop cassette, or the inverse orientation of the nucleic acid encoding the Responder flanked by sites for site specific recombinases in inverse orientation, are also envisaged ways to achieve a reversible inactive state of expressibility.
It is also envisaged to achieve transmission of (a) construct(s) which is not transmitted through sperm cells by propagation of said construct(s) in females. This is particularly useful when the construct(s) is/are integrated on the X chromosome and/or (an) autosome(s). It is furthermore envisaged that the construct(s) will only be activated in sperm cells, in particular if promoter(s) are used which activate transcription specifically during spermatogenesis and/or spermiogenesis. Thus, it is envisaged that selection against transmission of said construct(s) will be restricted to transmission through sperm cells, while transmission through the female germ cells occurs normally.

The use of constructs designed for selection against a genetic trait such as male sex is rendered in some rare cases difficult by the fact that the transgene construct may not or hardly be transmitted to the offspring of the carrier male animal. In such cases it is envisaged to use sperm cells at random or after preselection of cells carrying the transgene construct in order to significantly enhance the likelihood for the production of offspring carrying said transgene construct. Selection can be effected, e.g., by cell sorting.
It is also envisaged to make use of in vitro fertilization since it has been shown that transmission ratio distortion in mouse does not occur during in vitro fertilization procedures; other methods are ICSI (intracellular sperm cell injection), (Horiuch, Emuta et al. 2002)

Furthermore, the present invention relates to a non human male or female animal, preferably mammal, fish, bird or insect, wherein said non human male or female animal, preferably mammal, fish, bird or insect is transgenic for the nucleic acid molecule encoding an expression product with a Distorter function and/or the nucleic acid molecule encoding an expression product directed against the Distorter function and/or the nucleic acid molecule for inactivation of the Distorter function by homologous recombination as defined in the present invention and optionally for the nucleic acid molecule encoding an expression product with a Responder function.

The present invention also relates to a pair of non human male and female animals, preferably mammals, fish, birds or insects, wherein at least one of the male and/or female is a transgenic non human mammal, fish, bird or insect as defined in the present invention.

Preferably, the nucleic acid molecule or part thereof encoding an expression product with a Responder function and/or the nucleic acid molecule or part thereof encoding an expression product with a Distorter function and/or the nucleic acid molecule or part thereof encoding an expression product directed against the Distorter function and/or the nucleic acid molecule or part thereof for inactivation of the Distorter function by homologous recombination as defined in the present invention is/are flanked by recombinase recognition sites.

It is also preferred that one of the pair has (only) the Responder stably integrated into the germline whereas the partner of the pair has (only) integrated the Distorter into the germline. Upon crossing the offspring will carry both the Responder and the Distorter in the germline. In this manner, male offspring may be selected that is described in accordance with the main embodiments of the invention.

It is further preferred that the above pair of non-human male and female animal, preferably mammal, fish, bird or insect has further stably integrated into its genomic DNA a nucleic acid molecule encoding a site specific DNA recombinase.

In the specific cases with low or no transmission of the transgene construct designed for selection against a genetic trait it is envisaged to use constructs, which are in an inactive state and therefore not selected against under standard breeding conditions, but can be activated after expression of a site specific recombinase. Using this method the (inactive) transgene construct can be transmitted at Mendelian rates. After expression of a site specific recombinase (such as Cre) from an inducible construct or by breeding of the male or female carrying the Cre gene in an active state to the female or male carrying said inactive transgene construct offspring can be generated which carries said transgene construct in an active state allowing selection against the sperm cells carrying said transgene construct. The latter offspring could then be utilized for the production of animals, which do not carry the undesired genetic trait.
Several methods can be utilized to keep a transgene construct in an inactive state, the most common being the use of a transcription stop cassette and/or a reporter gene inserted between the promoter driving expression of the transgene construct and the open reading frame (ORF) of the gene kept inactive by this method. It is envisaged that the stop cassette and/or reporter is flanked by recognition sequences for the site specific recombinase in direct repeat orientation allowing deletion of the stop cassette and/or reporter upon recombination, and subsequent expression of the ORF made active by this recombination event.

It is more preferred that in the pair of non human male and female animal, preferably mammal, fish, bird or insect of the present invention said DNA recombinase is Cre, wherein said recognition sites are IoxP sites, or flp, wherein said recognition sites are FRT sites, or Φc31, wherein said recognition sites are att sites.

It is also more preferred that in the pair of transgenic non human male and female animals, preferably mammals, fish, birds or insects of the present invention said DNA recombinase is controlled by regulatory elements that are active prior to spermiogenesis.

The present invention also relates to sperm obtainable from a male of the transgenic non-human animal, preferably mammal, fish, bird or insect of the present invention.

The present invention further relates to the use of the sperm of the present invention for the production of offspring.

The present invention also relates to the use of the nucleic acid molecule encoding an expression product with a Distorter function as defined in the present invention, for the identification of chemicals or biological compounds able to trigger the (premature) activation or inhibition of the Responder/Distorter signalling cascade.

The term "Responder/Distorter signalling cascade" as used above refers to any G protein signalling cascade, wherein at least one of the G proteins or other proteins in the cascade confers Responder/Distorter function.

Such compounds could be applicable as potent contraceptiva since it is envisaged that the activation or inhibition (repression) of said signaling cascade may affect the motility of sperm, due to rapid exhaustion of their energy reserve, and/or by inhibiting sperm movement and/or by affecting the ability of sperm to fertilize ovulated eggs.
It is envisaged that the identification of said chemical or biological compounds could be achieved by standard screening technology using the activity of the wild type Distorter protein expressed in vitro or in cell culture cells as an assay. It is e.g. known that GTPase-activating proteins such as Tagap1 enhance the GTPase activity of target GTPases rendering them inactive, or that GEFs such as Fgd2 or Tiam2 exchange GDP for GTP in said GTPases rendering them active. Assay systems for the activity of GAPs and GEFs and GTPases and other proteins involved in G protein signaling are well known in the art (Balch 1995) (Der 2000) allowing an artisan to screen for compounds triggering or inhibiting said proteins in vitro or in cell culture systems. It is envisaged that the compounds are then tested for their effects on sperm motility in vitro and on their effect in preventing fertilization of egg cells by sperm in vivo.

The present invention further relates to the use of the nucleic acid molecule encoding an expression product with a Distorter function as defined in the present invention for the isolation of receptor molecules and/or other members of the Responder/Distorter signaling cascade to which said expression product may bind.

Furthermore, the nucleic acid molecule as defined in the method of the present invention or the expression product as defined in the method of the present invention can be used for the isolation of receptor molecules and/or other members of the Responder/Distorter signaling cascade to which said expression product which would be expected to be a (poly)peptide may bind. Said signal transducing molecules are envisaged to be preferably identified by immunoprecipitation of protein complexes involving the Distorter (poly)peptide and cloning of the corresponding genes encoding them, or by Two Hybrid Screening techniques in yeast employing standard technology. In particular, most preferably the Distorter gene or (poly)peptide may be used to isolate the membrane receptor of the signaling molecule which is envisaged to activate said Responder/Distorter signaling cascade. Said membrane receptor is envisaged to be most preferable as a target for the development of novel contraceptives.

The present invention also relates to a method for the detection of a nucleic acid molecule encoding an expression product with a Distorter function and/or a nucleic acid molecule encoding an expression product directed against the Distorter function and/or a nucleic acid molecule for inactivation of the Distorter function by homologous recombination as defined in the present invention in a non human male or female animal, preferably mammal, fish, bird or insect as defined in the present invention comprising identifying said nucleic acid molecule encoding an expression product with a Distorter function and/or said nucleic acid molecule encoding an expression product directed against the Distorter function and/or said nucleic acid molecule for inactivation of the Distorter function by homologous recombination in said non human male or female animal, preferably mammal, fish, bird or insect by polymerase chain reaction (PCR), gene (micro)array hybridization, single nucleotide polymorphism (SNP) analysis, and/or sequencing with primers hybridizing to said nucleic acid molecule.

The present invention also relates to a nucleic acid molecule encoding an expression product with a Distorter function, wherein said expression product with a Distorter function is a factor involved in G protein signaling, selected from the group consisting of: (a) a nucleic acid molecule comprising or consisting of the nucleic acid molecule of any one of SEQ ID NOs: 3 to 6 and 12 or a fragment thereof; (b) a nucleic acid molecule being an allelic variant or a homologue or orthologue of the nucleic acid molecule of (a); (c) a nucleic acid molecule which hybridizes under stringent conditions to the nucleic acid molecule of (a), wherein said nucleic acid molecule encodes a polypeptide which has (i) at the position corresponding to position 49 of SEQ ID NO: 17 an I (ii) at the position corresponding to position 144 of SEQ ID NO: 17 an L (iii) at the position corresponding to position 323 of SEQ ID NO: 17 a T and (iv) which terminates after position 442; (d) a nucleic acid molecule which hybridizes under stringent conditions to the nucleic acid molecule of (a), wherein said nucleic acid molecule encodes a polypeptide which has(i) at the position corresponding to position 49 of SEQ ID NO: 17 an I;(ii) at the position corresponding to position 137 of SEQ ID NO: 17 an E;(iii) at the position corresponding to position 207 of SEQ ID NO: 17 an F;(iv) at the position corresponding to position 301 of SEQ ID NO: 17 an M;(v) at the position corresponding to position 323 of SEQ ID NO: 17 an T;(vi) at the position corresponding to position 332 of SEQ ID NO: 17 a D;(vii) at the position corresponding to position 407-413 of SEQ ID NO: 17 an internal deletion;(viii) at the position corresponding to position 440 of SEQ ID NO: 17 an M;(ix) at the position corresponding to position 471 of SEQ ID NO: 17 an L;(x) at the position corresponding to position 552 of SEQ ID NO: 17 an I;(xi) at the position corresponding to position 596 of SEQ ID NO: 17 a K;(xii) at the position corresponding to position 607 of SEQ ID NO: 17 an R;(xiii) at the position corresponding to position 610 of SEQ ID NO: 17 an S; and (xiv) at the position corresponding to position 703 of SEQ ID NO: 17 a V; (e) a nucleic acid molecule which hybridizes under stringent conditions to the nucleic acid molecule of (a), wherein said nucleic acid molecule encodes a polypeptide which has (i) at the position corresponding to position 49 of SEQ ID NO: 17 an I; (ii) at the position corresponding to position 54 of SEQ ID NO: 17 a G; (iii) at the position corresponding to position 137 of SEQ ID NO: 17 an E; (iv) at the position corresponding to position 173 of SEQ ID NO: 17 a G; (v) at the position corresponding to position 207 of SEQ ID NO: 17 an F; (vi) at the position corresponding to position 301 of SEQ ID NO: 17 an M; (vii) at the position corresponding to position 323 of SEQ ID NO: 17 a T; (viii) at the position corresponding to position 332 of SEQ ID NO: 17 a D; (ix) at the position corresponding to position 407-413 of SEQ ID NO: 17 an internal deletion; (x) at the position corresponding to position 440 of SEQ ID NO: 17 an M; (xi) at the position corresponding to position 471 of SEQ ID NO: 17 an L; (xii) at the position corresponding to position 508 of SEQ ID NO: 17 an S; (xiii) at the position corresponding to position 552 of SEQ ID NO: 17 an I; (xiv) at the position corresponding to position 596 of SEQ ID NO: 17 a K; (xv) at the position corresponding to position 607 of SEQ ID NO: 17 an R; (xvi) at the position corresponding to position 610 of SEQ ID NO: 17 an S; and (xvii) at the position corresponding to position 703 of SEQ ID NO: 17 a V; (f) a nucleic acid molecule which hybridizes under stringent conditions to the nucleic acid molecule of (a), wherein said nucleic acid molecule encodes a polypeptide which has (i) at the position corresponding to position 49 of SEQ ID NO: 17 an I; (ii) at the position corresponding to position 137 of SEQ ID NO: 17 an E; (iii) at the position corresponding to position 207 of SEQ ID NO: 17 an F; (iv) at the position corresponding to position 301 of SEQ ID NO: 17 an M; (v) at the position corresponding to position 323 of SEQ ID NO: 17 a T; (vi) at the position corresponding to position 332 of SEQ ID NO: 17 a D; (vii) at the position corresponding to position 407-413 of SEQ ID NO: 17 an internal deletion; (viii) at the position corresponding to position 440 of SEQ ID NO: 17 an M; (ix) at the position corresponding to position 471 of SEQ ID NO: 17 an L; (x) at the position corresponding to position 530 of SEQ ID NO: 17 an E; (xi) at the position corresponding to position 552 of SEQ ID NO: 17 an I; (xii) at the position corresponding to position 573 of SEQ ID NO: 17 an R; (xiii) at the position corresponding to position 596 of SEQ ID NO: 17 a K; (xiv) at the position corresponding to position 607 of SEQ ID NO: 17 an R; (xv) at the position corresponding to position 610 of SEQ ID NO: 17 an S; and (xvi) at the position corresponding to position 703 of SEQ ID NO: 17 a V.

The nucleic acid molecule of the invention in any case retains the Distorter function. It has preferably a minimal length of at least 200 or 300 nucleotides. Such a molecule may also be used for example as a specific probe for hybridization reactions and would comprise at least one of the mutations of any one of SEQ ID NOs: 3 to 6. It is however also preferred that the nucleic acid molecules of the invention be significantly larger such as at least 500 or 1000 nucleotides. The nucleic acid molecules or fragments thereof of the invention may be fused to flanking sequences. In any case, the nucleic acid molecules of the invention may have a length of up to 500 nucleotides, 1000 nucleotides, 2000 nucleotides, 5000 nucleotides, 10000 nucleotides and in particular cases even up to 100000 nucleotides. When integrated into larger genomic regions, the nucleotides of the invention may have chromosomal length.

Additionally, the invention encompasses oligonucleotides/primers of a length of at least 8 and up to preferably 50 nucleotides, that are part of the above identified sequences or hybridize to the complementary strand thereof wherein said oligonucleotides/primers contain the sequence of at least one codon coding for any of the above-identified specific amino acid positions (or a complementary sequence thereof).

It is preferred that the nucleic acid molecule of the present invention is a DNA molecule.

It is furthermore preferred that said expression product is an RNA or a (poly)peptide.

The deduction of the amino acid sequence from the nucleic acid sequence of the invention allows the conclusion that the polypeptide is the expression product that contributes to the Responder/Distorter phenotype. However, it is not excluded that the mRNA contributes to said Responder/Distorter phenotype. Also, it is envisaged in accordance with the present invention that in certain embodiments the expression level, stage of expression during spermatogenesis or the copy number of said gene results in or contributes to the Distorter phenotype. Therefore, in a preferred embodiment of the nucleic acid molecule of the invention said expression product is an RNA or a (poly)peptide.

The present invention also relates to a recombinant DNA molecule comprising the nucleic acid molecule as defined above and a regulatory region being capable of controlling expression of said nucleic acid molecule.

It is further preferred that said regulatory region is a naturally occurring region or a genetically engineered derivative thereof.

It is further preferred that said regulatory region comprises or is a promoter.

The present invention also relates to a vector comprising the recombinant DNA molecule of the present invention.

The vector of the invention may simply be used for propagation of the genetic elements comprised therein. Advantageously, it is an expression vector and/or a targeting vector. Expression vectors such as Pichia pastoris derived vectors or vectors derived from viruses such as CMV, SV-40, baculovirus or retroviruses, vaccinia virus, adeno-associated virus, herpes viruses, or bovine papilloma virus, may be used for delivery of the recombinant DNA molecule or vector of the invention into targeted cell population. Methods which are well known to those skilled in the art can be used to construct recombinant viral vectors; see, for example, the techniques described in Sambrook, loc. cit. and Ausubel, loc. cit. Alternatively, the recombinant DNA molecules and vectors of the invention can be reconstituted into liposomes for delivery to target cells.

It is preferred that the vector of the present invention comprises a heterologous promoter.

It is preferred according to one further embodiment that said vector comprises a heterologous promoter.
Said heterologous promoter not naturally operatively linked with the nucleic acid contributing to the Distorter function may be used to determine a certain time point of the onset of Distorter expression. This time point may be the same or a different one that is set when the natural Distorter transcription unit is employed. For example, said heterologous promoter may also be active in the early or late haploid phase of spermatogenesis.

It is more preferred that said heterologous promoter is controlling gene expression in spermatogenesis and/or in spermiogenesis.

It is even more preferred that said the heterologous promoter is the testis promoter of c-kit, ACE, Tcr or Smok.

The present invention also relates to a host cell or organism transformed or transfected with the nucleic acid molecule of the present invention, the recombinant DNA molecule of the present invention or the vector of the present invention.

The host cell can be any prokaryotic or eukaryotic cell, such as a bacterial, insect, fungal, plant, animal or human cell. Prokaryotic host cells will usually only be employed for the propagation of the nucleic acid molecule of the invention and sometimes for the production of the expression product. Suitable mammalian, fish or bird cell lines are well known or can easily be determined by the person skilled in the art and comprise COS cells, Hela cells, primary embryonic cell lines etc.

The term "transfected or transformed" is used herein in its broadest possible sense and also refers to techniques such as electroporation, infection or particle bombardment.

The present invention furthermore relates to a method of recombinantly producing an expression product as defined for the nucleic acid of the present invention comprising the steps of culturing the host cell of the present invention under conditions to cause expression of the protein and recovering said protein from the culture.

The method of the invention is most advantageously carried out along conventional protocols which have been described, for example, in Sambrook, loc. cit.

The present invention also relates to an expression product encoded by the nucleic acid molecule of the present invention or obtainable by the above method of recombinantly producing an expression product.

In accordance with the invention, said expression product may either be an mRNA or a polypeptide. Said expression product is, in accordance with the present invention, involved in the Responder/Distorter phenotype and contributes to the phenomenon of transmission ratio distortion.

In particular, the expression products relating to a (poly)peptide are preferred. This embodiment therefore comprises the (poly)peptides as shown in any of the sequences listed as SEQ ID NOs: 19 to 22, referring to mouse Tagap1 ^{t1-t4}, respectively, and SEQ ID NO: 28, relating to mouse Fgd2.

The conditions and characteristics described in the description of the present invention for the nucleic acid molecules as used in connection with the method of the present invention are to be considered also applicable to the conditions and characteristics described in the description of the present invention for the nucleic acid molecules of the invention, and vice versa.

The present invention further relates to a method for the identification of a nucleic acid molecule encoding an expression product with a Distorter function, comprising the steps of (a) isolating a nucleic acid molecule encoding a candidate expression product with a Distorter function from the mouse t-complex by means of genomic localization, wherein said nucleic acid molecule is involved in G protein signalling; and (b) testing the nucleic acid molecule isolated in step (a) for a change of the transmission ratio of the Responder or of a genetic trait linked to a Responder in an experimental non human animal, wherein when said transmission ratio is enhanced or reduced, said nucleic acid molecule isolated in (a) is a nucleic acid molecule encoding an expression product with Distorter function.

The above method is exemplified, inter alia, in Example 4 for Fgd2. The methods described in detail in Example 4 provide an ideal example how a Distorter can be identified and verified in vivo by genetic testing of a null allele.

The present invention relates in addition to a method for the identification of an expression product of a nucleic acid molecule encoding a Distorter, comprising the steps of (a) isolating an expression product of a nucleic acid molecule encoding a candidate Distorter by means of protein-protein interaction with a known Distorter derived from the mouse t-complex; and (b) testing the nucleic acid molecule encoding said expression product isolated in (a) for change of the transmission ratio of the Responder or of a genetic trait linked to a Responder in an experimental non human animal, wherein when said transmission ratio is enhanced or reduced, said expression product isolated in (a) is an expression product with Distorter function.

It is preferred that in step (b) of the above identification methods, hypomorphic or hypermorphic alleles of said nucleic acid molecule are used for testing for change of the transmission ratio.

It is envisaged that (a) (poly)peptide(s) binding to a known Distorter (poly)peptide is/are identified by co-immunoprecipitation of protein complexes involving the Distorter (poly)peptide, or by affinity chromatography purification of a protein binding to said Distorter polypeptide or a part thereof, or by other methods allowing purification and analysis of protein complexes such as mass spectrometry, and subsequent cloning of the corresponding genes encoding the proteins binding to said Distorter (poly)peptide, or by Two Hybrid Screening techniques in yeast employing standard technology (Chien, Bartel et al. 1991).
Genetic testing in transgenic animals for the ability of the Distorter candidate identified by the methods described above to enhance or reduce the transmission ratio of the Responder can be performed using, for example, hypomorphic or amorphic or hypermorphic alleles of said Distaorter candidate, which are constructed for example by introduction of (a) nucleic acid molecule(s) expressing a shRNA directed against said Distorter candidate, or by targeting the nuclear gene locus of said Distorter candidate thereby inactivating the gene function, or by introducing a construct expressing the wild type Distorter candidate thereby increasing the dosage of the expression products of said Distorter candidate.

### The figures show:

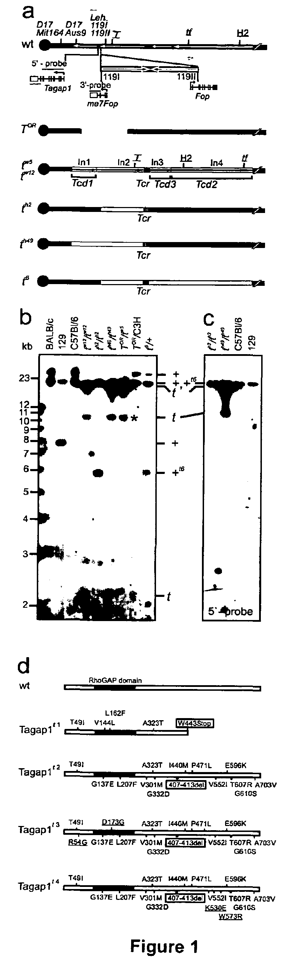

**Figure 1** *Tagap1* is a candidate for *Tcd1.* **a**, Schematic map of the *t*-complex on chromosome 17. The approximate positions of *Tagap1, Fop* and the fusion gene *me7Fop* are indicated, gene maps are expanded. The centromere is indicated by a filled circle, wild-type chromatin by filled bars, t-chromatin by open bars, inversions (In1-In4) by arrows. The molecular and genetic markers, and the structure of partial *t-*haplotypes have been described previously (Lyon 1984). The approximate extent of the deletion in *T^{OR}* is indicated by a gap, positions of *Tcd*s by brackets, *Tcr* by a hatched box. Maps are not to scale. **b**, Genomic mapping of *me7Fop* and *Tagap1* to the *Tcd1* region in *t*-haplotypes. The 3'-probe of *me7Fop* detects two wild-type bands, which are polymorphic in different strains and in *t⁶*, and three *t*-specific bands, which are absent from *T^{OR}* and not observed in *t⁶*, localizing both genes to the *Tcd1* interval. **c**, *Tagap1* maps to the *Tcd1* region and is amplified in *t*-haplotypes. A *Tagap1-*specific 5'-probe detects two polymorphic fragments in *t^{h49}*/*t^{h49}* DNA equalling four-fold the signal intensity of the wild-type band, as determined by quantification. **d**, *t*-haplotypes encode four different classes of *Tagap1* transcript. Schematic representation of representative cDNA clones isolated from testis of *t^{h49}*/*t^{h49}* mutant animals, in comparison to the wild-type gene. Mutations resulting in deletion of amino acid residues are boxed, mutations distinguishing products derived from different *Tagap1* loci are underlined.

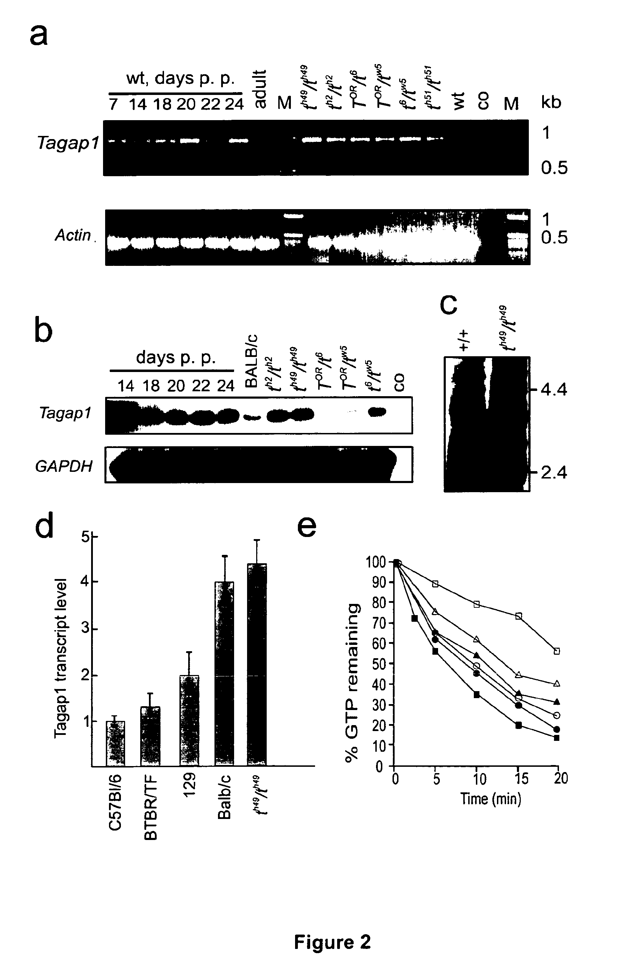

**Figure 2** Expression analysis and GAP-activity assays of *Tagap1.* **a**, *Tagap1* is already expressed at early stages of spermatogenesis. RT-PCR analysis of RNA isolated from various postnatal (p.p.) testes, and from testes of adult wild type mice or males carrying various *t*-haplotypes; Actin served as control. **b**, RNAse protection assay confirming the RT-PCR data. **c**, Northern blot analysis of 8 µg poly(A+)RNA hybridized with the *Tagap1-*specific 5'-probe. The *t*-specific mRNA migrates faster than the wild type. **d**, Analysis of *Tagap1* transcripts by quantitative PCR reveals up to four-fold higher levels of *Tagap1* transcript in *t^{h49}* as compared to wild type strains. **e**, Tagap1 enhances the GTPase activity of RhoA. Squares, RhoA; triangles, cdc42; circles, Rac1; open symbols, reaction carried out without Tagap1; filled symbols, with Tagap1. Abbr.: +, wild type; p.p., days post-partum.

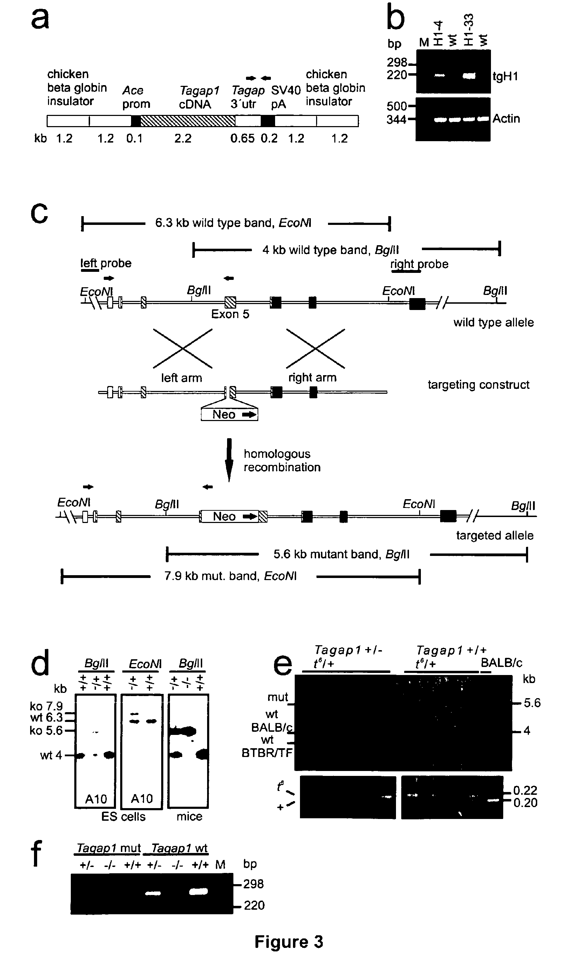

**Figure 3** Construction of gain- and loss-of-function alleles of *Tagap1.* **a**, Transgenic construct used for over-expression of wild type *Tagap1.* Black arrows indicate primers for genotyping. **b**, RT-PCR analysis of testis RNA verifying expression of the transgenic constructs. Actin served as positive control. Abbr.: H1-4, *Tg(Tagap1)H1-4Bgh;* H1-33, *Tg(Tagap1)H1-33Bgh.* **c**, Targeting of the *Tagap1* gene. Introns are depicted as double lines, exons as boxes; coding regions are hatched, GAP-domain encoding regions filled. A PGKneo selection cassette was integrated into exon 5. Primers used for expression analysis in (**e**) are indicated by black arrows. **d**, Identification of the targeted allele *Tagap1^{tm3Bgh}* in clone A10. Genomic Southern blot analysis identifies the predicted size *BglII* and *EcoNI* fragments, detected with the right and left probe, respectively, in the ES-cell clone A10. Right panel: genotyping of a heterozygous and a homozygous mutant male, confirming germ line transmission of the mutant allele. **e**, Genotyping of males used for testing the effect of the *Tagap1* knock-out allele on the transmission ratio of the *t⁶*-haplotype. **f,** RT-PCR analysis of testis RNA from wild type (+/+), heterozygous (+/-) and homozygous (-/-) mutant animals with primers specific for the mutated (left part) and wild type allele (right part), demonstrating loss of the wild-type *Tagap1* transcript in -/- animals.

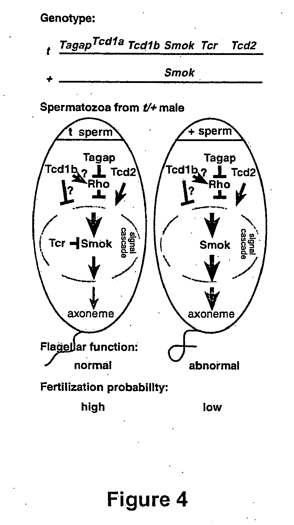

**Figure 4** Model of the role of *Tcd*s and *Tcr* in transmission ratio distortion. *t-*haplotypes encode several *Tcd*s *(Tcd1^{Tagap1}, Tcd1b, Tcd2* are indicated, wild type alleles not shown) and *Tcr* acting upstream of Smok kinase controlling flagellar behaviour. Tagap1 is a negative regulator of a Rho family member, which inhibits Smok. Tagap1^{Tcd1a} (Tagap1) enhances down-regulation of Rho, resulting in up-regulation of Smok. *Tcd1b* is a hypomorphic or amorphic allele of an activator of Rho or of an inhibitor of Smok, further enhancing Smok activity epistatically to Tagap1. Likewise, Tcd2 further promotes up-regulation of Smok. All sperm (*t* and +) produced by *t*/+ males are affected by Tcds, which act in *trans.* This negative effect of Tcds is counter-balanced by Tcr, which is restricted to *t*-sperm and thus rescues *t*-sperm only. This results in an advantage of *t*-sperm in fertilizing the eggs and promotes the transmission of the t-haplotype to the offspring. For details see text.

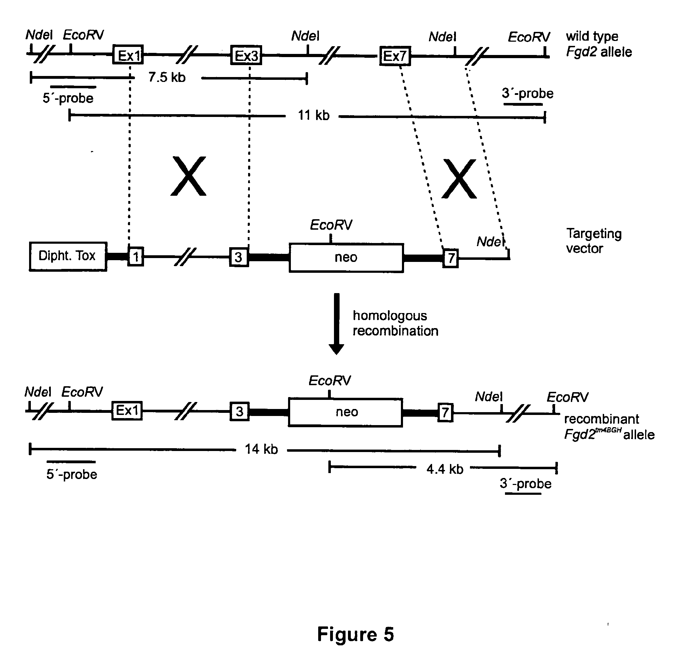

**Figure 5** Targeting of the mouse *Fgd2* gene by homologous recombination. The targeting vector was constructed by ligation of the left and right homology arm, both derived by PCR amplification of genomic DNA, to the vector pDT/pGKneoflox 3xpA (see Methods). Out of 132 clones analyzed, 1 displayed the expected RFLP with the 5'-probe (7.5 kb for the wild type allele and 14 kb for the targeted allele) and 3'-probe (11 kb versus 4.4 kb for the wild type allele and mutant allele respectively) demonstrating successful targeting of the genomic locus by homologous recombination.

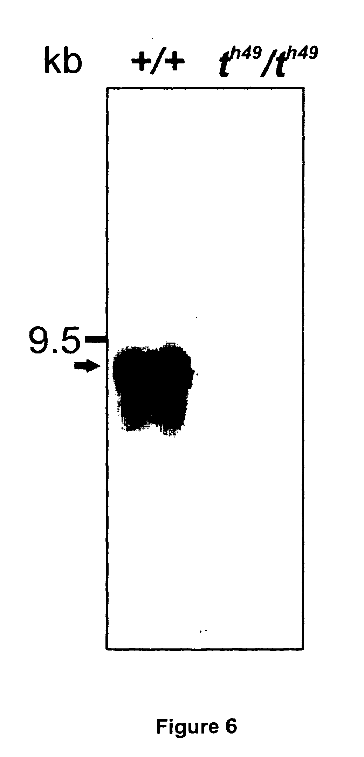

**Figure 6** Northern blot analysis of 8 µg poly(A+)RNA hybridized with a Tiam2 specific probe. The Tiam2 specific band, observed in wild type testis RNA is not detected in *t^{h49}*/*t^{h49},* indicating, that the gene is not expressed in the t-haplotype

The examples illustrate the invention.

### Material and Methods

### Mice and genetics

For mapping of *Tagap1* on genomic DNA the following *t*-haplotypes were used: *t^{w12}*/*t^{w12} t^{h2}*/*t^{h2}, t^{h49}*/*t^{h49}, t^{h51}*/*t^{h51}, T^{OR}*/*t^{w5}, T^{OR}*/*t⁶,* t⁶/+. The *t*-haplotypes *t^{w12}, t^{h49}, t^{h51}* and *t^{w5}* carry *Tcd1,* while *t⁶* and *t^{h2},* which is derived from *t⁶*, lack *Tcd1* activity. Southern blot analysis of *Kpn*I digested genomic DNA was performed by standard procedures (Church and Gilbert 1984) using a fragment corresponding to position 942-3001 of the *Tagap1* cDNA as 3'-probe, and position 124-942 as 5'-probe. Genotyping of mice for the transmission of *t*⁶ was done by PCR using primers for the marker *Hba-4ps.* Transgenic lines were generated in the inbred strain FVB/N by pronuclear injection of construct DNA using standard procedures. The *Tagap1^{tm3Bgh}* allele was generated in Balb/c ES-cells obtained by B. Ledermann (Basel Institute for Immunology) (Dinkel, Aicher et al. 1999). Also other ES cells known to the person skilled in the art, however, can be used. Transgenic and knock-out lines were back crossed several generations to the strain BTBR/TF-+*tf*/+*tf* before testing for Distorter activity.

### Transcript analysis

The 5'ends of *me7Fop* and *Tagap1* were obtained by 5'-RACE using the GeneRacer kit (Invitrogen). Standard reverse transcription was performed with 1 microgram of total RNA using AMV-RT (Promega). RNAse protection assays were done using standard procedures (Gilman 1997). The *Tagap1* probe was synthesized in vitro in the presence of [³²P] UTP from a fragment derived from the Tagap1 cDNA by PCR (sense 5'-GACTCCTAGGGTCAGAGTGTCATG-3', antisense 5'-TGGGCTCCACATCTGGGTCATT-3') cloned in pCRII TOPO (Invitrogen). The *GAPDH* control RNA was transcribed from the pTRI-GAPDH template (Ambion). Northern analysis was performed by standard techniques (Sambrook J. 1989) using 8 µg of single purified poly(A+)RNA using the Fast Track system (Invitrogen). Quantitative PCR analysis was carried out on an ABI PRISM 7900 HT SDS (Applied Biosystems) using the TaqMan probe 5'-ATCCTCTGCCTTAAAGGTCCTTCAACGGAA-3' (5' FAM and 3' TAMRA labeled) and primers sense 5'-CCAGACCCATCCAGGACATC-3' and antisense 5'-CTGGCAGCTTTCCTGAATATC-3'. As a reference, GAPDH expression was determined using the mouse GAPDH assay (Applied Biosystems).

### Gene targeting and transgene constructs

The Tagap1 targeting vector was constructed by ligation of the left and right arm, both derived by PCR amplification of genomic DNA, to the PGK-neo cassette. Culture, electroporation, selection, isolation of ES-cell clones, DNA preparation in 96 well plates and Southern blot analysis were done according to standard procedures (Ramirez-Solis, Davis et al. 1993). The transgenic construct *Tg(Tagap1)H1Bgh* consists of the *Angiotensin Converting Enzyme* (*ACE*) testis promoter and transcription start (extending from -91 to +17 bp), driving expression in elongating spermatids (Morita, Murata et al. 1993), followed by the complete ORF of wild-type *Tagap1,* its 3'UTR and the SV40 polyadenylation signal derived from the vector pCS2+ (Rupp, Snider et al. 1994), replacing the *Tagap1* polyA-signal sequence. This transcription unit is flanked by tandem copies of the chicken-globin insulator (Chung, Whiteley et al. 1993). Transgenic animals were identified by PCR using the primers: sense 5'-AGGGCCCTTGGGGTCAGG-3', antisense 1 5'-CTGTCAGTCTCCATTCCAATGAAG-3' and antisense 2: 5'-CAGTTAGCTGGCAAATGCTGTC-3'. The wild-type band is 541 bp in length, the transgenic construct produces bands of 165 bp and 266 bp.
For gene targeting of the Fgd2 locus, a fragment containing the PGK-promoter driven neomycin resistance gene flanked by IoxP sites was isolated from the vector pPGKneo Floxl (gift of Moises Mallo) by *EcoRV*/ *EcoR*I digest and ligated into the *EcoRV*/ *EcoR*I digested pDT Bluescript vector (provided by Achim Gossler), which contains the diphteria toxin-A chain coding sequence under the control of RNA polymerase II promoter. pDT/pGKneoflox 3xpA was digested with *Not*I, filled in, cut *Xbal* and ligated to the left homology region, which was obtained by PCR amplification of genomic DNA using primers s: 5'-ACTAGTCTGCTTCTGGGGTAACT -3' containing a *Spe*l site and as: 5'-ATAGGCCTGCTCCGTCT -3' followed by digestion with Spel. The obtained construct was digested *EcoRV*/ *Sal*l and the right homology region, obtained by PCR on genomic DNA with primers s (*EcoRV):* 5'-GATATCAAGAATCCCGCGGTACGAACTG -3' and as (*Sal*l*:* 5'-GTCGACGACAACGCCCGACATCATAGAG -3' and cut with *EcoR*V and *Sal*l was ligated into this vector. The resulting targeting vector was linearized by restriction digest with *Sal*I. Establishment of a BTBR/TF-ES cell line, culture, electroporation, selection, isolation of ES-cell clones, DNA preparation in 96 well plates and Southern blot analysis was done according to standard procedures. The left probe (LP) and right probe (RP) for Southern blot analysis were generated by PCR with primers LPs 5'- ACAGGTCTCACGTAGCCGAATC -3', LPas 5'-CGGGTGAAGCAGGTCTACCACA -3' and RPs 5'-TGGATGCCGCTCAGTTGCTAAT -3',RPas 5'- TGAAACTCAGTGTGTAGACCAG - 3'respectively.

### In vitro GAP assays

The catalytic domain of wild type Tagap1, small G proteins and the C-terminal polypeptide of Tagap1, the latter serving as negative control, were produced as GST-fusion proteins in *E. coli* BL21 using the pGEX vectors as described (Frangioni and Neel 1993) (Self and Hall 1995). For quantification of relative amount of proteins used, all preparations were adjusted relative to a BSA standard. GAP assays were performed in triplicate (G proteins at 6 nM; Tagap1: 15 nM) essentially as published (Self and Hall 1995).

### Example 1: Isolation of a candidate gene for Tcd1

We have used a positional cloning approach to identify a candidate for *Tcd1,* based on the following criteria: 1) The gene must be located in the genomic interval comprising *Tcd1,* 2) it must be expressed in testis, 3) show alterations in the *t-*haplotype form vs. the wild type, and 4) should encode a protein involved in signalling. The latter criterion was based on our proposal that *Tcd*s encode components of signalling cascades acting upstream of Smok (Herrmann, Koschorz et al. 1999).
Since chromosomal rearrangements have a high potential of affecting gene function we started our search for *Tcd* candidates in the region *D17Leh119I*, which marks the end of a large inverted duplication in the wild type chromosome (Herrmann, Barlow et al. 1987). Genomic fragments, spanning the duplication breakpoint were hybridised to a cDNA library and a gene, designated *me7Fop*, showing similarity with *FGF receptor oncogene partner (Fop)* was identified (Fig. 1 a). Northern analysis showed that this gene is highly expressed in wild type testis, whereas no transcripts are detectable in testes from males carrying the *Tcd1* region in the *t*-haplotype form (e.g. *t^{h51}*, *t^{h49}* or complete *t*-haplotypes). A detailed analysis of the gene structure showed that the 5'-region of *me7Fop* is derived from *Fop*, while most of the coding region and the 3'-untranslated sequence come from an unrelated gene.
The part of *me7Fop,* which is not derived from *Fop* occurs in a second locus on the wild type chromosome. This is shown by genomic mapping using the 3'-region of *me7Fop* as probe (Fig. 1b). In wild type genomes two bands are detectable, whereas *t*-haplotypes produce three polymorphic *t*-specific bands. Both wild type fragments are missing in the deletion chromosome *T^{OR}* since genomic DNA from a *T^{OR}*/*t^{w5}*animal shows only the *t*-specific bands. This data maps both gene fragments to the proximal t-haplotype.
The analysis of the partial *t*-haplotype *t⁶* allows a more accurate assignment of both genes to the region harbouring *Tcd1.* The *t*-haplotype *t⁶* and its derivative *t^{h2}* have lost *Tcd1* activity by a recombination event in which the proximal portion of the *t*-haplotype has been exchanged for wild type DNA (Lyon 1984). Thus *Tcd1* is located in the region, which is wild type in *t⁶.* The Southern blot analysis revealed no *t*-specific band in *t⁶* or *t^{h2}*, but a wild type and a *t⁶*-specific band. Accordingly, both gene fragments map to the *Tcd1* region (Fig. 1a). We mutated *me7Fop* by gene targeting and analysed its possible role as Distorter of the transmission ratio. Distorter activity was not observed excluding *me7Fop* as candidate for *Tcd1.*
We extended the analysis to the gene from which *me7Fop* was derived. 5'-RACE protocols and database searches were utilized to obtain a complete cDNA clone. Sequence analysis showed that it encodes a protein of 714 amino acid residues involving a domain with high similarity to GTPase-activating proteins (GAP) for Rho small G proteins (Fig. 1d).
In the course of our studies this gene appeared in public databases as *T-cell activation Rho GTPase-activating protein (Tagap1,* accession number NM_145968). Genomic Southern blot analysis using a *Tagap1*-specific 5'-fragment of the cDNA as probe showed that wild type strains contain a single band, which is also present in *t⁶*/+ DNA. In contrast, genomic DNA derived from t-haplotypes showed two stronger polymorphic bands (Fig. 1c). This polymorphism confirms the mapping of *Tagap1* to the *Tcd1* region. Quantification of the wild-type and the *t*-specific signals revealed that the *Tcd1*-bearing *t*-haplotypes harbour four *Tagap1* loci, while the wild-type genome contains a single complete *Tagap1* gene. This result was confirmed by quantitative PCR on genomic DNA using *Tagap1* specific primers. Hybridisation with the 3'-probe, which detects *me7Fop* and *Tagap1*, and quantification of the bands revealed two-fold higher signal intensity in genomic DNA from *t^{h49}*/*t^{h49}* mice compared to *t^{h2}*/*t^{h2}* mice or wild type strains.
In accordance with multiple genomic copies of *Tagap1* on the t-haplotype, sequencing of *Tagap1* cDNAs derived from testis of *t^{h49}*/*t^{h49}* males revealed several types of transcripts with multiple non-silent nucleotide changes compared to the wild type sequence (Fig. 1d). The major cDNA class (51 out of 74 clones analysed), derived from *Tagap1^{t1}* contains a transition of G to A at codon 433, turning a tryptophane (TGG) into a premature stop codon (TGA). This mutation truncates the predicted protein, leaving the N-terminal RhoGAP domain intact. Two additional mutations (V144L, L162F) were found in the RhoGAP domain of Tagap1^{t1}, and two (T491, A323T) outside of this region. None of these alterations were found in transcripts derived from wild-type strains, nor in the partial t-haplotypes lacking *Tcd1,* in *t⁶* and *t*^{*h*2}.
The remaining three *t*-specific Tagap1 cDNAs do not contain the W443X mutation. Instead, a number of non-silent point mutations and a 21 base pair deletion 3' to the RhoGAP domain were detected in these clones (Fig. 1d). Two alterations in the RhoGAP domain (G137E, L270F) are shared by all three genes, while *Tagap1^{t3}* differs from *Tagap1^{t2}* and *Tagapl^{t4}* by one additional mutation (D173G) in this domain. Aside from shared alterations, these cDNAs also show differences distinguishing them from each other, suggesting that they are derived from three distinct *Tagap1* genes, which arose by triplication of a single locus.
The combined genomic and cDNA sequence data demonstrate that *t-*haplotypes contain four *Tagap1* loci in the *Tcd1* region, while the wild type has two, one complete (*Tagap1*) and one altered gene *(me7Fop),* which has lost the GAP-domain due to a rearrangement. The fact that the *t*-alleles of *Tagap1* are altered with respect to the wild type is consistent with the criteria for a *Tcd* candidate.
RNA expression analysis by RT-PCR and RNAse protection assays showed that *Tagap1* is transcribed in the testis already at the earliest stage analysed, day 7 *post partum* (Fig. 2 a,b). Thus *Tagap1* is expressed already in diploid spermatocytes, which may be conducive to distribution of the gene products to all sperm cells, since spermatids develop in a syncytium. Northern blot analysis using poly(A+) RNA suggested low level transcription in this organ (Fig. 2c). In situ hybridisation analysis on testis sections using a *Tagap1* specific probe did not produce distinct signals.
The transcript detected in the t-haplotype shows a slightly faster migration compared to the wild type mRNA (Fig. 2c). The reason for this is unclear, as no major size differences (except for the 21-base deletion) were observed in any of the *t*-specific cDNA clones analysed. It is conceivable that poly-adenylation differences may account for the smaller transcript size. Shortening of the poly-A tail has been shown to accompany translational activation of some mRNAs during spermiogenesis (Kleene 1989). Whether or not the observed difference has a functional relevance, however, remains to be determined.
*Tagap1* transcripts were detected in all organs examined by RT-PCR analysis, and *Tagap1* ESTs have been reported in public databases from a large variety of tissues and organs, suggesting that the gene is ubiquitously expressed.
Quantitative RT-PCR showed that *t*-haplotypes express up to four fold higher levels of *Tagap1* transcripts than wild-type strains (Fig. 2d). This finding suggests that sperm derived from t/+ males on different wild type backgrounds may produce substantially different levels of Tagap1 protein. This may have a profound effect on the transmission ratio of the *t*-haplotype, consistent with earlier reports that the genetic background has an important impact in transmission ratio distortion (Gummere, McCormick et al. 1986).
Finally we examined the specificity of the GAP-domain of wild type Tagap1 towards three "classical" small GTPases, RhoA, cdc42 and Rac1, which are, among several others, expressed in testis (Wennerberg and Der 2004). G proteins act as molecular switches, which transmit a signal in their active, GTP-bound form, whereas they become inactive after GTP hydrolysis. GAPs enhance the intrinsic GTPase activity of small G proteins, promoting their inactive state. Our data show that the GTPase activity of RhoA was strongly enhanced by the GAP domain of Tagap1, whereas the other family members were only mildly (Cdc42) or hardly (Rac1) stimulated, identifying RhoA as a possible in vivo target of Tagap1 (Fig. 2e).

### Example 2: Tagap1 distorts the transmission ratio of t-haplotypes

Since the genetic and molecular data suggested that the *t*-loci of *Tagap1* might cause a gain-of-function phenotype, we tested whether over-expression of wild-type *Tagap1* in elongating spermatids, from a transgene construct controlled by the testis-specific ACE promoter, would alter the transmission ratio of the partial *t*-haplotype *t⁶* lacking *Tcd1* (Fig. 1a, 3a) (Howard, Balogh et al. 1993). Two independent transgenic lines harbouring the construct and expressing the transcript were generated, crossed into males carrying *t⁶* and analysed (Fig. 3b, Table 1). In both lines, a significant increase of the transmission of the *t⁶*-haplotype to the offspring was observed, as compared to non-transgenic litter mates (combined data: 88% vs. 80% *t⁶* offspring, p<0.01). Thus, a dosage increase of wild type *Tagap1* in testis phenocopied a *t-complex Distorter,* consistent with the idea that the *t*-loci of *Tagap1* encode *Tcd1* activity.
To create a loss-of-function allele of *Tagap1,* we disrupted the gene by inserting a selection cassette into exon 5, resulting in premature termination of the transcript (Fig. 3 c,d,f). Accordingly, the translation product is predicted to be truncated upstream of the RhoGAP domain. This allele, termed *Tagap^{tm3Bgh}* (in accordance with standard nomenclature) was bred in *trans* to the partial *t*-haplotype *t⁶.* Litter mates carrying either the wild type allele or *Tagap^{tm3Bgh}* in trans to *t⁶* were tested for transmission ratio distortion (Fig. 3e, Table1). Complementary to the transgenic experiments, the transmission ratio of *t⁶* from *Tagap^{tm3Bgh}*/*+; t⁶*/*+* males was strongly reduced compared to the ratio obtained from *Tagap1* +/+; *t⁶*/+ litter mates (69% vs. 84% *t⁶* offspring; Table 1). Statistical analysis demonstrated that the difference is highly significant (p < 0.001). Thus, the loss of function experiment confirmed the results of the gain-of-function experiment, directly demonstrating a role of *Tagap1* in transmission ratio distortion.
Taken together, the genetic and molecular data strongly suggest that the *t-*haplotype loci of *Tagap1* represent *Tcd1.* From the transgenic gain-of-function phenotype one would conclude that these loci act as dominant gain-of-function mutation. Consistent with this conclusion is the fact the *Tagap1* gene is amplified in t-haplotypes and that the four loci together express up to fourfold more transcript in testis than wild type strains. The inactivation (in terms of GAP activity) of one of the originally two *Tagap1* genes by a chromosomal rearrangement in the wild type may have had a selective advantage over the progenitor chromosome since it decreased the overall Tagap1 activity in t/+ heterozygotes, possibly "defending" the wild type chromosome better against the disadvantageous hyperactivity caused by the *t-*specific *Tagap1* loci.

It has been shown that a large deletion of the wild type chromosome, *T^{22H},* phenocopies *Tcd1,* which led to the suggestion that *Tcd1* represents a hypomorphic or amorphic allele (Lyon 1992). In contrast, we demonstrated that a hypermorphic allele of *Tagap1* phenocopies a *t-complex Distorter* located in the *Tcd1* region. This discrepancy could be reconciled by recent data suggesting the existence of two separate loci, *Tcd1a* and *Tcd1b,* which are both lacking from *T^{22H}* (ref. (Lyon, Schimenti et al. 2000)). Moreover, the distortion of the t⁶ transmission ratio caused by the gain- and loss-of-function alleles of *Tagap1* is considerably lower than that expected for *Tcd1* encoded by the partial *t*-haplotype *t^{h51}* (8-15% for *Tagap1* vs. >27% for *t^{h51}* ; ref. (Lyon 1984)). Though some of this difference may be accounted for by variation in the genetic background or by a stronger effect of the *Tagap1* loci in the t-haplotype, our data support the identification of two *Tcd1* loci.
We suggest that the *t-Tagap1* loci, in accordance with their map position on the chromosome, encode *Tcd1a,* and should be named *Tagap1^{Tcd1a}* (according to nomenclature rules). The contribution of each of the four loci and the exact mechanism by which they produce a hypermorph remains to be explored in detail.
In accordance with the finding of two *Tcd1* loci and the data shown here, we predict that the second locus, *Tcd1b*, represents a hypomorphic or amorphic allele of a gene acting upstream of or epistatically to the G protein controlled by *Tagap1.*

### Example 3: Model of the role of Tagap1 in transmission ratio distortion

Whereas the applicant does not wish to be bound by any theory, the following model of the role of Tagap1 is envisaged.

We have previously proposed that the *Tcd*s act upstream of *Smok* (Herrmann, Koschorz et al. 1999). According to our model, Smok activity is enhanced through the action of *t*-Distorters in all spermatozoa derived from *t*/+ males resulting in abnormal flagellar function. This negative effect of the *t*-Distorters is counterbalanced by Tcr, which is restricted to cells expressing the gene, thus rescuing *t*-sperm, while +-sperm remain dysfunctional. This would then lead to an advantage of the *t*-sperm in fertilising the eggs.
The findings presented here allow us to refine this model (Fig. 4): Tagap1 down-regulates a member of the Rho subfamily of small G proteins, which acts as negative regulator of Smok kinases. Tagap1^{Tcd1a} enhances down-regulation of this Rho GTPase, which leads to indirect up-regulation of Smok. We predict that the wild type allele of *Tcd1b* encodes either an activator of the Rho protein controlled by Tagap1, or an indirect inhibitor of Smok acting through another factor. If the *t-*haplotype allele *Tcd1b* represents a hypomorphic or amorphic allele, as suggested, this mutation would cause a reduction of the Tcd1b protein level indirectly resulting in up-regulation of Smok. Tcd1b would thus act additively or synergistically with and epistatically to *Tagap1^{Tcd1a}.* Tcd2 would further enhance this effect. Only *Tcr* expressing spermatozoa would be rescued from the motility defect caused by *Tcd*s*,* resulting in preferential fertilisation of the eggs by *t*-sperm. Of course, at this point of analysis, other models are conceivable.
Tagap1 for the first time links Rho signalling to transmission ratio distortion. Previous reports have provided evidence for a role of Rho-GTPases in sperm motility, and the Rho binding protein Rhophilin and its interaction partner Ropporin are found in the flagellum(Hinsch, Habermann et al. 1993)·(Nakamura, Fujita et al. 1999)·(Fujita, Nakamura et al. 2000). Rhophilin is localised on the outer surface of the outer dense fibers of the sperm tail, directly opposing Ropporin, which is localised at the inner surface of the fibrous sheath (Fujita, Nakamura et al. 2000).
Rho-GTPases are well known for their essential role in cell motility and chemotaxis, which has been extensively studied in human neutrophils, fibroblasts and in the slime mould *Dictyostelium discoideum* (Van Haastert and Devreotes 2004). In these cell types Rho-GTPases control repeated extension of pseudopodia at the leading edge in response to a shallow gradient of signalling molecules, enabling the cell to move towards the stimulus. Whether these analogous functions of Rho-GTPases in cell motility in neutrophils and in sperm motility are part of a common mechanism, despite the fact that the former involves actin and myosin fibers while the latter involves microtubuli remains to be explored.
The identification of a *t-complex-Distorter* provides access to understanding the molecular principles of transmission ratio distortion and promotes the investigation of the role of Rho signalling in sperm motility.

### Example 4: Fgd2 is a candidate for Tcd2

The identification of Tagap11 as Distorter of the transmission ratio of t-haplotypes for the first time demonstrated an important role of small GTPases (preferentially of the Rho type) in transmission ratio distortion. This finding led us to suggest that other proteins involved in G protein signalling might also play a role in TRD. Therefore, the genomic region comprising the t-haplotype was searched with bioinformatics tools for genes encoding proteins of this group. Several were identified, among them *Fgd2,* encoding for a protein containing a Dbl homology domain and belonging to the GEF (guanine nucleotide exchange factor) family of proteins. *Fgd2* is located in the distal portion of the t-haplotype, the In4 region. *Fgd2* cDNA fragments were isolated by RT-PCR from testis of a male carrying the t-haplotypes *t⁶*/*t^{w5}.* Sequence analysis demonstrated a number of mutations in the *t*-specific transcript with respect to the wild type transcript suggesting *Fgd2* as candidate for Tcd2.
On the basis of these data, involvement in G protein signalling, location in the *t-*haplotype region, expression in testis and modification of the coding region in the *t-*allele, Fgd2 was genetically analysed with respect to enhancement or reduction of the transmission ratio of the Tcr carrying t-haplotype *t^{h49},* which lacks Tcd2. We engineered a targeted (loss-of-function) allele of *Fgd2, Fgd2^{tm4Bgh},* in which the Dbl homology domain was inactivated, in embryonic stem cells as described, and crossed into animals carrying *t^{h49}.* Males heterozygous for *Fgd2^{tm4Bgh}* and for *t^{h49}* were tested for the transmission ratio of *t^{h49}* to the offspring. Litter mates heterozygous for *t^{h49}* and wild type at the *Fgd2* locus served as control. The breeding results demonstrate a significant reduction of the transmission ratio of t^{h49} from males carrying *Fgd2^{tm4Bgh}* as compared to litter mates of genotype *t^{h49}*/*+*;+/+. This result as shown in Table 2 and Figure 5 clearly demonstrates that *Fgd2* is involved in transmission ratio distortion and a candidate for Tcd2.
Thus the teachings of Tagap11 led us to the discovery of another Distorter, Fgd2.

### Example 5: Tiam2 is a candidate for Tcd1b

Another candidate for a *t*-Distorter was identified with bioinformatics tools in the Tcd1 subregion of the *t*-haplotype. This region contains another member of the family of Dbl homology domain proteins, *Tiam2.* Thus, this gene also belongs to the GEF family of proteins involved in G protein signalling. Primary characterization of the *Tiam2* transcripts in *t*-haplotypes failed to identifiy a *t*-specific transcript in testis using the sensitive RT-PCR technology, suggesting that the *t*-allele of *Tiam2* is not transcribed in testis. This assumption was confirmed by Northern analysis, which failed to identify a transcript of the expected size in RNA derived from the testis of a *t^{h49}*/*t^{h49}*male, whereas a strong band was detected in testis RNA of a wild type control male (Figure 6). Thus, the *t*-haplotype appears to carry a loss-of-function allele of *Tiam2,* strongly suggesting *Tiam2* as candidate for a second Distorter in the Tcd1 region. According to our model of TRD (see example 3), *Tiam2* is a candidate for Tcd1b.

### References

Balch, W. E., Der, C.J., Hall, A. (editors) (1995). "Small GTPases and Their Regulators." Methods in Enzymology 256**.**
Bishop, A. L. and A. Hall (2000). "Rho GTPases and their effector proteins." Biochem J 348 Pt 2: 241-55.
Bullard, D. C., C. Ticknor, et al. (1992). "Functional analysis of a t complex responder locus transgene in mice." Mamm Genome 3(10): 579-87.
Cassiday, L. A. and L. J. Maher, 3rd (2003). "Yeast genetic selections to optimize RNA decoys for transcription factor NF-kappa B." Proc Natl Acad Sci U S A 100(7): 3930-5.
Chapman, S. C., A. Lawson, et al. (2005). "Ubiquitous GFP expression in transgenic chickens using a lentiviral vector." Development 132(5): 935-40.
Chien, C. T., P. L. Bartel, et al. (1991). "The two-hybrid system: a method to identify and clone genes for proteins that interact with a protein of interest." Proc Natl Acad Sci U S A 88(21): 9578-82.
Chung, J. H., M. Whiteley, et al. (1993). "A 5' element of the chicken beta-globin domain serves as an insulator in human erythroid cells and protects against position effect in Drosophila." Cell 74(3): 505-14.
Church, G. M. and W. Gilbert (1984). "Genomic sequencing." Proc Natl Acad Sci U S A 81(7): 1991-5.
DePamphilis, e. W. P. M. M. L. (1993). "Guide to techniques in mouse development." Methods in Enzymology, Academic Press NY, 1993. Vol. 225: .
Der, C. J., Hall, A. (2000). "Regulators and effectors of small GT Pases." Methods in Enzymology. Vol.325**.**
DerMardirossian, C. and G. M. Bokoch (2005). "GDIs: central regulatory molecules in Rho GTPase activation." Trends Cell Biol 15(7): 356-63.
Dinkel, A., W. K. Aicher, et al. (1999). "Efficient generation of transgenic BALB/c mice using BALB/c embryonic stem cells." J Immunol Methods 223(2): 255-60.
Donovan, S., K. M. Shannon, et al. (2002). "GTPase activating proteins: critical regulators of intracellular signaling." Biochim Biophys Acta 1602(1): 23-45.
Dvorsky, R. and M. R. Ahmadian (2004). "Always look on the bright site of Rho: structural implications for a conserved intermolecular interface." EMBO Rep 5(12): 1130-6.
Fox, H. S., G. R. Martin, et al. (1985). "Molecular probes define different regions of the mouse t complex." Cell 40(1): 63-9.
Frangioni, J. V. and B. G. Neel (1993). "Solubilization and purification of enzymatically active glutathione S-transferase (pGEX) fusion proteins." Anal Biochem 210(1): 179-87.
Fraser, L. R. and K. Dudley (1999). "New insights into the t-complex and control of sperm function." Bioessays 21(4): 304-12.
Fujita, A., K. Nakamura, et al. (2000). "Ropporin, a sperm-specific binding protein of rhophilin, that is localized in the fibrous sheath of sperm flagella." J Cell Sci 113 ( Pt 1): 103-12.
Geijsen, N., M. Horoschak, et al. (2004). "Derivation of embryonic germ cells and male gametes from embryonic stem cells." Nature 427(6970): 148-54.
Gilman, M. (1997). Ribonuclease Protection Assay, John Wiley & Sons, Inc., New York.
Gummere, G. R., P. J. McCormick, et al. (1986). "The influence of genetic background and the homologous chromosome 17 on t-haplotype transmission ratio distortion in mice." Genetics 114(1): 235-45.
Hames, S. J. H. a. B. D. (1985). ""Nucleic acid hybridization, a practical approach"." IRL Press, Oxford.
Herrmann, B., M. Bucan, et al. (1986). "Genetic analysis of the proximal portion of the mouse t complex: evidence for a second inversion within t haplotypes." Cell 44(3): 469-76.
Herrmann, B. G., D. P. Barlow, et al. (1987). "A large inverted duplication allows homologous recombination between chromosomes heterozygous for the proximal t complex inversion." Cell 48(5): 813-25.
Herrmann, B. G., B. Koschorz, et al. (1999). "A protein kinase encoded by the t complex responder gene causes non-mendelian inheritance." Nature 402(6758): 141-6.
Hinsch, K. D., B. Habermann, et al. (1993). "ADP-ribosylation of Rho proteins inhibits sperm motility." FEBS Lett 334(1): 32-6.
Horiuch, T., C. Emuta, et al. (2002). "Birth of normal calves after intracytoplasmic sperm injection of bovine oocytes: a methodological approach." Theriogenology 57(3): 1013-24.
Howard, T., R. Balogh, et al. (1993). "Sperm-specific expression of angiotensin-converting enzyme (ACE) is mediated by a 91-base-pair promoter containing a CRE-like element." Mol Cell Biol 13(1): 18-27.
Hubner, K., G. Fuhrmann, et al. (2003). "Derivation of oocytes from mouse embryonic stem cells." Science 300(5623): 1251-6.
Joyner, A. L. E. (1993). "Gene Targeting, A Practical Approach." Oxford University Press.
Kim, V. N. (2005). "MicroRNA biogenesis: coordinated cropping and dicing." Nat Rev Mol Cell Biol 6(5): 376-85.
Kleene, K. C. (1989). "Poly(A) shortening accompanies the activation of translation of five mRNAs during spermiogenesis in the mouse." Development 106(2): 367-73.
Kurtz, S. E., K. Esposito, et al. (2003). "Inhibition of an activated Ras protein with genetically selected peptide aptamers." Biotechnol Bioeng 82(1): 38-46.
Lever, A. M., P. M. Strappe, et al. (2004). "Lentiviral vectors." J Biomed Sci 11(4): 439-49.
Lyon, M. F. (1984). "Transmission ratio distortion in mouse t-haplotypes is due to multiple distorter genes acting on a responder locus." Cell 37(2): 621-8.
Lyon, M. F. (1986). "Male sterility of the mouse t-complex is due to homozygosity of the distorter genes." Cell 44(2): 357-63.
Lyon, M. F. (1992). "Deletion of mouse t-complex distorter-1 produces an effect like that of the t-form of the distorter." Genet Res 59(1): 27-33.
Lyon, M. F. (2003). "Transmission ratio distortion in mice." Annu Rev Genet 37: 393-408.
Lyon, M. F., J. C. Schimenti, et al. (2000). "Narrowing the critical regions for mouse t complex transmission ratio distortion factors by use of deletions." Genetics 155(2): 793-801.
Morita, T., K. Murata, et al. (1993). "Mouse t haplotype-specific double insertion of B2 repetitive sequences in the Tcp-1 intron 7." Mamm Genome 4 (1): 58-9.
Nadeau, J. H., D. Varnum, et al. (1989). "Genetic evidence for two t complex tail interaction (tct) loci in t haplotypes." Genetics 122(4): 895-903.
Nakamura, K., A. Fujita, et al. (1999). "Rhophilin, a small GTPase Rho-binding protein, is abundantly expressed in the mouse testis and localized in the principal piece of the sperm tail." FEBS Lett 445(1): 9-13.
Pirottin, D., L. Grobet, et al. (2005). "Transgenic engineering of male-specific muscular hypertrophy." Proc Natl Acad Sci U S A 102(18): 6413-8.
Planchart, A., Y. You, et al. (2000). "Physical mapping of male fertility and meiotic drive quantitative trait loci in the mouse t complex using chromosome deficiencies." Genetics 155(2): 803-12.
Ramirez-Solis, R., A. C. Davis, et al. (1993). "Gene targeting in embryonic stem cells." Methods Enzymol 225: 855-78.
Rosen, L. L., D. C. Bullard, et al. (1990). "Molecular cloning of the t complex responder genetic locus." Genomics 8(1): 134-40.
Rupp, R. A., L. Snider, et al. (1994). "Xenopus embryos regulate the nuclear localization of XMyoD." Genes Dev 8(11): 1311-23.
Sambrook J., F., E.F., Maniatis, T. (1989). Molecular Cloning, A Laboratory Manual, Cold Spring Harbor Laboratory Press.
Schimenti, J. (2000). "Segregation distortion of mouse t haplotypes the molecular basis emerges." Trends Genet 16(6): 240-3.
Schimenti, J., L. Vold, et al. (1987). "An unstable family of large DNA elements in the center of the mouse t complex." J Mol Biol 194(4): 583-94.
Schmidt, A. and A. Hall (2002). "Guanine nucleotide exchange factors for Rho GTPases: turning on the switch." Genes Dev 16(13): 1587-609.
Schmidt, S., S. Diriong, et al. (2002). "Identification of the first Rho-GEF inhibitor, TRIPalpha, which targets the RhoA-specific GEF domain of Trio." FEBS Lett 523(1-3): 35-42.
Self, A. J. and A. Hall (1995). "Measurement of intrinsic nucleotide exchange and GTP hydrolysis rates." Methods Enzymol 256: 67-76.
Self, A. J. and A. Hall (1995). "Purification of recombinant Rho/Rac/G25K from Escherichia coli." Methods Enzymol 256: 3-10.
Silver, L. M. and D. Remis (1987). "Five of the nine genetically defined regions of mouse t haplotypes are involved in transmission ratio distortion." Genet Res 49(1): 51-6.
Van Haastert, P. J. and P. N. Devreotes (2004). "Chemotaxis: signalling the way forward." Nat Rev Mol Cell Biol 5(8): 626-34.
Wennerberg, K. and C. J. Der (2004). "Rho-family GTPases: it's not only Rac and Rho (and I like it)." J Cell Sci 117(Pt 8): 1301-12.
Wilmut, I., A. E. Schnieke, et al. (1997). "Viable offspring derived from fetal and adult mammalian cells." Nature 385(6619): 810-3.
Willison, K. & Ashworth A. Mammalian spermatogenic gene expression. Trends Genet. 3, 351 - 355 (1987).

**Table 1**

| | | Offspring | | | | | |
|---|---|---|---|---|---|---|---|
| Genotype of male | Number of males | *t⁶* | wt | total | *% t⁶* | *χ*² | P |
| *TgH1-33*/0; *t⁶l+* | 5 | 218 | 39 | 257 | 85 | 7.08 | 0.01 |
| *t⁶l+* | 5 | 125 | 43 | 168 | 74 | | |
| *TgH1-4*/0*; t⁶*/*+* | 7 | 190 | 19 | 209 | 91 | 5.17 | 0.025 |
| *t⁶*/*+* | 5 | 200 | 39 | 239 | 84 | | |

| Combined data: | | | | | | | |
|---|---|---|---|---|---|---|---|
| *Tg*/0*; t⁶*/*+* | 12 | 408 | 58 | 466 | 88 | 9.57 | 0.01 |
| *t⁶*/*+* | 10 | 325 | 82 | 407 | 80 | | |
| *Tagap1^{tm3Bgh}*/*+; t⁶*/*+* | 9 | 245 | 109 | 354 | 69 | 21.09 | 0.001 |
| *Tagap1 +*/*+; t⁶*/*+* | 9 | 292 | 56 | 348 | 84 | | |

**Abbr.: TgH1-33, Tg(Tagap1)H1-33Bgh; TgH1-4, Tg(Tagap1)H1-4Bgh, wt, wild type.**

### Table 1 Transmission ratio of t⁶ from males lacking or over-expressing Tagap1.

For overexpression experiments, two independent transgenic lines (TgH1-33 and tgH1-4) were established, the expression of the transgenic construct was verified and both lines were bred to the partial t-haplotype *t⁶*. The transmission ratio of *t⁶* was compared between transgenic and non-transgenic animals. Both lines significantly increase the transmission ratio of *t⁶.*
An inactivated *Tagap1* allele (loss-of-function) produced by gene targeting results in the complementary effect on the transmission ratio of the t-haplotype (lower part). The transmission ratio of the t-haplotype by t⁶/+ animals is strongly reduced by heterozygozity for *Tagap1^{tm3Bgn}.*

**Table 2:**

| Genotype of male | Number of males | Offspring | | | | | |
|---|---|---|---|---|---|---|---|
| | | t^{h49} | wt | total | % t^{h49} | χ² | P |
| *Fgd2 +*/*+; t^{h49}*/*+* | 7 | 93 | 100 | 193 | 48 | 9.72 | 0.001 |
| *Fgd2^{tm4Bgh}*/*+; t^{h49}*/*+* | 7 | 85 | 169 | 253 | 34 | | |

### Table 2: Transmission ratio of t^{h49} from males lacking Fgd2.

The transmission ratio of the t-haplotype by *t^{h49}* animals is decreased by a loss-of-function allele of Fgd2.

**Table 3:**

| **Overview of the Sequences disclosed in the Sequence listing** | |
|---|---|
| Coding Sequences | Organism/gene/allele, isoform |
| 1 | Tagap1 mouse wildtype FVB/N |
| 2 | Tagap1 mouse wildtype BALB/c |
| 3 | Tagap1 t1 mouse |
| 4 | Tagap1 t2 mouse |
| 5 | Tagap1 t3 mouse |
| 6 | Tagap1 t4 mouse |
| 7 | Tagap homo sapiens |
| 8 | Tagap Rattus |
| 9 | FGD2 mouse wildtype |
| 10 | FGD2 mouse isoform 1 |
| 11 | **FGD2 mouse isoform2** |
| 12 | FGD2 mouse isoform 1 t⁶/t^{w5} |
| 13 | Tiam2 mouse wildtype |
| 14 | Tiam2 homo sapiens |
| 15 | Tcr, short 5'utr mus musculus |
| 16 | Tcr, long 5'utr mus musculus |
| **Deduced Protein sequences** | |
| 17 | Tagap1 mouse wildtype FVB/N |
| 18 | Tagap1 mouse wildtype BALB/c |
| 19 | Tagap1 t1 mouse |
| 20 | Tagap1 t2 mouse |
| 21 | Tagap1 t3 mouse |
| 22 | Tagap1 t4 mouse |
| 23 | Tagap homo sapiens |
| 24 | Tagap Rattus |
| 25 | FGD2 mouse wildtype |
| 26 | FGD2 mouse wildtype isoform 1 |
| 27 | FGD2 mouse wildtype isoform2 |
| 28 | FGD2 mouse isoform 1 t⁶/t^{w5} |
| 29 | Tiam2 mouse wildtype |
| 30 | Tiam2 homo sapiens |

## Claims

1. A method for producing a transgenic non human male animal, preferably a mammal, fish, bird or insect, wherein the transgene(s) confer(s) a change in the transmission ratio of (a) genetic trait(s) to the offspring of said non human male animal, preferably mammal, fish, bird or insect to a non-Mendelian ratio, said method comprising introducing
(a) a first nucleic acid molecule encoding an expression product with a Responder function into a chromosome of a non-human germ cell, (fertilized) egg cell, embryonic cell or a cell derived therefrom, of the same species as the transgenic male to be prepared, said chromosome containing or conferring said genetic trait(s), thereby linking on said chromosome said Responder function to the genetic trait(s); and
(b) at least one second nucleic acid molecule encoding an expression product with a Distorter function into (a) chromosome(s) of a non-human germ cell, (fertilized) egg cell, embryonic cell or a cell derived of the same species as the transgenic male to be prepared, wherein said expression product with a Distorter function is a factor involved in G protein signaling,
wherein said first nucleic acid molecule encoding an expression product with Responder function and said at least one second nucleic acid molecule encoding an expression product with a Distorter function are introduced into the same or different chromosomes.

2. A method for producing a transgenic non human male animal, preferably a mammal, fish, bird or insect, wherein the transgene(s) confer(s) a change in the transmission ratio of (a) genetic trait(s) to the offspring of said non human male animal, preferably mammal, fish, bird or insect to a non-Mendelian ratio, said method comprising introducing
(a) a first nucleic acid molecule encoding an expression product with a Responder function into a chromosome of a non-human germ cell, (fertilized) egg cell, embryonic cell or a cell derived therefrom, of the same species as the transgenic male to be prepared, said chromosome containing or conferring said genetic trait(s), thereby linking on said chromosome said Responder function to the genetic trait(s); and
(b) at least one second nucleic acid molecule encoding an expression product directed against the Distorter function into (a) chromosome(s) of a non-human germ cell, (fertilized) egg cell, embryonic cell or a cell derived therefrom, of the same species as the transgenic male to be prepared, wherein said Distorter function is an expression product which is encoded by a nucleic acid molecule encoding an expression product with a Distorter function, wherein said expression product with a Distorter function is a factor involved in G protein signaling; and/or
(c) a second nucleic acid molecule for inactivation of the Distorter function by homologous recombination, wherein said second nucleic acid molecule for inactivation of the Distorter function by homologous recombination is at least partially partially identical to said nucleic acid molecule encoding an expression product with a Distorter function,
wherein said first nucleic acid molecule encoding an expression product with a Responder function and said at least one second nucleic acid molecule encoding an expression product directed against the Distorter function and/or said second nucleic acid molecule for inactivation of the Distorter function by homologous recombination are introduced into the same or different chromosomes,
thereby partially or completely inactivating the Distorter function.

3. The method of claim 1 or 2, wherein said mammal is selected from the group consisting of Mus, Rattus, Bos, Sus and Ovis.

4. The method of any one of claims 1 to 3, wherein said genetic trait is sex.

5. The method of any one of claims 1 to 4, wherein said chromosome is an X or Y chromosome or a corresponding sex chromosome in birds, fish or insect.

6. The method of any one of claims 1 to 4, wherein said chromosome is an autosome.

7. The method of any one of claims 1 to 6, wherein said factor involved in G protein signaling is a factor involved in Rho signaling.

8. The method of any one of claims 1 to 7, wherein said first nucleic acid molecule encoding an expression product with Responder function is selected from the group consisting of
(a) a nucleic acid molecule comprising or consisting of the nucleic acid molecule as shown in SEQ ID NO: 15 or 16 or a fragment thereof;
(b) a nucleic acid molecule being an allelic variant or a homologue or orthologue of the nucleic acid molecule of (a);
(c) a nucleic acid molecule hybridizing to a nucleic acid molecule complementary to the nucleic acid molecule of (a) or (b); and
(d) a nucleic acid molecule which is related to the nucleic acid molecule of (a), (b) or (c) by the degeneration of the genetic code.

9. The method of any one of claims 1 to 8, wherein said (at least one) second nucleic acid molecule encoding an expression product with a Distorter function is/are selected from the group consisting of
(a) a nucleic acid molecule comprising or consisting of the nucleic acid molecule as shown in any one of SEQ ID NOs:1 to 14 or a fragment thereof;
(b) a nucleic acid molecule being an allelic variant or a homologue or orthologue of the nucleic acid molecules of (a);
(c) a nucleic acid molecule hybridizing to a nucleic acid molecule complementary to the nucleic acid molecule of (a) or (b); and
(d) a nucleic acid molecule which is related to the nucleic acid molecule of (a), (b) or (c) by the degeneration of the genetic code.

10. The method of any one of claims 1 and 3 to 8, wherein said at least one second nucleic acid molecule encoding an expression product with a Distorter function is/are selected from the group consisting of
(a) a nucleic acid molecule comprising or consisting of the nucleic acid molecule as shown in any one of SEQ ID NOs: 1 to 12 or a fragment thereof;
(b) a nucleic acid molecule being an allelic variant or a homologue or orthologue of the nucleic acid molecule of (a);
(c) a nucleic acid molecule hybridizing to a nucleic acid molecule complementary to the nucleic acid molecule of (a) or (b); and
(d) a nucleic acid molecule which is related to the nucleic acid molecule of (a), (b) or (c) by the degeneration of the genetic code,
thereby enhancing said transmission ratio of said genetic trait(s).

11. The method of any one of claims 1 and 3 to 8, wherein said at least one second nucleic acid molecule encoding an expression product with a Distorter function is/are selected from the group consisting of
(a) a nucleic acid molecule comprising or consisting of the nucleic acid molecule as shown in SEQ ID NO: 13 or 14 or a fragment thereof;
(b) a nucleic acid molecule being an allelic variant or a homologue or orthologue of the nucleic acid molecule of (a);
(c) a nucleic acid molecule hybridizing to a nucleic acid molecule complementary to the nucleic acid molecule of (a) or (b); and
(d) a nucleic acid molecule which is related to the nucleic acid molecule of (a), (b) or (c) by the degeneration of the genetic code,
thereby reducing said transmission ratio of said genetic trait(s).

12. The method of any one of claims 2 to 8, wherein said nucleic acid molecule encoding an expression product with a Distorter function is selected from the group consisting of
(a) a nucleic acid molecule comprising or consisting of the nucleic acid molecule as shown in any one of SEQ ID NOs 1 to 12 or a fragment thereof;
(b) a nucleic acid molecule being an allelic variant or a homologue or orthologue of the nucleic acid molecule of (a);
(c) a nucleic acid molecule hybridizing to a nucleic acid molecule complementary to the nucleic acid molecule of (a) or (b); and
(d) a nucleic acid molecule which is related to the nucleic acid molecule of (a), (b) or (c) by the degeneration of the genetic code,
thereby reducing said transmission ratio of said genetic trait(s).

13. The method of any one of claims 2 to 8, wherein said nucleic acid molecule encoding an expression product with a Distorter function is selected from the group consisting of
(a) a nucleic acid molecule comprising or consisting of the nucleic acid molecule as shown in SEQ ID NO 13 or 14 or a fragment thereof;
(b) a nucleic acid molecule being an allelic variant or a homologue or orthologue of the nucleic acid molecule of (a);
(c) a nucleic acid molecule hybridizing to a nucleic acid molecule complementary to the nucleic acid molecule of (a) or (b); and
(d) a nucleic acid molecule which is related to the nucleic acid molecule of (a), (b) or (c) by the degeneration of the genetic code,
thereby enhancing said transmission ratio of said genetic trait(s).

14. The method of any one of claims 1 to 13, further comprising crossing the transgenic non human male mammal, fish, bird or insect obtained by the method of anyone of claims 1 to 13 with a non human female mammal, fish, bird or insect and analyzing the offspring of said cross for transmission of said genetic trait(s).

15. The method of any one of claims 1 to 14, wherein said Responder function and/or said Distorter function is the mouse-t-complex Responder/Distorter function.

16. The method of any one of claims 2 to 15, wherein said expression product directed against the Distorter function is an aptamer, a siRNA or shRNA or miRNA, a ribozyme, or an antisense nucleic acid molecule specifically hybridizing to said nucleic acid molecules encoding a factor involved in G protein signaling, or is an antibody, an antibody fragment or derivative thereof.

17. The method of any one of claims 1 to 16, wherein said at least one second nucleic acid molecule encoding the expression product with a Distorter function is modified, thereby further reducing or further enhancing the Distorter function activity.

18. The method of any one of claims 5 to 17, wherein said first nucleic acid molecule encoding an expression product with a Responder function and said at least one second nucleic acid molecule encoding an expression product with Distorter function and/or said at least one second nucleic acid molecule encoding an expression product directed against the Distorter function and/or said second nucleic acid molecule for inactivation of the Distorter function by homologous recombination and a promoter controlling expression in spermatogenesis and/or spermiogenesis and/or a stop cassette are integrated in said X or Y chromosome or corresponding sex chromosome or in one of said autosomes in a reversible inactive state of expressibility.

19. A non human male or female mammal, fish, bird or insect, wherein said non human male or female mammal, fish, bird or insect is transgenic for the nucleic acid molecule encoding an expression product with a Responder function and the nucleic acid molecule encoding an expression product with a Distorter function and/or the nucleic acid molecule encoding an expression product directed against the Distorter function and/or the nucleic acid molecule for inactivation of the Distorter function by homologous recombination as defined in any one of claims 1 to 18.

20. A pair of non human male and female animals, preferably mammals, fish, birds or insects, wherein at least one of the male and/or female is a transgenic non human mammal, fish, bird or insect as defined in claim 19.

21. The pair of non-human male and female animals, preferably mammals, fish, birds or insects of claim 20, wherein the nucleic acid molecule or part thereof encoding an expression product with a Responder function and/or the nucleic acid molecule or part thereof encoding an expression product with a Distorter function and/or the nucleic acid molecule or part thereof encoding an expression product directed against the Distorter function and/or the nucleic acid molecule or part thereof for inactivation of the Distorter function by homologous recombination as defined in any one of claims 1 to 18 is/are flanked by recombinase recognition sites.

22. The pair of non-human male and female animals, preferably mammals, fish, birds or insects of claim 20 or 21 having further stably integrated into its genomic DNA a nucleic acid molecule encoding a site specific DNA recombinase.

23. The pair of non human male and female animals, preferably mammals, fish, birds or insects of claim 22, wherein said DNA recombinase is Cre, wherein said recognition sites are loxP sites, or flp, wherein said recognition sites are FRT sites, or Φc31, wherein said recognition sites are att sites.

24. The pair of non human male and female animals, preferably mammals, fish, birds or insects of claim 22 or 23, wherein said DNA recombinase is controlled by regulatory elements that are active prior to spermiogenesis.

25. Sperm obtainable from a male of the transgenic non-human animal, preferably mammal, fish, bird or insect as defined in any one of claims 19 to 24.

26. Use of the sperm of claim 25 for the production of offspring.

27. Use of the nucleic acid molecule encoding an expression product with a Distorter function as defined in any one of claims 1 to 18, for the identification of chemicals or biological compounds able to trigger the (premature) activation or inhibition of the Responder/Distorter signalling cascade.

28. Use of the nucleic acid molecule encoding an expression product with a Distorter function as defined in any one of claims 1 to 18 for the isolation of receptor molecules and/or other members of the Responder/Distorter signaling cascade to which said expression product may bind.

29. A method for the detection of the nucleic acid molecule encoding an expression product with a Distorter function and/or the nucleic acid molecule encoding an expression product directed against the Distorter function and/or the nucleic acid molecule for inactivation of the Distorter function by homologous recombination as defined in any one of claims 1 to 18 in the non human male or female mammal, fish, bird or insect as defined in claim 19 comprising identifying said nucleic acid molecule encoding an expression product with a Distorter function and/or said nucleic acid molecule encoding an expression product directed against the Distorter function and/or said nucleic acid molecule for inactivation of the Distorter function by homologous recombination in said non human male or female mammal, fish, bird or insect by polymerase chain reaction (PCR), gene (micro)array hybridization, single nucleotide polymorphism (SNP) analysis, and/or sequencing with primers hybridizing to said nucleic acid molecule.

30. A nucleic acid molecule encoding an expression product with a Distorter function, wherein said expression product with a Distorter function is a factor involved in G protein signaling, selected from the group consisting of:
(a) a nucleic acid molecule comprising or consisting of the nucleic acid molecule of any one of SEQ ID NOs: 3 to 6 and 12 or a fragment thereof;
(b) a nucleic acid molecule being an allelic variant or a homologue or orthologue of the nucleic acid molecule of (a);
(c) a nucleic acid molecule which hybridizes under stringent conditions to the nucleic acid molecule of (a), wherein said nucleic acid molecule encodes a polypeptide which has
(i) at the position corresponding to position 49 of SEQ ID NO: 17 an I
(ii) at the position corresponding to position 144 of SEQ ID NO: 17 an L
(iii) at the position corresponding to position 323 of SEQ ID NO: 17 a T and
(iv) which terminates after position 442;
(d) a nucleic acid molecule which hybridizes under stringent conditions to the nucleic acid molecule of (a), wherein said nucleic acid molecule encodes a polypeptide which has
(i) at the position corresponding to position 49 of SEQ ID NO: 17 an I;
(ii) at the position corresponding to position 137 of SEQ ID NO: 17 an E;
(iii) at the position corresponding to position 207 of SEQ ID NO: 17 an F;
(iv) at the position corresponding to position 301 of SEQ ID NO: 17 an M;
(v) at the position corresponding to position 323 of SEQ ID NO: 17 an T;
(vi) at the position corresponding to position 332 of SEQ ID NO: 17 a D;
(vii) at the position corresponding to position 407-413 of SEQ ID NO: 17 an internal deletion;
(viii) at the position corresponding to position 440 of SEQ ID NO: 17 an M;
(ix) at the position corresponding to position 471 of SEQ ID NO: 17 an L;
(x) at the position corresponding to position 552 of SEQ ID NO: 17 an I;
(xi) at the position corresponding to position 596 of SEQ ID NO: 17 a K;
(xii) at the position corresponding to position 607 of SEQ ID NO: 17 an R;
(xiii) at the position corresponding to position 610 of SEQ ID NO: 17 an S; and
(xiv) at the position corresponding to position 703 of SEQ ID NO: 17 a V;
(e) a nucleic acid molecule which hybridizes under stringent conditions to the nucleic acid molecule of (a), wherein said nucleic acid molecule encodes a polypeptide which has
(i) at the position corresponding to position 49 of SEQ ID NO: 17 an I;
(ii) at the position corresponding to position 54 of SEQ ID NO: 17 a G;
(iii) at the position corresponding to position 137 of SEQ ID NO: 17 an E;
(iv) at the position corresponding to position 173 of SEQ ID NO: 17 a G;
(v) at the position corresponding to position 207 of SEQ ID NO: 17 an F;
(vi) at the position corresponding to position 301 of SEQ ID NO: 17 an M;
(vii) at the position corresponding to position 323 of SEQ ID NO: 17 a T;
(viii) at the position corresponding to position 332 of SEQ ID NO: 17 a D;
(ix) at the position corresponding to position 407-413 of SEQ ID NO: 17 an internal deletion;
(x) at the position corresponding to position 440 of SEQ ID NO: 17 an M;
(xi) at the position corresponding to position 471 of SEQ ID NO: 17 an L;
(xii) at the position corresponding to position 508 of SEQ ID NO: 17 an S;
(xiii) at the position corresponding to position 552 of SEQ ID NO: 17 an I;
(xiv) at the position corresponding to position 596 of SEQ ID NO: 17 a K;
(xv) at the position corresponding to position 607 of SEQ ID NO: 17 an R;
(xvi) at the position corresponding to position 610 of SEQ ID NO: 17 an S; and
(xvii) at the position corresponding to position 703 of SEQ ID NO: 17 a V; and
(f) a nucleic acid molecule which hybridizes under stringent conditions to the nucleic acid molecule of (a), wherein said nucleic acid molecule encodes a polypeptide which has
(i) at the position corresponding to position 49 of SEQ ID NO: 17 an I;
(ii) at the position corresponding to position 137 of SEQ ID NO: 17 an E;
(iii) at the position corresponding to position 207 of SEQ ID NO: 17 an F;
(iv) at the position corresponding to position 301 of SEQ ID NO: 17 an M;
(v) at the position corresponding to position 323 of SEQ ID NO: 17 a T;
(vi) at the position corresponding to position 332 of SEQ ID NO: 17 a D;
(vii) at the position corresponding to position 407-413 of SEQ ID NO: 17 an internal deletion;
(viii) at the position corresponding to position 440 of SEQ ID NO: 17 an M;
(ix) at the position corresponding to position 471 of SEQ ID NO: 17 an L;
(x) at the position corresponding to position 530 of SEQ ID NO: 17 an E;
(xi) at the position corresponding to position 552 of SEQ ID NO: 17 an I;
(xii) at the position corresponding to position 573 of SEQ ID NO: 17 an R;
(xiii) at the position corresponding to position 596 of SEQ ID NO: 17 a K;
(xiv) at the position corresponding to position 607 of SEQ ID NO: 17 an R;
(xv) at the position corresponding to position 610 of SEQ ID NO: 17 an S; and
(xvi) at the position corresponding to position 703 of SEQ ID NO: 17 a V.

31. The nucleic acid molecule of claim 30 which is a DNA molecule.

32. The nucleic acid molecule of claim 30 or 31, wherein said expression product is an RNA or a (poly)peptide.

33. A recombinant DNA molecule comprising the nucleic acid molecule of any one of claims 30 to 32 and a regulatory region being capable of controlling expression of said nucleic acid molecule.

34. The recombinant DNA molecule of claim 33, wherein said regulatory region is a naturally occurring regulatory region or a genetically engineered derivative thereof.

35. The recombinant DNA molecule of claim 33 or 34, wherein said regulatory region comprises or is a promoter.

36. A vector comprising the recombinant DNA molecule of any one of claims 33 to 35.

37. The vector of claim 36 comprising a heterologous promoter.

38. The vector of claim 37, wherein the heterologous promoter is controlling gene expression in spermatogenesis and/or in spermiogenesis.

39. The vector of claim 38, wherein the heterologous promoter is the testis promoter of c-kit, ACE, Tcr or Smok.

40. A host cell or organism transformed or transfected with the nucleic acid molecule of any one of claims 30 to 32, the recombinant DNA molecule of any one of claims 33 to 35 or the vector of any one of claims 36 to 39.

41. A method of recombinantly producing an expression product as defined in any one of claims 30 to 39 comprising the steps of culturing the host cell of claim 40 under conditions to cause expression of the protein and recovering said protein from the culture.

42. An expression product encoded by the nucleic acid molecule of any one of claims 30 to 32 or obtainable by the method of claim 41.

43. A method for the identification of a nucleic acid molecule encoding an expression product with a Distorter function, comprising the steps of
(a) isolating a nucleic acid molecule encoding a candidate expression product with a Distorter function from the mouse t-complex by means of genomic localization, wherein said nucleic acid molecule is involved in G protein signalling; and
(b) testing the nucleic acid molecule isolated in step (a) for a change of the transmission ratio of the Responder or of a genetic trait linked to a Responder in an experimental non human animal, wherein when said transmission ratio is enhanced or reduced, said nucleic acid molecule isolated in (a) is a nucleic acid molecule encoding an expression product with Distorter function.

44. A method for the identification of an expression product of a nucleic acid molecule encoding a Distorter, comprising the steps of
(a) isolating an expression product of a nucleic acid molecule encoding a candidate Distorter by means of protein-protein interaction with a known Distorter derived from the mouse t-complex; and
(b) testing the nucleic acid molecule encoding said expression product isolated in (a) for change of the transmission ratio of the Responder or of a genetic trait linked to a Responder in an experimental non human animal, wherein when said transmission ratio is enhanced or reduced, said expression product isolated in (a) is an expression product with Distorter function.

45. The method of claim 43 or 44, wherein in step (b) hypomorphic or hypermorphic alleles of said nucleic acid molecule are used for testing for change of the transmission ratio.


SEQUENCE LISTING

<110>  Max-Planck-Gesellschaft zur Förderung der Wissenschaften
       e.V.

<120>  Isolation of the t-complex Distorters and applications thereof

<130>  K 3062 EP

<160>  30

<170>  PatentIn version 3.3

<210>  1
<211>  2145
<212>  DNA
<213>  Tagap1 mouse wildtype FVB/N Accession Number NM_145968

<400>  1
atgaagctga taagcagtct cgatggttca aaaacactta atgctaacaa catggagaca      60

ttaattgaat gtcagtcaga gggtgatatc aaagtgcctc ccctgctgac atcatgtgag     120

agtgaagaca gcatttgcca gctaactgaa attaagaaga gaaagaaagt gctgtcctgg     180

ccatctctca tgagaaagct ctctccttca tccgacttct ctgggtcatt ggaaccagag     240

ctgaaagtgt cgctgtttga ccaacccttg tcgatcatct gtggggagaa cgacacactt     300

cccagaccca tccaggacat cctcaccatc ctctgcctta aaggtccttc aacggaaggg     360

atattcagga aagctgccag cgagaaagcc cgcaaggagc tgaaggaggg gcttaactgt     420

ggggtctccg tgaatctgaa gcagctccct gtgcacctcc tagctgtggt cttcaaggac     480

ttcctccgag ggatacccct gaagctactc tcctgtgatc tctttgagga ctggatgggc     540

gccctggaga agcccaccga ggaggacaga atcgaggccc tgaagcaggt tgctggtggc     600

ctcccccggc ccaaccttct cctgctcagg cacttgctct acgtgctcca cctcatcagc     660

aagaacgctg aggtcaacaa gatggactcc agcaacctcg ccatctgcat cgggcctaac     720

atgctcactc tgaagaacga ccagagcctg tccttccagg cccagaagga cctgaacaat     780

aaggttaaga tcttggtgga attcctcatc gacaactgct ttgagatatt tggggagaac     840

attcggacgc gttcccgcat cacttctgac gactccctgg aacacactga cagttcagac     900

gtgtcaactc tgcagaatga ctcggcctat gacagcaatg acccagatgt ggagcccaca     960

agtggcgcag cctctcccaa caggcaactg gagggtccca ctcccacaat ggctggtctg    1020

gatacccggg gccaacggga cacctgtgag tcaagctcag agtccagcgt tagcatggta    1080

gtcaggctga aaagctccat tgtccaacaa gacaggcggt tctctgaacc caacatgtca    1140

ccctcacgag agtgcctcgt gggcccaaca tccaagcaaa agctagcaag gagtgaggac    1200

agcttcactc tgtcccagga cgcctcctgt tctgaaggcg atgaagctga agatcccttt    1260

acagaggaag tcttcccagc agtggacagc aaacccaaga gacctgtgga tttgaaaata    1320

aagaattgga cccaaggttt agcatctcca cagggacaca taaccaaagc tttctccaga    1380

tcctccccag gcgaatcttt gggcagctca cctgtgcctt ctccatcctg ccccaagaga 1440

aacttcttca ccagacacca gagtttcaca acaaagacag acaaaaccaa accccagaga 1500

gaaattagaa agcactccat gtcgttttcc tttgcgtctc acaagaaagt gctgccccga 1560

acctccagca ttgggtctga gaaatccaaa gacttttcta gagaccaact ccagaaggac 1620

ttgaggaaag agagccaact ttctggcaga atcgtccagg aaaatgagtc agaaatccaa 1680

agccaaacat ctctgggctt cagcttgtct ggcacctggg ccctctcagt cgacaacacg 1740

ttccagttag tggatatgag gaaaccagga agcccaccat cttacgaaga ggccatttat 1800

taccagacat caggactcac agcctacggt ggccagacag ttgggagtat gaggtcaaga 1860

atgttcaagc caagcacagc agtgccccct gtgccttctc accatggagg tgacctcagt 1920

gaagggacac ctggtggaca cagattgtct tctgtgactg agcactggac acacagtcag 1980

actgtccatg tctctataga aactcagggg agatctgagc tacaccagtt gaggacagtg 2040

tccgagtcca tgcagaaggc taagctggac tgtcttgggc cacaacacag ccacttagtc 2100

tttgaggctg accaactctg ctgtgctaga gaatcctaca tttaa 2145

<210> 2
<211> 2145
<212> DNA
<213> Tagap1 mouse wildtype BALB/c

<400> 2
   atgaagctga taagcagtct cgatggttca aaaacactta atgctaacaa catggagaca 60
   ttaattgaat gtcagtcaga gggtgatatc aaagtgcctc ccctgctgac atcatgtgag 120
   agtgaagaca gcatttgcca gctaactgaa attaagaaga gaaagaaagt gctgtcctgg 180
   ccatctctca tgagaaagct ctctccttca tccgacttct ctgggtcatt ggaaccagag 240
   ctgaaagtgt cgctgtttga ccaacccttg tcgatcatct gtggggagaa cgacacactt 300
   cccagaccca tccaggacat cctcaccatc ctctgcctta aaggtccttc aacggaaggg 360
   atattcagga aagctgccag cgagaaagcc cgcaaggagc tgaaggaggg gcttaactgt 420
   ggggtctccg tgaatctgaa gcagctccct gtgcacctcc tagctgtggt cttcaaggac 480
   ttcctccgag ggatacccct gaagctactc tcctgtgatc tctttgagga ctggatgggc 540
   gccctggaga agcccaccga ggaggacaga atcgaggccc tgaagcaggt tgctggtggc 600
   ctcccccggc ccaaccttct cctgctcagg cacttgctct acgtgctcca cctcatcagc 660
   aagaacgctg aggtcaacaa gatggactcc agcaacctcg ccatctgcat cgggcctaac 720
   atgctcactc tgaagaacga ccagagcctg tccttccagg cccagaagga cctgaacaat 780
   aaggttaaga tcttggtgga attcctcatc gacaactgct ttgagatatt tggggagaac 840
   attcggacgc gttcccgcat cacttctgac gactccctgg aacacactga cagttcagac 900
   gtgtcaactc tgcagaatga ctcggcctat gacagcaatg acccagatgt ggagcccaca 960
   agtggcgcag cctctcccaa caggcaactg gagggtccca ctcccacaat ggctggtctg 1020
   gatacccggg gccaccggga cacctgtgag tcaagctcag agtccagcgt tagcatggta 1080
   gtcaggctga aaagctccat tgtccaacaa gacaggcggt tctctgaacc caacatgtca 1140
   ccctcacgag agtgcctcgt gggcccaaca tccaagcaaa agctagcaag gagtgaggac 1200
   agcttcactc tgtcccagga cgcctcctgt tctgaaggcg atgaagctga agatcccttt 1260
   acagaggaag tcttcccagc agtggacagc aaacccaaga gacctgtgga tttgaaaata 1320
   aagaattgga cccaaggttt agcatctcca cagggacaca taaccaaagc tttctccaga 1380
   tcctccccag gcgaatcttt gggcagctca cctgtgcctt ctccatcctg ccccaagaga 1440
   aacttcttca ccagacacca gagtttcaca acaaagacag acaaaaccaa accccagaga 1500
   gaaattagaa agcactccat gtcgttttcc tttgcgtctc acaagaaagt gctgccccga 1560
   acctccagca ttgggtctga gaaatccaaa gacttttcta gagaccaact ccagaaggac 1620
   ttgaggaaag agagccaact ttctggcaga atcgtccagg aaaatgagtc agaaatccaa 1680
   agccaaacat ctctgggctt cagcttgtct ggcacctggg ccctctcagt cgacaacacg 1740
   ttccagttag tggatatgag gaaaccagga agcccaccat cttacgaaga ggccatttat 1800
   taccagacat caggactcac agcctacggt ggccagacag ttgggagtat gaggtcaaga 1860
   atgttcaagc caagcacagc agtgccccct gtgccttctc accatggagg tgacctcagt 1920
   gaagggacac ctggtggaca cagattgtct tctgtgactg agcactggac acacagtcag 1980
   actgtccatg tctctataga aactcagggg agatctgagc tacaccagtt gaggacagtg 2040
   tccgagtcca tgcagaaggc taagctggac tgtcttgggc cacaacacag ccacttagtc 2100
   tttgaggctg accaactctg ctgtgctaga gaatcctaca tttaa 2145

<210> 3
<211> 1329
<212> DNA
<213> Tagap1 t1 mouse

<400> 3
atgaagctga taagcagtct cgatggttca aaaacactta atgctaacaa catggagaca 60

ttaattgaat gtcagtcaga gggtgatatc aaagtgcctc ccctgctgac atcatgtgag 120

agtgaagaca gcatttgcca gctaattgaa attaagaaga gaaagaaagt gctgtcctgg 180

ccatctctca tgagaaagct ctctccttca tccgacttct ctgggtcatt ggaaccagag 240

ctgaaagtgt cgctgtttga ccaacccttg tcgatcatct gtggggagaa cgacacactt 300

cccagaccca tccaggacat cctcaccatc ctctgcctta aaggtccttc aacggaaggg 360

atattcagga aagctgccag cgagaaagcc cgcaaggagc tgaaggaggg gcttaactgt 420

ggggtctcct tgaatctgaa gcagctccct gtgcacctcc tagctgtggt cttcaaggac 480

ttctttcgag ggatacccct gaagctactc tcctgtgatc tctttgagga ctggatgggc 540

gccctggaga agcccaccga ggaggacaga atcgaggccc tgaagcaggt tgctggtggc 600

ctcccccggc ccaaccttct cctgctcagg cacttgctct acgtgctcca cctcatcagc 660

aagaacgctg aggtcaacaa gatggactcc agcaacctcg ccatctgcat cgggcctaac 720

atgctcactc tgaagaacga ccagagcctg tccttccagg cccagaagga cctgaacaat 780

aaggttaaga tcttggtgga attcctcatc gacaactgct ttgagatatt tggggagaac 840

attcggacgc gttcccgcat cacttctgac gactccctgg aacacactga cagttcagac 900

gtgtcaactc tgcagaatga ctcggcctat gacagcaatg acccagatgt ggagcccaca 960

agtggcacag cctctcccaa caggcaactg gagggtccca ctcccacaat ggctggtctg 1020

gatacccggg gccaccggga cacctgtgag tcaagctcag agtccagcgt tagcatggta 1080

gtcaggctga aaagctccat tgtccaacaa gacaggcggt tctctgaacc caacatgtca 1140

ccctcacgag agtgcctcgt gggcccaaca tccaaacaaa agctaacaag gagtgaggac 1200

agcttcactc tgtcccagga cgcctcctgt tctgaaggcg atgaagctga agatcccttt 1260

acagaggaag tcttcccagc agtggacagc aaacccaaga gacctgtgga tttgaaaata 1320

aagaattga 1329

<210> 4
<211> 2124
<212> DNA
<213> Tagap1 t2 mouse

<400> 4
atgaagctga taagcagtct cgatggttca aaaacactta atgctaacaa catggagaca 60

ttaattgaat gtcagtcaga gggtgatatc aaagtgcctc ccctgctgac atcatgtgag 120

agtgaagaca gcatttgcca gctaattgaa attaagaaga gaaaaaaagt gctgtcctgg 180

ccatctctca tgagaaagct ctctccttca tccgacttct ctgggtcatt ggaaccagag 240

ctgaaagtgt cgctgtttga ccaacccttg tcgatcatct gtggggagaa cgacacactt 300

cccagaccca tccaggacat cctcaccatc ctctgcctta aaggtccttc aacggaaggg 360

atattcagga aagctgccag cgagaaagcc cgcaaggagc tgaaggagga gcttaactgt 420

ggggtctccg tgaatctgaa gcagctccct gtgcacctcc tagctgtggt cttcaaggac 480

ttcctccgag ggatacccct gaagctactc tcctgtgatc tctttgagga ctggatgggc 540

gccctggaga agcccacgga ggcggacaga atcgaggccc tgaagcaggt tgctggcggc 600

ctcccccggc ccaacctttt cctgctcagg cacttgctct acgtgctcca cctcatcagc 660

aagaacgctg aggtcaacaa gatggactcc agcaaccttg ccatctgcat cgggcctaac 720

atgctcactc tgaagaacga ccagagcctg tccttccagg cccagaagga cctgaacaat 780

aaggttaaga tcttggtgga attcctcatt gacaactgct ttgagatatt tggggagaac 840

attcggacgc gttcccgcat cacttctgac gactccctgg aacacactga cagttcagac 900

atgtcaactc tgcagaatga ctcggcctat gacagcaatg acccagatgt ggagcccaca 960

agtggcacag cctctcccaa caggcaactg gaggatccca ctcccacaat ggctggtctg 1020

gatacccggg gccaccggga cacctgtgag tcaagctcag agtccagcgt tagcatggta 1080

gtcaggctga aaagctccat tgtccaacaa gacaggcggt tctctgaacc caacatgtca 1140

ccctcacgag agtgcctcgt gggcccaaca tccaagcaaa agctaacaag gagtgaggac 1200

agcttcactc tgtcccagga tgaagctgaa gatcccttta cagaggaagt cttcccagca 1260

gtggacagca aacccaagag acctgtggat ttgaaaatga agaattggac ccaaggttta 1320

gcatctccac agggacacat aaccaaagct ttctccagat cctccccagg cgaatctttg 1380

ggcagctcac ttgtgccttc tccatcctgc cccaagagaa acttcttcac cagacaccag 1440

agtttcacaa caaagacaga caaaaccaaa ccccagagag aaattagaaa gcactccatg 1500

tcgttttcct ttgcgtctca caagaaagtg ctgccccgaa cctccagcat tgggtctgag 1560

aaatccaaag acttttctag agaccaactc cagaaggact tgaggaaaga gagccaactt 1620

tctggcagaa tcatccagga aaatgagtca gaaatccaaa gccaaacatc tctgggcttc 1680

agcttgtctg gcacctgggc cctctcagtg gacaacacgt tccagttagt ggatatgagg 1740

aaaccaggaa gcccaccatc ttacaaagag gccatttatt accagacatc aggactcaga 1800

gcctacagtg gccagacagt tgggagtatg aggtcaagaa tgttcaagcc aagcacagcg 1860

gtacctcctg tgccttctca ccatggaggt gacctcagtg aagggacacc tggtggacac 1920

agattgtctt ctgtgactga gcactggaca cacagtcaga ctgtccatgt ctctatagaa 1980

actcagggga gatctgagct acaccagttg aggacagtgt ccgagtccat gcagaaggct 2040

aagctggact gtcttgggcc acaacacagc cacttagtct ttgaggttga ccaactctgc 2100

tgtgctagag aatcctacat ttaa 2124

<210> 5
<211> 2124
<212> DNA
<213> Tagap1 t3 mouse

<400> 5
atgaagctga ttagcagtct cgatggttca aaaacactta atgctaacaa catggagaca 60

ttaattgaat gtcagtcaga gggtgatatc aaagtgcctc ccctgctgac atcatgtgag 120

agtgaagaca gcatttgcca gctaattgaa attaagaagg gaaaaaaagt gctgtcctgg 180

ccatctctca tgagaaagct ctctccttca tccgacttct ctgggtcatt ggaaccagag 240

ctgaaagtgt cgctgtttga ccaacccttg tcgatcatct gtggggagaa cgacacactt 300

cccagaccca tccaggacat cctcaccatc ctctgcctta aaggtccttc aacggaaggg 360

atattcagga aagctgccag cgagaaagcc cgcaaggagc tgaaggagga gcttaactgt 420

ggggtctccg tgaatctgaa gcagctccct gtgcacctcc tagctgtggt cttcaaggac 480

ttcctccgag ggatacccct gaagctactc tcctgtggtc tctttgagga ctggatgggc 540

gccctggaga agcccacgga ggaggacaga atcgaggccc tgaagcaggt tgctggtggc 600

ctcccccggc ccaacctttt cctgctcagg cacttgctct acgtgctcca cctcatcagc 660

aagaacgctg aggtcaacaa gatggactcc agcaaccttg ccatctgcat cggccctaac 720

atgctcactc tgaagaacga ccagagcctg tccttccagg cccagaagga cctgaacaat 780

aaggttaaga tcttggtgga attcctcatt gacaactgct ttgagatatt tggggagaac 840

attcggacgc gttcccgcat cacttctgac gactccctgg aacacactga cagttcagac 900

atgtcaactc tgcagaatga ctcggcctat gacagcaatg acccagatgt ggagcccaca 960

agtggcacag cctctcccaa caggcagctg gaggatccca ctcccacaat ggctggtctg 1020

gatacccggg gccaccggga cacctgtgag tcaagctcag agtccagcgt tagcatggta 1080

gtcaggctga aaagctccat tgtccaacaa gacaggcggt tctctgaacc caacatgtca 1140

ccctcacgag agtgcctcgt gggcccaaca tccaagcaaa agctaacaag gagtgaggac 1200

agcttcactc tgtcccagga tgaagctgaa gatcccttta cagaggaagt cttcccagca 1260

gtggacagca aacccaagag acctgtggat ttgaaaatga agaattggac ccaaggttta 1320

gcatctccac agggacacat aaccaaagct ttctccagat cctccccagg cgaatctttg 1380

ggcagctcac ttgtgccttc tccatcctgc cccaagagaa acttcttcac cagacaccag 1440

agtttcacaa caaagacaga caaaaccaaa ccccagagag aaattagaaa gcactccatg 1500

tcgttttcct ttgcgtctca caagaaagtg ctgccccgaa cctccagcat tgggtctgag 1560

aaatccaaag acttttctag agaccaactc cagaaggact tgaggaaaga gagccaactt 1620

tctggcagaa tcatccagga aaatgagtca gaaatccaaa gccaaacatc tctgggcttc 1680

agcttgtctg gcacctgggc cctctcagtg gacaacacgt tccagttagt ggatatgagg 1740

aaaccaggaa gcccaccatc ttacaaagag gccatttatt accagacatc aggactcaga 1800

gcctacagtg gccagacagt tgggagtatg aggtcaagaa tgttcaagcc aagcacagcg 1860

gtaccccctg tgccttctca ccatggaggt gacctcagtg aagggacacc tggtggacac 1920

agattgtctt ctgtgactga gcactggaca cacagtcaga ctgtccatgt ctctatagaa 1980

actcagggga gatctgagct acaccagttg aggacagtgt ccgagtccat gcagaaggct 2040

aagctggact gtcttgggcc acaacacagc cacttagtct ttgaggttga ccaactctgc 2100

tgtgctagag aatcctacat ttaa 2124

<210> 6
<211> 2124

<212>  DNA
<213>  Tagap1 t4 mouse

<400>  6
atgaagctga ttagcagtct cgatggttca aaaacactta atgctaacaa catggagaca      60

ttaattgaat gtcagtcaga gggtgatatc aaagtgcctc ccctgctgac atcatgtgag     120

agtgaagaca gcatttgcca gctaattgaa attaagaaga gaaagaaagt gctgtcctgg     180

ccatctctca tgagaaagct ctctccttca tccgacttct ctgggtcatt ggaaccagag     240

ctgaaagtgt cgctgtttga ccaacccttg tcgatcatct gtggggagaa cgacacactt     300

cccagaccca tccaggacat cctcaccatc ctctgcctta aaggtccttc aacggaaggg     360

atattcagga aagctgccag cgagaaagcc cgcaaggagc tgaaggagga gcttaactgt     420

ggggtctccg tgaatctgaa gcagctccct gtgcacctcc tagctgtggt cttcaaggac     480

ttcctccgag ggatacccct gaagctactc tcctgtgatc tctttgagga ctggatgggc     540

gccctggaga agcccacgga ggaggacaga atcgaggccc tgaagcaggt tgctggtggc     600

ctcccccggc ccaacctttt cctgctcagg cacttgctct acgtgctcca cctcatcagc     660

aagaacgctg aggtcaacaa gatggactcc agcaaccttg ccatctgcat cgggcctaac     720

atgctcactc tgaagaacga ccagagcctg tccttccagg cccagaagga cctgaacaat     780

aaggttaaga tcttggtgga attcctcatt gacaactgct ttgagatatt tggggagaac     840

attcggacgc gttcccgcat cacttctgac gactccctgg aacacactga cagttcagac     900

atgtcaactc tgcagaatga ctcggcctat gacagcaatg acccagatgt ggagcccaca     960

agtggcacag cctctcccaa caggcaactg gaggatccca ctcccacaat ggctggtctg    1020

gatacccggg gccaccggga cacctgtgag tcaagctcag agtccagcgt tagcatggta    1080

gtcaggctga aaagctccat tgtccaacaa gacaggcggt tctctgaacc caacatgtca    1140

ccctcacgag agtgcctcgt gggcccaaca tccaagcaaa agctaacaag gagtgaggac    1200

agcttcactc tgtcccagga tgaagctgaa gatcccttta cagaggaagt cttcccagca    1260

gtggacagca aacccaagag acctgtggat ttgaaaatga agaattggac ccaaggttta    1320

gcatctccac agggacacat aaccaaagct ttctccagat cctccccagg cgaatctttg    1380

ggcagctcac ttgtgccttc tccatcctgc cccaagagaa acttcttcac cagacaccag    1440

agtttcacaa caaagacaga caaaaccaaa ccccagagag aaattagaaa gcactccatg    1500

tcgttttcct ttgcgtctca caagaaagtg ctgccccgaa cctccagcat tgggtctgag    1560

aaatccgaag acttttctag agaccaactc cagaaggact tgaggaaaga gagccaactt    1620

tctggcagaa tcatccagga aaatgagtca gaaatccaaa gccaaacatc tctgggcttc    1680

agcttgtctg gcacccgggc cctctcagtg gacaacacgt tccagttagt ggatatgagg    1740

aaaccaggaa gcccaccatc ttacaaagag gccatttatt accagacatc aggactcaga    1800

gcctacagtg gccagacagt tgggagtatg aggtcaagaa tgttcaagcc aagcacagcg 1860

gtaccccctg tgccttctca ccatggaggt gacctcagtg aagggacacc tggtggacac 1920

agattgtctt ctgtgactga gcactggaca cacagtcaga ctgtccatgt ctctatagaa 1980

actcagggga gatctgagct acaccagttg aggacagtgt ccgagtccat gcagaaggct 2040

aagctggact gtcttgggcc acaacacagc cacttagtct ttgaggttga ccaactctgc 2100

tgtgctagag aatcctacat ttaa 2124

<210> 7
<211> 3363
<212> DNA
<213> Tagap homo sapiens Accession number NM_054114

<400> 7
gacatagctg ccctaaaagg aatgaggaag cgagagctct ccagtgtctg gctggctccg 60

tccgtgtgac agcccatgat gttctttccg gtctctgtaa tattctgaat ttccacctgc 120

ccgccccttc gcttataatg cagagcatgt gaagggagac cggctcggtc tctctctctc 180

ccagtggact agaaggagca gagagttatg ctgtttctcc cattctttac agctcaccgg 240

atgtaaaaga actctggcta gagaccctcc aaggacagag gcacagccac acgggagtga 300

aatccacccc tggacagtca gccgcaatac tgatgaagct gagaagcagc cacaatgctt 360

caaaaacact aaacgccaat aatatggaga cactaatcga atgtcaatca gagggtgata 420

tcaaggaaca tcccctgttg gcatcatgtg agagtgaaga cagtatttgc cagctcattg 480

aagttaagaa gagaaagaag gtgctgtcct ggccctttct catgagaagg ctctcccctg 540

catcagattt ttctggggct ttggagacag acttgaaagc atcgctattt gatcagccct 600

tgtcaattat ctgcggtgac agtgacacac tccccagacc catccaggac attctcacta 660

ttctatgcct taaaggccct tcaacggaag ggatattcag gagagcagcc aacgagaaag 720

cccgtaagga gctgaaggag gagctcaact ctggggatgc ggtggatctg gagaggctcc 780

ccgtgcacct cctcgctgtg gtctttaagg acttcctcag aagtatcccc cggaagctac 840

tttcaagcga cctctttgag gagtggatgg gtgctctgga gatgcaggac gaggaggaca 900

gaatcgaggc cctgaaacag gttgcagata agctcccccg gcccaacctc ctgctactca 960

agcacttggt ctatgtgctg cacctcatca gcaagaactc tgaggtgaac aggatggact 1020

ccagcaatct ggccatctgc attggaccca acatgctcac cctggagaat gaccagagcc 1080

tgtcatttga agcccagaag gacctgaaca acaaggtgaa gacactggtg gaattcctca 1140

ttgataactg ctttgaaata tttggggaga acattccagt gcattccagt atcacttctg 1200

atgactccct ggagcacact gacagttcag atgtgtcgac cctgcagaat gactcagcct 1260

acgacagcaa cgaccctgat gtggaatcca acagcagcag tggcatcagc tctcccagca 1320

ggcagcccca ggtgcccatg gccacagctg ctggcttgga tagcgcgggc ccacaggatg 1380

cccgagaggt cagcccagag cccattgtga gcaccgtggc caggctgaaa agctccctcg 1440
cacagcccga taggagatac tcagagccca gcatgccatc ctcccaggag tgcctcgaga 1500
gccgggtgac aaaccaaaca ctaacaaaga gtgaagggga cttccccgtg ccccgggtag 1560
gctctcgttt ggaaagtgag gaggctgaag acccatttcc agaggaggtc ttccctgcag 1620
tgcaaggcaa aaccaagagg ccggtggacc tgaagatcaa gaacttggcc ccgggttcgg 1680
tgctcccgcg ggcactggtt ctcaaagcct tctccagcag ctcgctggac gcgtcctctg 1740
acagctcgcc cgtggcttct ccttccagtc ccaaaagaaa tttcttcagc agacatcagt 1800
ctttcaccac aaagacagag aaaggcaagc ccagccgaga aattaaaaag cactccatgt 1860
ctttcacctt tgcccctcac aaaaaagtgc tgaccaaaaa cctcagcgcg ggctctggga 1920
aatcgcaaga ctttaccagg gaccacgtcc cgaggggtgt cagaaaggaa agccagcttg 1980
ccggccgaat cgtgcaggaa aatgggtgtg aaacccacaa ccaaacagcc cgcggcttct 2040
gcctgagacc ccacgccctc tcggtggatg atgtgttcca gggagctgac tgggagaggc 2100
ctggaagccc accctcttat gaagaggcca tgcagggccc ggcagccaga ctagtggcct 2160
ccgagagcca gaccgtgggg agcatgacgg tggggagcat gagggcgagg atgctggagg 2220
cgcactgcct cctaccccct cttccacctg ctcaccacgt agaggactca agacacaggg 2280
gcagcaaaga gccactccct ggccacggac tctctcccct gcctgagcga tggaaacaga 2340
gcagaactgt ccatgcttct ggggactctc tggggcacgt gtctggccca gggagacctg 2400
agctcctccc gctgaggacc gtctccgagt ccgtgcagag gaataagcgg gactgtctcg 2460
tgcgacgatg tagccagccg gtctttgagg ctgaccaatt ccaatatgcc aaagaatcgt 2520
atatttagga gggaggccat acgccatgcc atagcttgtg ctatctgtaa atatgagact 2580
tgtaaagaac tgcctgtaga ttgtttttaa aaggtcttga ataagctcct tgagaaagtt 2640
gtggaaagcc ctcctcagtg aggatagcta caccatggcc atggcgcatc agatagtctc 2700
tgtgtacctg gatttgtgca atatgtaaaa atgtatcaaa tgtattatag ataaggtgtt 2760
aggtgcaaag gatgtctaat aatccctgca cacgttttga acttgcagtg aagtacactg 2820
ctgttccttg cttcctgggg cacttttctc ttggttagtg tttaaaaatt atcttcgctt 2880
ttttaatgtg gcctcaaatg tcatgccaat tttcacatct tccacaaact ccatttaggg 2940
agaaatgttt aaatctctgg tataagttta ctccatacca gagtaaacta tatattactc 3000
tatataagca gtcttgcaat aactaatcac caccatagaa gaaagaaaca gactgcaagg 3060
aacagagttg agtgtctgga gtcatcaaag gcattaaaaa ctccagtaaa agctggggcc 3120
gtagcaaaaa tcatgaaaaa cacttcaacg tgtcctttca atcatccaat taaatgtggg 3180
tagattaatg aaaatgtatt acatcaatat taactcatct atagcacttt gagtatcttt 3240
gtagttcatg atatcctatc ctataatgtg gaggtaaatg attttatatg cattgggggt 3300

catatataaa acttcaatgt aatttcacta caataaattg ccttccttat ttgaaagtaa 3360

aaa 3363

<210> 8
<211> 3027
<212> DNA
<213> Tagap Rattus Accession number XM_217791

<400> 8
atggaattgt taagtgctcg cctgccccga gcaggagaaa catttgtccc aatatctgag 60

catcagcact ggaagccaga gaacgcccac aacaacggag ctcagtgttt cccagagctc 120

acgttaggcc ggacgcatca aggcaacaag gtcctggaca gtgagtgggt cacacagatg 180

ctaaggacca cactagcctt tcggagggca ctttatatca agcttacagc atccattcta 240

gaacttcaga acaggaagaa cgggaccttc catgtacttc ggggtgagaa ttacaagttc 300

agcagtgttg aacacagttc tctggctggc atacagcagg tgttcagcaa gcattcccag 360

tctgtccaag actgttctga cgttgaaaaa gttgtagcaa tagttgtggc tgagaaacct 420

ggaatctcat tcaagaaccc accaccggct ttaatggctg gccccaggga cttggcagcc 480

gcagagctgg agcgaggaga agcaaagaga cctccggaag agatccagtg cgatggggtg 540

tcccagataa cagctcctct ggaatggagg gaaagcaggt tctatgagaa tagtgtgagt 600

gataaaacag atgaattcct gtgcccacag atccgctaca agagctccac aaggggtcgc 660

tgcaggcgcc acgcaggggg tcggtacatg cgcctagcaa ggcccctgcc actggataac 720

aaccacctgg agactcatcg tgtgaggaca gtgctagtgt ctattttgtc cagacccctg 780

agtcatcctg tgagggacgc tgcttcactt gggctcactc aaacacagaa ccaaagcctc 840

gaggtagaga aaattcccct tggactgtca gccccgaatc caatgaagtt gataagcagt 900

ctcgatggtg caaaaacgct taatgccaac aacatggaca cattaattga atgtcagtca 960

gagggcgata tcaaggtgct tccattgctg acgtcatgtg agagtgaaga cagcatttgc 1020

cagctaattg aagttaagaa gagaaagaaa gtgctgtcct ggccatctct catgagaaag 1080

ctctctcctt caccagactt ctctgggtca ttggaaccag agctgaaagt gtcgctgttt 1140

gatcaaccct tgtcaatcat ctgtaaggag aatgacacac tccctagacc catccaggac 1200

atcctcacca tcctctgcct taaaggtcct tcaactgaag gaatattcag gaaagcagcc 1260

agcgagaaag cccgcaagga gctgaaggag gagcttaact gtgggggctc tgtgaatctg 1320

aaccagctcc ctgtgcacct cctggctgtg gtcttcaagg acttcctccg aggaatcccc 1380

ctgaagctgc tctcctgtga cctctttgag gactggatgg gagccctgga gaagcccagt 1440

gaggaggaca ggatcgaggc cctgaagcag gttgctgatc ggctcccccg gcccaacctc 1500

cttctgctca ggaccttagt ctacgtgcta caccttatca gcaagaacgc cgaggtcaac 1560

aagatggact ccagcaacct ggccatctgc atcggaccca acatgctcac actgaagaat 1620

gaccagagcc tgtccttcca ggcccagagg gacctgaaca ataaggttaa gatcttggtg 1680

gaattcctca ttgacaactg ccttgaaata tttggggaga acattccgac acatccccgc 1740

atcacttctg atgactctct ggaacacact gacagctcag acgtgtcgac tctgcagaac 1800

gactcagcct atgacagcaa tgacccggat gtagagcccg cgaatggagt tgcctctccc 1860

tgcaggcagt tggagggtcc ctctaccaca gtggctggca tggatacccg ggggccccgg 1920

aacacctgtg agtcgagctc agaatccagt gtcagcatgg tagccaggct gaaaagctcc 1980

attggccagc aagacaggcg tttctctgaa cccaacatgc caccctcacg agagtgcttc 2040

gtgggcccga taaccaagca aaagctaacg aggagcgagg acagcttcgt tctgccccag 2100

gaagcctcct gttctgaagg caatgaagcc gaagatccct ttacagagga agtcttccca 2160

gcagttgaag gcaaacccat gagaccagtg gatttgaaga taaagaactt gacccaaggt 2220

ttagcatctc cacagggacc tgcaaacaaa gctttctcca gcttctccgc gggggaatct 2280

ttggacagct cacctgtgcc ttctccatcc tgtcccaaga gaaacttctt caccagacac 2340

cagagtttca ccacaaagac ggacaagacc aagccccaga gagaaattag aaagcactcc 2400

atgtcatttt cctttgcgtc tcacaagaaa gtgctgcccc ggacctccag cattgggtct 2460

gagaaatcca aagacttctc tagagaccag ctccagaagg acttgaggaa agagagccag 2520

cttgccggca gaatcatccg ggaaaatgag tccgaaatcc aaagccaaac aaatctgggc 2580

tccagcttgt ctggaacctg ggccctctca gttgataaca cgttccagtt cattgatgtg 2640

aggaagccag gaagcccacc atcttatgaa gaggccattt attaccacac atcaggactc 2700

acagcctaca gtggccagac agttgggagt atgagagcaa gaatgttgaa gcagagcatg 2760

gcggtgcccc ctgtgccttc tcaccatgaa ggtgagctca gtgaagggat acctggtgga 2820

cacagatcgt cttccgtgac tgagcactgg acacagagtc agactgtcca tgtctctgta 2880

gaaactcggg ggagatctga gctacatcga ttgaggacag tgtctgagtc cgtgcagagg 2940

gctaagctgg actaccttgg gcagcaacgc agccacttgg tctttgaggt tgaccaactc 3000

cgatgtgcta aagaatccta catttag 3027

<210> 9
<211> 2181
<212> DNA
<213> FGD2 mouse wildtype Accession number AF017368

<400> 9
atggagcgag cctgtgagaa gcaggactca gtgtgcaacc tggtggctgt gtttgagaac 60

aacaggactc cgggagaagc acctggatcc catagccttg aggaccagcc ccatatccct 120

gaacaccagc tgtccctgtc cccagagcct tgggaggcac cccctgtcaa ggaggccttg 180

aagtctgaat tccggccagt gagcaggaca tatctgagtt ccctcaagaa caagctatcg 240

agtggggctt ggaggagatc ctgtcagcct ggggtcagcc cggggccaga gacacaggaa 300
cctgaggaga agagggtcgt gcgagagctt ctggagacgg agcaggccta tgtggctcgc 360
ctgcacctgc ttgaccaggt gttcttccag gagctgctga gggaggcagg ccgcagcaag 420
gccttccctg aggacgtggt gaagctcatt ttctccaaca tctcctccat ctatcgtttc 480
cacgcccagt tcttccttcc cgagctgcag cggcgcgtgg atgactgggc agccacgccc 540
cgcattgggg atgtgatcca gaagctggcc ccgtttctga aaatgtacag cgagtacgtg 600
aagaactttg agcgggccgc ggaactgctg gccacgtgga tggacaagtc tcagcccttc 660
caggaggtgg tcacccgcat ccagtgcagc gaggcctcca gcagcctgac cctgcagcac 720
cacatgttgg agcctgtgca aagaatcccg cggtacgaac tgctgctcaa ggaatatgtg 780
cagaagctgc cagcccaggc cccagacctc gaagatgccc agagagcact ggacatgatc 840
ttctcagctg cacagcactc caatgcagcc attgcagaga tggagcggct gcagggcctg 900
tgggatgtgt accagcgcct gggcttggag gatgacatcg tggacccctc caacaccctg 960
ctccgagagg gccctgttct caagatctct ttccgccgca gcgacccaat ggaacgctac 1020
ctggttttgt tcaacaacat gcttctgtat tgtgtacccc gagtcctcca agtgggtgcc 1080
cagttccagg tgcggactcg catcgatgtg gccggcatga aggtgcggga gctgaccgat 1140
gctgagttcc cacactcctt cctggtgtcc ggaaagcagc gcacactgga gctgcaggcc 1200
cggtcccgag atgaaatggt ttcctggatg caggcctgcc aggcagccat tgaccaggtt 1260
gagaagcgga gtgagacctt caaggctgct gtccagggac ctcaggggga cacgcaggag 1320
cccaagccac aggtagagga gctgggtctc cgagcgcctc agtgggtccg ggacaagatg 1380
gtgaccatgt gcatgcgctg ccaggagccc ttcaatgccc tgactcgtcg gcgccaccac 1440
tgccgggctt gcggctatgt agtgtgtgcc aagtgctctg actaccgtgc ggagctgaaa 1500
tatgacagca acaggcccaa ccgagtctgc ctgacctgct acacatttct cactggaaac 1560
gtactccctc aaggcaagga ggacaagagg cggggcatct tggagaaaga ggcctcagca 1620
gcacccgagc agagtctggt gtgcagcttc ctgcagctca taggggacaa gtgcagcagg 1680
agccttcccc ggagctggtg tgtgatcccc cgggatgacc cccttgtcct gtatgtctat 1740
gcagcccccc aggacacaaa ggctcacacc tccatccccc tactgggcta tcaggtgatt 1800
tcagggcccc aggggacctc gggttttcca gctgcaacag tcaggcgagc agtacacctt 1860
caaggccgag tctgtggagc tgcagggccg ctgggtgaca gctatcaagc gtgcgccagt 1920
ggcacccctg aggacctgac gaagaagatg tgtctgactg aaccagcagc cagctgctca 1980
tcgagagtcc atgacagcct tcccaggcct accccatggt tctatcactt caccccaagc 2040
tgggctaccc ctgacccacc tgatgtctat accgagacaa ttcatcctga ctctgtatcc 2100
agcagacaca ggccctttcc ttcaggaaga taccaccaag tgtcccagct ggctggagag 2160

ggggcggata tccctggatc a                                              2181

<210>  10
<211>  1965
<212>  DNA
<213>  FGD2 mouse isoform 1; Accession number AK042260

<400>  10
atggagcgag cctgtgagaa gcaggactca gtgtgcaacc tggtggctgt gtttgagaac      60

aacaggactc cgggagaagc acctggatcc catagccttg aggaccagcc ccatagccct     120

gaacaccagc tgtccctgtc cccagagcct tgggaggcac cccctgtcaa ggaggccttg     180

aagtctgaat tccggccagt gagcaggaca tatctgagtt ccctcaagaa caagttatcg     240

agtggggctt ggaggagatc ctgtcagcct ggggtcagcc cggggccaga gacacaggaa     300

cctgaggaga agagggtcgt gcgagagctt ctggagacgg agcaggccta tgtggctcgc     360

ctgcacctgc ttgaccaggt gttcttccag gagctgctga gggaggcagg ccgcagcaag     420

gccttccctg aggacgtggt gaagctcatt ttctccaaca tctcctccat ctatcgtttc     480

cacgcccagt tcttccttcc cgagctgcag cggcgcgtgg atgactgggc agccacgccc     540

cgcattgggg atgtgatcca gaagctggcc ccgtttctga aaatgtacag cgagtacgtg     600

aagaactttg agcgggccgc ggaactgctg gccacgtgga tggacaagtc tcagcccttc     660

caggaggtgg tcacccgcat ccagtgcagc gaggcctcca gcagcctgac cctgcagcac     720

cacatgttgg agcctgtgca aagaatcccg cggtacgaac tgctgctcaa ggaatatgtg     780

cagaagctgc cagcccaggc cccagacctc gaagatgccc agagagcact ggacatgatc     840

ttctcagctg cacagcactc caatgcagcc attgcagaga tggagcggct gcagggcctg     900

tgggatgtgt accagcgcct gggcttggag gatgacatcg tggacccctc caacaccctg     960

ctccgagagg gccctgttct caagatctct ttccgccgca gcgacccaat ggaacgctac    1020

ctggttttgt tcaacaacat gcttctgtat tgtgtacccc gagtcctcca agtgggtgcc    1080

cagttccagg tgcggactcg catcgatgtg gccggcatga aggtgcggga gctgaccgat    1140

gctgagttcc cacactcctt cctggtgtcc ggaaagcagc gcacactgga gctgcaggcc    1200

cggtcccgag atgaaatggt ttcctggatg caggcctgcc aggcagccat tgaccaggtt    1260

gagaagcgga gtgagacctt caaggctgct gtccagggac ctcaggggga cacgcaggag    1320

cccaagccac aggtagagga gctgggtctc cgagcgcctc agtgggtccg ggacaagatg    1380

gtgaccatgt gcatgcgctg ccaggagccc ttcaatgccc tgactcgtcg gcgccaccac    1440

tgccgggctt gcggctatgt agtgtgtgcc aagtgctctg actaccgtgc ggagctgaaa    1500

tatgacagca acaggcccaa ccgagtctgc ctgacctgct acacatttct cactggaaac    1560

gtactccctc aaggcaagga ggacaagagg cggggcatct tggagaaaga ggcctcagca    1620

gcacccgagc agagtctggt gtgcagcttc ctgcagctca taggggacaa gtgcagcagg 1680

agccttcccc ggagctggtg tgtgatcccc cgggatgacc cccttgtcct gtatgtctat 1740

gcagcccccc aggacacaaa ggctcacacc tccatccccc tactgggcta tcaggtgatt 1800

tcagggcccc agggggaccc tcgggttttc cagctgcaac agtcaggcca gcagtacacc 1860

ttcaaggccg agtctgtgga gctgcagggc cgctgggtga cagctatcaa gcgtgcggcc 1920

agtggccgga cccctgaggg acctgacgaa gaagatgtgt ctgac 1965

<210> 11
<211> 1387
<212> DNA
<213> FGD2 mouse isoform 2; Accession number BC021845

<400> 11
atgtacagcg agtacgtgaa gaactttgag cgggccgcgg aactgctggc cacgtggatg 60

gacaagtctc agcccttcca ggaggtggtc acccgcatcc agtgcagcga ggcctccagc 120

agcctgaccc tgcagcacca catgttggag cctgtgcaaa gaatcccgcg gtacgaactg 180

ctgctcaagg aatatgtgca gaagctgcca gcccaggccc cagacctcga agatgcccag 240

agagcactgg acatgatctt ctcagctgca cagcactcca atgcagccat tgcagagatg 300

gagcggctgc agggcctgtg ggatgtgtac cagcgcctgg gcttggagga tgacatcgtg 360

gacccctcca acaccctgct ccgagagggc cctgttctca agatctcttt ccgccgcagc 420

gacccaatgg aacgctacct ggttttgttc aacaacatgc ttctgtattg tgtaccccga 480

gtcctccaag tgggtgccca gttccaggtg cggactcgca tcgatgtggc cggcatgaag 540

gtgcgggagc tgaccgatgc tgagttccca cactccttcc tggtgtccgg aaagcagcgc 600

acactggagc tgcaggcccg gtcccgagat gaaatggttt cctggatgca ggcctgccag 660

gcagccattg accaggttga gaagcggagt gagaccttca aggctgctgt ccagggacct 720

cagggggaca cgcaggagcc caagccacag gtagaggagc tgggtctccg agcgcctcag 780

tgggtccggg acaagatggt gaccatgtgc atgcgctgcc aggagccctt caatgccctg 840

actcgtcggc gccaccactg ccgggcttgc ggctatgtag tgtgtgccaa gtgctctgac 900

taccgtgcgg agctgaaata tgacagcaac aggcccaacc gagtctgcct gacctgctac 960

acatttctca ctggaaacgt actccctcaa ggcaaggagg acaagaggcg gggcatcttg 1020

gagaaagagg cctcagcagc acccgagcag agtctggtgt gcagcttcct gcagctcata 1080

ggagacaagt gcagcaggag ccttccccgg agttggtgtg tgatcccccg ggatgacccc 1140

cttgtcctgt atgtctatgc agccccccag gacacaaagg ctcacacctc catcccccta 1200

ctgggctatc aggtgatttc agggccccag ggggaccctc gggttttcca gctgcaacag 1260

tcaggccagc agtacacctt caaggccgag tctgtggagc tgcagggccg ctgggtgaca 1320

gctatcaagc gtgcggccag tggccggacc cctgagggac ctgacgaaga agatgtgtct 1380

gactgac                                                          1387

<210>  12
<211>  1968
<212>  DNA
<213>  FGD2 mouse isoform 1 t6/tw5

<400>  12
atggagcgag cctgtgagaa gcaggactca gtgtgcaacc tggtggctgt gtttgagaac      60

aacagcagga ctccgggaga agcacctgga tcccatagcc ttgaggacca gctccatagc     120

cctgaacacc agctgtccct gtccccagag ccttgggagg caccccctgt caaggaggcc     180

ttgaagtctg aattccggcc agtgagcagg acatatctga gttccctcaa gaacaagcta     240

tcgagtgggg cttggaggag atcctgccag cctggggtca gcccggggcc agagacacag     300

gaacctgagg agaagagggt cgtgcgagag cttctggaga cggagcaggc ctatgtggct     360

cgcctgcacc tgcttgacca ggtgttcttc caggagctgc tgagggaggc aggccgcagc     420

aaggccttcc ctgaggacgt ggtgaagctc attttctcca acatctcctc catctatcgt     480

ttccacgccc agttcttcct tcccgagctg cagcggcgcg tggatgactg ggcagccacg     540

ccccgcattg gggatgtgat ccagaagctg gccccgtttc tgaaaatgta cagcgagtac     600

gtgaagaact ttgagcgggc cgcggaactg ctggccacgt ggatggacaa gtctcagccc     660

ttccaggagg tggtcacccg catccagtgc agcgaggcct cgggcagcct gaccctgcag     720

caccacatgt tggagcctgt gcaaagaatc ccgcggtacg aactgctgct caaggaatat     780

gtgcagaagc tgccagccca ggccccagac ctcgaagatg cccagagagc actggacatg     840

atcttctcag ctgcacagca ctccaatgca gccattgcag agatggagcg gctgcagggc     900

ctgtgggatg tgtaccagcg cctgggcttg gaggatgaca tcgtggaccc ctccaacacc     960

ctgctccgag agggccctgt tctcaagatc tctttccgcc gcagcgaccc aatggaacgc    1020

tacctggttt tgttcaacaa catgcttctg tattgtgtac cccgagtcct ccaagtgggt    1080

gcccagttcc aggtgcggac tcgcatcgat gtggccggca tgaaggtgcg ggagctgacc    1140

gatgctgagt tcccacactc cttcctggtg tccggaaagc agcgcacact ggagctgcag    1200

gcccggtccc gagatgaaat ggtttcctgg atgcaggcct gccaggcagc cattgaccag    1260

gttgagaagc ggagtgagac cttcaaggct gctgtccagg gacctcaggg ggacacgcag    1320

gagcccaagc cacaggtaga ggagctgggt ctccgagcgc ctcagtgggt ccgggacaag    1380

atggtgacca tgtgcatgcg ttgccaggag cccttcaatg ccctgactcg tcggcgccac    1440

cactgccggg cttgcggcta tgtagtgtgt gccaagtgct ctgactaccg tgcggagctg    1500

aaatatgaca gcaacaggcc caaccgagtc tgcctgacct gctacacatt tctcactgga    1560

aacgtactcc ctcaaggcaa ggaggacaag aggcggggca tcttggagaa agaggcctca    1620

gcagcacccg agcagagtct ggtgtgcagc ttcctgcagc tcataggaga caagtgcagc 1680

aggagccttc cccggagctg gtgtgtgatc ccccgggatg acccccttgt gctgtatgtc 1740

tatgcagccc cccaggacac aaaggctcac acctccatcc ccctactggg ctatcaggtg 1800

atttcagggc cccaggggga ccctcgggtt ttccagctgc aacagtcagg ccagcagtac 1860

accttcaagg ccgagtctgt ggagctgcag ggccgctggg tgacagctat caagcgtgcg 1920

gccagtggcc ggacccctga gggacctgac gaagaagatg tgtctgac 1968

<210> 13
<211> 5756
<212> DNA
<213> Tiam2 mouse wildtype; Accession number BC067048

<400> 13
cggagctaga gaagccttga ccttcccggc tcagcgagtg gagaacaaag tgacggagaa 60

aacttcgcgg agtgtgccct gagtccccac ggggcctcgc tcaggggcgc agagccgggc 120

tcgaccggtc ctctgtgttg aaacttggac cgaggatcac ttcatggatt tgcctcacgt 180

acctatgttt gttaatttcc tgtgtcgttc ccaagatcac gtaaaaccct ctgagcatta 240

ttgacctccc tcccagcaaa agccaggctg cagtgtaatt tagaccatgg ctcatattga 300

tacaactgtt ctcaattttg gttaaaatgg ggaactccga gagtcaatat accttccaag 360

gatccaagaa tcatagtaat actgtcactg gtgctaagca aaagccttgc tctctgaaaa 420

tacgcagcgt tcatgcaaaa gacgagaagt ccttacatgg ttggactcat gggagcagcg 480

gtgcaggcta caagtccagg tccctagccc gaagctgcct ttctcacttt aagaatcacc 540

agccttacgc caccagactc agtggaccca catgtaaagt ctcaaagggc accacctact 600

ctaagcacag agcaaatacc ccgggaaatg atttccaggg caacagtggt gctttcttac 660

ctgagaatgg cttccactat gttgaccgcg agtcagagga aagccatatc acctccaatg 720

ggcaccttct cacctgctat gggagaaagg aaagcctcgc ctccactcct ccaggcgagg 780

accacaggag ccccagggtg ctcatcaaga ccctgggaaa gctagacggg tgtttaagag 840

tcgagttcca caacggtggc aacccccaca aggggacctc cgaggacccc agtggacctg 900

tacggctgct gagatactcc cctaccttag catcggaaac ctgcccggtg cgggaaacca 960

ggcggcattc cgctgcaggc tccccatcca gccagcggcc ctctcccact gactctcgcc 1020

tgcgctccag caaaggcagc tccctgagct cagagtcatc ctggtatgac tcccccctgg 1080

gcaacgctgg ggaggtgagc gaggtggagg gctccttcct ggctcccagc actccagacc 1140

ccagcctccc cagcagcttc ccacccagtg acaccaaaaa gcctttcaac caaagctctt 1200

ccctctcctc cctccgggaa ttgtacaaag atcccaacct ggggtgccgc tcaccttccg 1260

gcacctgcct ttcttccaat gagtacatca gctctcaagt cagcctgaac aaccgagtct 1320

cctttgcgtc tgacatggat gtgccctcca gggtggatca cagggatccc ctgcactata 1380

gttcctttac tctcccctgt cgcaagtcca aagccttaac tgaagatgca gctaagaaag 1440
acaccctcaa agccagaatg cggcgcttca gtgactggac aggaagcctc tccaggaaga 1500
agaggaaact gcaggaaccc aggtccatgg agggcagtga gtactttgat agccactcag 1560
atggactgaa tgcagaaggg caggtgcccg cgcagacatc ttccttactg tggtcagggg 1620
gctcggctca gaccctgcct cacagaagcg aatccactca cgctatcagc gtcgatcccc 1680
tccgacagaa catctatgag aatttcatgc gagagctcga aatgagcagg agcaacacag 1740
aacacgtgga aacttccaca gagaccatgg agtccagcag cgagtctgtc agctcactgg 1800
agcagctgga tctgctcttt gagaaggagc agggagtggt ccggaaagcc gggtggctct 1860
tcttcaaacc ccttgtcacc ttgcagaagg agaggaaact ggagctggtg gctcggagga 1920
agtggaaaca atactgggtg accctgaaag gctgtactct gctgttttat gagacctatg 1980
gaaagaattc cacagagcag aatagtgccc cacggtgtgc cctctttgca gaggacagca 2040
tcgtgcagtc tgtcccagag catcccaaga aggaacacgt gttctgcctg agtaactcct 2100
gtggggacgt ctacctattc caggccacta gccagacaga tctggaaaac tgggtcacag 2160
ccatccactc gtgcgcatcc ctctttgcaa agaagcacgg aaaggaggac acggtgcgac 2220
tgctaaagag ccagaccaga agcctgcttc agaagataga catggatagc aagatgaaga 2280
agatggcaga gttgcagctg tctgtggtga gcgaccccaa gaacaggaag gccatcgaga 2340
atcagatccg gcaatgggag cagaatctgg aaaaattcca catggacctg ttccgcatgc 2400
gctgctattt ggcgagctta caaggtgggg agttaccaaa tcccaagagt ctccttgctg 2460
ccaccagccg cccctccaag ctggctcttg gcaggctggg cgtcctgtct gtttcgtctt 2520
tccatgctct ggtgtgttcc agagatgatt ccactctcag gaaaagaaca ctttccctta 2580
cccagagagg aaaaagcaag aaaggcatat ttcttcattg aaaggtctgg acaccctag 2640
caagaaaggg tagggagaag agagcttcca taactcagat gtttgattct agccacagcc 2700
atggatttct tggaactcag ctacctcaaa agtccactaa ctccaacaag gcccatgacc 2760
tgcatctgta tggctccgca gtagacagcg cgctgcgaga cagcatgtgg gaagtccaga 2820
cttatgtcca cttccaggat aacgaaggag ttactgtgac catcaagcca gagcacaggg 2880
tggaagatgt tctggctttg gtgtgcaaga tgagacagtt ggaacccact cactatggtc 2940
ttcagctccg aaaggtggtc gataaaagtg tggagtggtg tgtgcccgcg ctgtatgaat 3000
acatgcaaga gcaggtttat gatgaaatcg aagttttccc actcagtgtg tatgacgtgc 3060
agctaaccaa gactggggac atgactgact ttgggtttgc agtcacagtt caggtggacg 3120
aacaccagca tctcaaccgg atatttatca gtgatgttct ccctgacagc ctggcatacg 3180
gaggagggct gagaaagggc aacgaaatca caagcttaaa tggggaacca gtgtctgacc 3240
ttgacatcca gcagatggag gctttgtttt ctgagaagag cgttggcctc actctagtcg 3300

cccggcctgt gaccacaaga cgaaccctgt gtgcttcctg gtcagacagt gacctgttct 3360
ccaggaccca gaaaagtctg ccgccctctc ccaaccagtc ccagctgctg gaggaattcc 3420
tggataactt tagaaaaacc gccacgagtg atttcagcaa tgtccctgag atcacaactg 3480
gcttgaagag gagccagaca gaaggcaccc tggatcaagt gccccacagg gaaaagatgg 3540
agcagacatt cctgagcgct gaccagattg cggagctctg cagggacttg aacaacaccc 3600
acaccaacag tatggaagca ccaacagaga gccatgaccc acctcccagg cctctggctc 3660
gtcacctctc agatgcagat cgcctccgga aagtcatcca ggagcttgta gacacagaga 3720
agtcttacgt gaaggatctg agctgcctct ttgaactata cttggagcca cttcagaatg 3780
agacctttct tacccaagat gagatggagt cactttttgg gagcctgcca gagatgctgg 3840
agtttcaaaa ggtgttcctg gagacgttgg aggatgcgat ctccgcttcc tcggacttta 3900
gtgtcctgga acccccctca cagtttcgaa aattgctgtt ctcccttgga ggttctttcc 3960
tctactatgc ggatcacttt aagctataca gtgggttctg tgccaaccac attaaagtac 4020
agagggttct agagcgagct aaaacggaca aggccttcaa ggcttttctg gatgcccgaa 4080
atcccaccaa gcagcactcc tccacgctgg agtcctatct catcaagcct gttcagagag 4140
tgctcaagta tcctctgctt ctcaaggagc tagtgtcact gactgaccat gagagtgaag 4200
aacactatca cctgacagaa gcactaaagg ccatggaaaa agtcgccagt cacatcaatg 4260
agatgcagaa gatctacgag gattacggga tggtgtttga ccagctggtg gcagagcaga 4320
gtggcacaga gaaggaagtg acagagctgt ccatggggga acttctgatg cactctacag 4380
tttcctggtt gaatccgttc ctgtctctag gaaaagccag gaaggacatt gagctcacag 4440
tatttgtttt taagagagct gtcatactgg tttataaaga aaactgcaag ctgaaaaaga 4500
aactgccctc gaattcccgg cctgctcaca actctgctga cttggatcca tttaaattcc 4560
gctggttgat tcccatatct gcgcttcaag ttagactggg gaacacggca gggactgaaa 4620
ataattccac gtgggagctg attcatacca agtcggaaat tgaaggacgg ccagaaacca 4680
tctttcaact gtgctgcagt gacagcgaga acaaaaccag cattgttaag gtgattcgtt 4740
ctattctgag agagaacttc cggcgccaca taaagtgtga gctgccactg gagaagacgt 4800
gtaaggaccg gctagtacct cttaagaacc gagttcctgt ttcagccaaa ttagcctcgt 4860
ccaggtcgtt gaagggcctc agaacatcct ccagcagcga gtggcccagc gagcccagca 4920
agggcaactc actggactca gatgagtgca gcctgagcag tggcacccag agtagcggct 4980
gccccgtagc cgagagcagg cgagactcta agagcaccga gctggagaaa gacgctcagg 5040
agggcctggc ggagtttcca gatggtctta tcaaagaaag cgacattctg agtgatgaag 5100
atgaggactt ccaccaccct ctgaaacagg gtagccctac taaggacatt gagattcagt 5160
tccagagact gaaaatctct gaggaatccg acgtgcaccc agttgggcag cagcctctca 5220

cagagtcagg tgaacagccc aagctggtca ggggccattt ttgccccatt aaacggaaag 5280

caaacagcac caagaggggc agaggaactt tgctcaaggc gcagactcgt caccagtccc 5340

tggacagcca cccagaaact gccagcattg atctaaactt ggtcctggag agagaattca 5400

gtgtccagag cttaacttca gtcgtcaatg aggagggttt ttatgaaaca cagagccatg 5460

gcaaatcatg atttgtgtag tgtgcttaca atgctcattt gttttcaaat tggtggaaaa 5520

gagaaactgc aggaaaacta ttcatgtttg agttttacgc agtatatttt ttccacaaaa 5580

cctgtaaaga tttaagttat tttaatttat tgtgaatccg aaaactagat ttagttggtc 5640

agaatctgta aagtactagc acctatccct ttggagcagc aatcaaatga tttagtcttg 5700

taataaaatt gcattttaat taaaaaaaaa aaaaaaaaaa aaaaaaaaaa aaaaaa 5756

<210> 14
<211> 5302
<212> DNA
<213> Tiam2 homo sapiens Accession number NM_012454

<400> 14
gctcacttca tggactcact ttgcgtgctt gttaaatgtg ctgtgttgct cccaagacca 60

tgtaaagcct actgaccact aacctccctc acagcagaaa ctagacgtca ggttaaaatg 120

ggcaactccg acagtcagta cacccttcaa ggatctaaaa atcatagcaa tactattact 180

ggtgctaagc aaattccttg ctccctgaaa atacgtggca ttcatgcaaa agaggaaaag 240

tcattgcatg gatggggtca cggaagcaac ggagcaggtt acaagtccag gtccctggcc 300

cgaagctgcc tttctcactt taagagtaac cagccttacg catcgagact cggtggcccc 360

acatgcaagg tctccagagg tgttgcctac tccacgcaca ggacaaatgc cccagggaag 420

gatttccagg gcatcagtgc tgctttctca actgagaatg gcttccactc tgttggccac 480

gagctggcag ataaccacat cacctccaga gactgcaacg gacaccttct caactgctac 540

gggaggaatg agagcattgc ctccacccca ccgggcgaag accgcaagag cccccgagtg 600

ctcatcaaaa cgctggggaa gctggatggg tgtttaaggg tcgagttcca caatggtggc 660

aaccccagca aagtgcctgc agaggactgc agtgagccgg tgcagctgct gaggtactca 720

cctaccttag catcggaaac ctcccctgtg cctgaagcca ggagggggtc cagcgccgat 780

tccctgccca gccatcgccc ctctcccacg gactctcgcc tgcggtccag caaaggcagc 840

tccctgagtt ctgagtcatc ctggtacgac tccccttggg gcaatgctgg agagctgagc 900

gaggctgagg gctccttcct ggcccccggc atgcctgacc ccagtctcca tgccagcttc 960

ccacctggcg atgccaaaaa gcctttcaac caaagctctt ccctctcctc cctccgggaa 1020

ctgtacaaag atgccaacct ggggagcctc tccccctcag gtatccgcct ttctgatgaa 1080

tacatgggca cgcatgccag cctgagcaac cgtgtctctt ttgcttccga cattgatgtg 1140

ccctccagag tggcacacgg ggaccccatc cagtacagtt ccttcactct cccctgtcgg 1200
aagcccaaag cctttgttga ggatactgcg aagaaggact ccctcaaagc caggatgcga 1260
cggatcagtg actggacggg aagcctctca aggaagaaaa ggaaactcca ggagccgagg 1320
tccaaggagg gcagtgacta ctttgacagt cgctctgatg gactgaatac agatgtgcag 1380
ggatcctccc aggcatctgc ttttctgtgg tcagggggct ctactcagat cctgtctcag 1440
agaagtgaat ccacacatgc gattggcagc gatcccctcc ggcagaacat ttatgagaat 1500
ttcatgcgag agttggaaat gagcaggacc aacactgaga acatagaaac atctacagaa 1560
accgccgagt ccagcagcga gtcactcagc tctctggaac agctggatct gctctttgag 1620
aaggaacagg gggtggtccg gaaggccggg tggctcttct tcaagcccct ggtcactgtg 1680
cagaaggaaa ggaagcttga gctggtggca cgaaggaaat ggaaacagta ctgggtaacg 1740
ctgaaaggat gcacgctgct gttttatgag acctatggga agaattccat ggatcagagc 1800
agtgcccctc ggtgtgctct gtttgcagaa gacagcatag tgcagtctgt tccagagcat 1860
cccaagaaag aaaatgtgtt ctgcctcagc aactcctttg gagatgtcta ccttttccag 1920
gccaccagcc agacagatct agaaaactgg gtcactgctg tacactctgc ttgtgcatcc 1980
ctttttgcaa agaagcatgg gaaagaggac acgctgcggc tgctgaagaa ccagaccaaa 2040
aacctgcttc agaagataga catggacagc aagatgaaga agatggcaga gctgcagctg 2100
tccgtggtga gcgacccaaa gaacaggaaa gccatagaga accagatcca gcaatgggag 2160
cagaatcttg agaaatttca catggatctg ttcaggatgc gctgctatct ggccagccta 2220
caaggtgggg agttaccgaa cccaaagagt ctccttgcag ccgccagccg cccctccaag 2280
ctggccctcg gcaggctggg catcttgtct gtttcctctt tccatgctct ggtatgttct 2340
agagatgact ctgctctccg gaaaaggaca ctgtcactga cccagcgagg gagaaacaag 2400
aagggaatat tttcttcgtt aaaagggctg gacacactgg ccagaaaagg caaggagaag 2460
agaccttcta taactcaggt cgatgaactt ctgcatatat atggttcaac agtagacggt 2520
gttccccgag acaatgcatg ggaaatccag acttatgtcc actttcagga caatcacgga 2580
gttactgtag ggatcaagcc agagcacaga gtagaagata ttttgacttt ggcatgcaag 2640
atgaggcagt tggaacccag ccattatggc ctacagcttc gaaaattagt agatgacaat 2700
gttgagtatt gcatccctgc accatatgaa tatatgcaac aacaggttta tgatgaaata 2760
gaagtctttc cactaaatgt ttatgacgtg cagctcacga agactgggag tgtgtgtgac 2820
tttgggtttg cagttacagc gcaggtggat gagcgtcagc atctcagccg gatatttata 2880
agcgacgttc ttcccgatgg cctggcgtat ggggaagggc tgagaaaggg caatgagatc 2940
atgaccttaa atggggaagc tgtgtctgat cttgacctta agcagatgga ggccctgttt 3000
tctgagaaga gcgtcggact cactctgatt gcccggcctc cggacacaaa agcaaccctg 3060

tgtacatcct ggtcagacag tgacctgttc tccagggacc agaagagtct gctgccccct 3120

cctaaccagt cccaactgct ggaggaattc ctggataact ttaaaaagaa tacagccaat 3180

gatttcagca acgtccctga tatcacaaca ggtctgaaaa ggagtcagac agatggcact 3240

ctggatcagg tttcccacag ggagaaaatg gagcagacat tcaggagtgc tgagcagatc 3300

actgcactgt gcaggagttt taacgacagt caggccaacg gcatggaagg accgcgggag 3360

aatcaggatc ctcctccgag gcctctggcc cgccacctgt ctgatgcaga ccgcctccgc 3420

aaagtcatcc aggagcttgt ggacacagag aagtcctacg tgaaggattt gagctgcctc 3480

tttgaattat acttggagcc acttcagaat gagacctttc ttacccaaga tgagatggag 3540

tcactttttg gaagtttgcc agagatgctt gagtttcaga aggtgtttct ggagaccctg 3600

gaggatggga tttcagcatc atctgacttt aacaccctag aaaccccctc acagtttaga 3660

aaattactgt tttcccttgg aggctctttc ctttattacg cggaccactt taaactgtac 3720

agtggattct gtgctaacca tatcaaagta cagaaggttc tggagcgagc taaaactgac 3780

aaagccttca aggcttttct ggacgcccgg aaccccacca agcagcattc ctccacgctg 3840

gagtcctacc tcatcaagcc ggttcagaga gtgctcaagt acccgctgct gctcaaggag 3900

ctggtgtccc tgacggacca ggagagcgag gagcactacc acctgacgga agcactaaag 3960

gcaatggaga aagtagcgag ccacatcaat gagatgcaga agatctatga ggattatggg 4020

accgtgtttg accagctagt agctgagcag agcggaacag agaaggaggt aacagaactt 4080

tcgatgggag agcttctgat gcactctacg gtttcctggt tgaatccatt tctgtctcta 4140

ggaaaagcta gaaaggacct tgagctcaca gtatttgttt ttaagagagc cgtcatactg 4200

gtttataaag aaaactgcaa actgaaaaag aaattgccct cgaattcccg gcctgcacac 4260

aactctactg acttggaccc atttaaattc cgctggttga tccccatctc cgcgcttcaa 4320

gtcagactgg ggaatccagc agggacagaa aataattcca tatgggaact gatccatacg 4380

aagtcagaaa tagaaggacg gccagaaacc atctttcagt tgtgttgcag tgacagtgaa 4440

agcaaaacca acattgttaa ggtgattcgt tctattctga gggagaactt caggcgtcac 4500

ataaagtgtg aattaccact ggagaaaacg tgtaaggatc gcctggtacc tcttaagaac 4560

cgagttcctg tttcggccaa attagcttca tccaggtctt taaaagtcct gaagaattcc 4620

tccagcaacg agtggaccgg tgagactggc aagggaacct tgctggactc tgacgagggc 4680

agcttgagca gcggcaccca gagcagcggc tgccccacgg ctgagggcag gcaggactcc 4740

aagagcactt ctcccgggaa atacccacac cccggcttgg cagattttgc tgacaatctc 4800

atcaaagaga gtgacatcct gagcgatgaa gatgatgacc accgtcagac tgtgaagcag 4860

ggcagcccta ctaaagacat cgaaattcag ttccagagac tgaggatttc cgaggaccca 4920

gacgttcacc ccgaggctga gcagcagcct ggcccggagt cgggtgaggg tcagaaagga 4980

ggagagcagc ccaaactggt ccgggggcac ttctgcccca ttaaacgaaa agccaacagc 5040

accaagaggg acagaggaac tttgctcaag gcgcagatcc gtcaccagtc ccttgacagt 5100

cagtctgaaa atgccaccat cgacctaaat tctgttctag agcgagaatt cagtgtccag 5160

agtttaacat ctgttgtcag tgaggagtgt ttttatgaaa cagagagcca cggaaaatca 5220

tagtatgatt caatccagat atgggttaaa ttcctcattt tacttttaaa ctggtggtaa 5280

agtggaaatt gcaaaaaaaa aa 5302

<210> 15
<211> 2076
<212> DNA
<213> Tcr, short 5'utr mus musculus

<400> 15
gtttgggagg agcttgtgtg tgtgagttgt gttttaagtt tatttgcgtg tgagtacctt 60

tgggtttttg tgtgtgtctg tgtgtgtttg tgtgtgtata actgtgggtg actgtaagtg 120

cacctgtgtg tttgtacgtg agtgtgtaag actgtgtgtg tgcacaagag cgtgtgtagg 180

tgcacgtgtt gtaggtgtga gaacacctgt tgtgtttagg ccatcagtca gcttggtcat 240

tgtttctaag gtagcattta tactttgtta cctcaagtgg gctctgggag tcaacagaag 300

tcagaaaagc tcagatccaa gccccctttt tctgacatgg agaaatttca tgctcaatat 360

gagatgctag agactattgg ccagggaggc tgcgcccagg tgaagctggc ccgacaccgc 420

ctcacaggca cccacgtggc tgtcaaagtg attgtaaaga gggagtgttg gttcaaccct 480

gtcatgtctg aggcagagtt actgatgatg accgatcatc cgaatatcat ctctctcctt 540

caagtcattg agaccaagaa gaaagtatac ctcattatgg agttgtgcga gggtaaatca 600

ctttaccaac acatccaaaa tgctggctac ctgcaggagg atgaagcacg cccattattc 660

aagcagctct taagtgctat gaactactgc cacaaccagg gtatagttca cagggacctg 720

acacctgaca atattatggt agaaaaagat gggaaagtga agatcattga ttttggactc 780

ggcacccaag agaagccagg gcaaaaccac aacttattct gtgagattta cccatttagt 840

actcctgagg tgctctttaa cagaccctat gatatgcgca agatcgatgt gtggggtctt 900

ggagttgtgc tgtattttat ggtaactgga aagattctgt ttgatactgc cagcgtagaa 960

aagctgcgaa agcaaattgt tgcagaaaag tgttctgttc cctgtagact gtcagtagag 1020

ctccaagacc tgattagact tttaatgacg gacatccccg aacttaggcc cactgttgct 1080

gaagttatgg tgcatccctg ggtcacagaa ggctcagggg tgttaccaga tccttgtgaa 1140

gaacatatac ccctcaagcc agaccctgcg attgcaaaag caatgggatt tatcgggttc 1200

caagctcaag acattgaaga ttcgttatgt cagagaaaat tcaacgaaac catggcatct 1260

tattgtctac tgaaaaaaca gattcttaag gaatgtgaca ggccaatccg ggctcagccc 1320

atgaatccat ctgtgacccc actctcttcc cttgttgatg ctcctacttt ccatctcgga 1380

cttcggagga cagagactga acccacaggt ctcagattat ctgacaataa ggaagtgcct 1440

gtctgtggca atagtactag taagaaaaga gagagaagtt tcagtgggcc gggtgttctc 1500

agcaggccga ttaacacaac acccacaatg gaccaaacac acacccgtac ttggagtggt 1560

ccctgcattt actcaaatgt ttgcacaatc catccaaaca gcatcaatga gagtacagaa 1620

ggccacatca gtacctcagc agaggataag cctgtccaca gcagaggctg gcccagaggc 1680

atcaagggct ggactaggaa gataggaaat gcaatgagga agctctgttg ctgtatccca 1740

tccaaagaga catctcacct ggggcagaga agagtctgcc caaaaattta agacacagga 1800

aggatgtcag gagaatgagc atccagcatg gcccagcctt tcagaccgaa ggcaagctct 1860

acctgatcct ggacttcctg cggggaggtg acctcttcac caggctttcc aaagaggtga 1920

tgttcacgga ggaggatgtc aagttctacc tggctgagct ggccttggct ctagaccacc 1980

tccatggcct ggggatcatc tacagggatc tgaagccaga gaatatcctc ctggatgaag 2040

agggacatat taagatcaca gattttggct tgagca 2076

<210> 16
<211> 2827
<212> DNA
<213> Tcr, long 5'utr mus musculus

<400> 16
   gagaggagtt ggtggagttg gtggagtttg gtggatttgg tggagttggt ggtgcccttt 60
   gcgatttcgt tgtatctagt gagccgtgtg tggattttgt gtttgattgg ttcgtgtgtg 120
   agcttttgtg tgtgtgtgtg tgtgtgtgtg tgtgtgtgtg tgtgtgtgtg tgtgtagatc 180
   agtgtgtgtt tgggaggagc ttgtgtgtgt gagttgtgtt ttaagtttat ttgcgtgtga 240
   gtacctttgg gtttttgtgt gtgtctgtgt gtgtttgtgt gtgtataact gtgggtgact 300
   gtaagtgcac ctgtgtgttt gtacgtgagt gtgtaagact gtgtgtgtgc acaagagcgt 360
   gtgtaggtgc acgtgttgta ggtgtgagaa cacctgttgt gtttaggcca tcagtcagct 420
   tggtcattgt ttctaaggta gcatttatac tttgttacct caagtgggct ctgggagtca 480
   acagaagtca gaaaagctca gatccaagcc cctttttctt gacatggaga aatttcatgc 540
   tcaatatgag atgctagaga ctattggcca gggaggctgc gcccaggtga agctggcccg 600
   acaccgcctc acaggcaccc acgtggctgt caaagtgatt gtaaagaggg agtgttggtt 660
   caaccctgtc atgtctgagg cagagttact gatgatgacc gatcatccga atatcatctc 720
   tctccttcaa gtcattgaga ccaagaagaa agtatacctc attatggagt tgtgcgaggg 780
   taaatcactt taccaacaca tccaaaatgc tggctacctg caggaggatg aagcacgccc 840
   attattcaag cagctcttaa gtgctatgaa ctactgccac aaccagggta tagttcacag 900
   ggacctgaca cctgacaata ttatggtaga aaaagatggg aaagtgaaga tcattgattt 960
   tggactcggc acccaagaga agccagggca aaaccacaac ttattctgtg agatttaccc 1020
   atttagtact cctgaggtgc tctttaacag accctatgat atgcgcaaga tcgatgtgtg 1080
   gggtcttgga gttgtgctgt attttatggt aactggaaag attctgtttg atactgccag 1140
   cgtagaaaag ctgcgaaagc aaattgttgc agaaaagtgt tctgttccct gtagactgtc 1200
   agtagagctc caagacctga ttagactttt aatgacggac atccccgaac ttaggcccac 1260
   tgttgctgaa gttatggtgc atccctgggt cacagaaggc tcaggggtgt taccagatcc 1320
   ttgtgaagaa catatacccc tcaagccaga ccctgcgatt gcaaaagcaa tgggatttat 1380
   cgggttccaa gctcaagaca ttgaagattc gttatgtcag agaaaattca acgaaaccat 1440
   ggcatcttat tgtctactga aaaaacagat tcttaaggaa tgtgacaggc caatccgggc 1500
   tcagcccatg aatccatctg tgaccccact ctcttccctt gttgatgctc ctactttcca 1560
   tctcggactt cggaggacag agactgaacc cacaggtctc agattatctg acaataagga 1620
   agtgcctgtc tgtggcaata gtactagtaa gaaaagagag agaagtttca gtgggccggg 1680
   tgttctcagc aggccgatta acacaacacc cacaatggac caaacacaca cccgtacttg 1740
   gagtggtccc tgcatttact caaatgtttg cacaatccat ccaaacagca tcaatgagag 1800
   tacagaaggc cacatcagta cctcagcaga ggataagcct gtccacagca gaggctggcc 1860
   cagaggcatc aagggctgga ctaggaagat aggaaatgca atgaggaagc tctgttgctg 1920
   tatcccatcc aaagagacat ctcacctggg gcagagaaga gtctgcccaa aaatttaaga 1980
   cacaggaagg atgtcaggag aatgagcatc cagcatggcc cagcctttca gaccgaaggc 2040
   aagctctacc tgatcctgga cttcctgcgg ggaggtgacc tcttcaccag gctttccaaa 2100
   gaggtgatgt tcacggagga ggatgtcaag ttctacctgg ctgagctggc cttggctcta 2160
   gaccacctcc atggcctggg gatcatctac agggatctga agccagagaa tatcctcctg 2220
   gatgaagagg gacatattaa gatcacagat tttggcttga gcaaggaggc caccgaccat 2280
   gacaagagag cctattcatt ttgtgggact attgaataca tggcgcccga ggtggtgaac 2340
   cggcgtggac acacacagag tgccgactgg tggtccttcg gtgtgctcat gttcgagatg 2400
   ctcacagggt ccctgccatt ccaggggaag gacaggaagg aaacaatggc ccgcatcctc 2460
   aaagcaaagc tgggtatgcc ttagttcctc agtgcggagg ctcagagcct gctcagggcc 2520
   cttttcaagc ggaacccctg caaccggcta ggtaagggtc cctgtgacac ccccacccca 2580
   ggaatgcaat gaggctgccc tctagacccc ccttaggaat gtgagaggcc accattctgt 2640
   tccccacggg atgtggagga cttcctcctt atgccccaac tctgaactgt atgcttttcc 2700
   ttgctaaggt tgcaggaagc agaggtaccc cgacgctggg gaaacactca catgtggcct 2760
   ggcgcccaca ggcacgtgga cttatcagga ttgctgaaag gcatttgaaa aaaaaaaaaa 2820
   aaaaaaa 2827

<210>  17
<211>  714
<212>  PRT
<213>  Tagap1 mouse wildtype FVB/N

<400>  17

Met Lys Leu Ile Ser Ser Leu Asp Gly Ser Lys Thr Leu Asn Ala Asn
1               5                   10                  15

Asn Met Glu Thr Leu Ile Glu Cys Gln Ser Glu Gly Asp Ile Lys Val
            20                  25                  30

Pro Pro Leu Leu Thr Ser Cys Glu Ser Glu Asp Ser Ile Cys Gln Leu
        35                  40                  45

Thr Glu Ile Lys Lys Arg Lys Lys Val Leu Ser Trp Pro Ser Leu Met
    50                  55                  60

Arg Lys Leu Ser Pro Ser Ser Asp Phe Ser Gly Ser Leu Glu Pro Glu
65                  70                  75                  80

Leu Lys Val Ser Leu Phe Asp Gln Pro Leu Ser Ile Ile Cys Gly Glu
                85                  90                  95

Asn Asp Thr Leu Pro Arg Pro Ile Gln Asp Ile Leu Thr Ile Leu Cys
            100                 105                 110

Leu Lys Gly Pro Ser Thr Glu Gly Ile Phe Arg Lys Ala Ala Ser Glu
        115                 120                 125

Lys Ala Arg Lys Glu Leu Lys Glu Gly Leu Asn Cys Gly Val Ser Val
    130                 135                 140

Asn Leu Lys Gln Leu Pro Val His Leu Leu Ala Val Val Phe Lys Asp
145                 150                 155                 160

Phe Leu Arg Gly Ile Pro Leu Lys Leu Leu Ser Cys Asp Leu Phe Glu
                165                 170                 175

Asp Trp Met Gly Ala Leu Glu Lys Pro Thr Glu Glu Asp Arg Ile Glu
            180                 185                 190

Ala Leu Lys Gln Val Ala Gly Gly Leu Pro Arg Pro Asn Leu Leu Leu
        195                 200                 205

Leu Arg His Leu Leu Tyr Val Leu His Leu Ile Ser Lys Asn Ala Glu
    210                 215                 220

Val Asn Lys Met Asp Ser Ser Asn Leu Ala Ile Cys Ile Gly Pro Asn
225                 230                 235                 240

Met Leu Thr Leu Lys Asn Asp Gln Ser Leu Ser Phe Gln Ala Gln Lys
                245                 250                 255

Asp Leu Asn Asn Lys Val Lys Ile Leu Val Glu Phe Leu Ile Asp Asn
            260                 265                 270

Cys Phe Glu Ile Phe Gly Glu Asn Ile Arg Thr Arg Ser Arg Ile Thr
        275                 280                 285

Ser Asp Asp Ser Leu Glu His Thr Asp Ser Ser Asp Val Ser Thr Leu
    290                 295                 300

Gln Asn Asp Ser Ala Tyr Asp Ser Asn Asp Pro Asp Val Glu Pro Thr
305                 310                 315                 320

Ser Gly Ala Ala Ser Pro Asn Arg Gln Leu Glu Gly Pro Thr Pro Thr
                325                 330                 335

Met Ala Gly Leu Asp Thr Arg Gly Gln Arg Asp Thr Cys Glu Ser Ser
            340                 345                 350

Ser Glu Ser Ser Val Ser Met Val Val Arg Leu Lys Ser Ser Ile Val
        355                 360                 365

Gln Gln Asp Arg Arg Phe Ser Glu Pro Asn Met Ser Pro Ser Arg Glu
    370                 375                 380

Cys Leu Val Gly Pro Thr Ser Lys Gln Lys Leu Ala Arg Ser Glu Asp
385                 390                 395                 400

Ser Phe Thr Leu Ser Gln Asp Ala Ser Cys Ser Glu Gly Asp Glu Ala
                405                 410                 415

Glu Asp Pro Phe Thr Glu Glu Val Phe Pro Ala Val Asp Ser Lys Pro
            420                 425                 430

Lys Arg Pro Val Asp Leu Lys Ile Lys Asn Trp Thr Gln Gly Leu Ala
        435                 440                 445

Ser Pro Gln Gly His Ile Thr Lys Ala Phe Ser Arg Ser Ser Pro Gly
    450                 455                 460

Glu Ser Leu Gly Ser Ser Pro Val Pro Ser Pro Ser Cys Pro Lys Arg
465                 470                 475                 480

Asn Phe Phe Thr Arg His Gln Ser Phe Thr Thr Lys Thr Asp Lys Thr
                485                 490                 495

Lys Pro Gln Arg Glu Ile Arg Lys His Ser Met Ser Phe Ser Phe Ala
            500                 505                 510

Ser His Lys Lys Val Leu Pro Arg Thr Ser Ser Ile Gly Ser Glu Lys
        515                 520                 525

Ser Lys Asp Phe Ser Arg Asp Gln Leu Gln Lys Asp Leu Arg Lys Glu
    530                 535                 540

Ser Gln Leu Ser Gly Arg Ile Val Gln Glu Asn Glu Ser Glu Ile Gln
545                 550                 555                 560

Ser Gln Thr Ser Leu Gly Phe Ser Leu Ser Gly Thr Trp Ala Leu Ser
                565                 570                 575

Val Asp Asn Thr Phe Gln Leu Val Asp Met Arg Lys Pro Gly Ser Pro
            580                 585                 590

Pro Ser Tyr Glu Glu Ala Ile Tyr Tyr Gln Thr Ser Gly Leu Thr Ala
        595                 600                 605

Tyr Gly Gly Gln Thr Val Gly Ser Met Arg Ser Arg Met Phe Lys Pro
    610                 615                 620

Ser Thr Ala Val Pro Pro Val Pro Ser His His Gly Gly Asp Leu Ser
625                 630                 635                 640

Glu Gly Thr Pro Gly Gly His Arg Leu Ser Ser Val Thr Glu His Trp
                645                 650                 655

Thr His Ser Gln Thr Val His Val Ser Ile Glu Thr Gln Gly Arg Ser
            660                 665                 670

Glu Leu His Gln Leu Arg Thr Val Ser Glu Ser Met Gln Lys Ala Lys
        675                 680                 685

Leu Asp Cys Leu Gly Pro Gln His Ser His Leu Val Phe Glu Ala Asp
    690                 695                 700

Gln Leu Cys Cys Ala Arg Glu Ser Tyr Ile
705                 710

<210>  18
<211>  714
<212>  PRT
<213>  Tagap1 mouse wildtype BALB/c

<400>  18

Met Lys Leu Ile Ser Ser Leu Asp Gly Ser Lys Thr Leu Asn Ala Asn
1               5                   10                  15

Asn Met Glu Thr Leu Ile Glu Cys Gln Ser Glu Gly Asp Ile Lys Val
            20                  25                  30

Pro Pro Leu Leu Thr Ser Cys Glu Ser Glu Asp Ser Ile Cys Gln Leu
        35                  40                  45

Thr Glu Ile Lys Lys Arg Lys Lys Val Leu Ser Trp Pro Ser Leu Met
    50                  55                  60

Arg Lys Leu Ser Pro Ser Ser Asp Phe Ser Gly Ser Leu Glu Pro Glu
65                  70                  75                  80

Leu Lys Val Ser Leu Phe Asp Gln Pro Leu Ser Ile Ile Cys Gly Glu
                85                  90                  95

Asn Asp Thr Leu Pro Arg Pro Ile Gln Asp Ile Leu Thr Ile Leu Cys
            100                 105                 110

Leu Lys Gly Pro Ser Thr Glu Gly Ile Phe Arg Lys Ala Ala Ser Glu
        115                 120                 125

Lys Ala Arg Lys Glu Leu Lys Glu Gly Leu Asn Cys Gly Val Ser Val
    130                 135                 140

Asn Leu Lys Gln Leu Pro Val His Leu Leu Ala Val Val Phe Lys Asp
145                 150                 155                 160

Phe Leu Arg Gly Ile Pro Leu Lys Leu Leu Ser Cys Asp Leu Phe Glu
                165                 170                 175

Asp Trp Met Gly Ala Leu Glu Lys Pro Thr Glu Glu Asp Arg Ile Glu
            180                 185                 190

Ala Leu Lys Gln Val Ala Gly Gly Leu Pro Arg Pro Asn Leu Leu Leu
        195                 200                 205

Leu Arg His Leu Leu Tyr Val Leu His Leu Ile Ser Lys Asn Ala Glu
    210                 215                 220

Val Asn Lys Met Asp Ser Ser Asn Leu Ala Ile Cys Ile Gly Pro Asn
225                 230                 235                 240

Met Leu Thr Leu Lys Asn Asp Gln Ser Leu Ser Phe Gln Ala Gln Lys

245                 250                 255

Asp Leu Asn Asn Lys Val Lys Ile Leu Val Glu Phe Leu Ile Asp Asn
            260                 265                 270

Cys Phe Glu Ile Phe Gly Glu Asn Ile Arg Thr Arg Ser Arg Ile Thr
        275                 280                 285

Ser Asp Asp Ser Leu Glu His Thr Asp Ser Ser Asp Val Ser Thr Leu
    290                 295                 300

Gln Asn Asp Ser Ala Tyr Asp Ser Asn Asp Pro Asp Val Glu Pro Thr
305                 310                 315                 320

Ser Gly Ala Ala Ser Pro Asn Arg Gln Leu Glu Gly Pro Thr Pro Thr
                325                 330                 335

Met Ala Gly Leu Asp Thr Arg Gly His Arg Asp Thr Cys Glu Ser Ser
            340                 345                 350

Ser Glu Ser Ser Val Ser Met Val Val Arg Leu Lys Ser Ser Ile Val
        355                 360                 365

Gln Gln Asp Arg Arg Phe Ser Glu Pro Asn Met Ser Pro Ser Arg Glu
    370                 375                 380

Cys Leu Val Gly Pro Thr Ser Lys Gln Lys Leu Ala Arg Ser Glu Asp
385                 390                 395                 400

Ser Phe Thr Leu Ser Gln Asp Ala Ser Cys Ser Glu Gly Asp Glu Ala
                405                 410                 415

Glu Asp Pro Phe Thr Glu Glu Val Phe Pro Ala Val Asp Ser Lys Pro
            420                 425                 430

Lys Arg Pro Val Asp Leu Lys Ile Lys Asn Trp Thr Gln Gly Leu Ala
        435                 440                 445

Ser Pro Gln Gly His Ile Thr Lys Ala Phe Ser Arg Ser Ser Pro Gly
    450                 455                 460

Glu Ser Leu Gly Ser Ser Pro Val Pro Ser Pro Ser Cys Pro Lys Arg
465                 470                 475                 480

Asn Phe Phe Thr Arg His Gln Ser Phe Thr Thr Lys Thr Asp Lys Thr
                485                 490                 495

Lys Pro Gln Arg Glu Ile Arg Lys His Ser Met Ser Phe Ser Phe Ala

500                 505                 510

Ser His Lys Lys Val Leu Pro Arg Thr Ser Ser Ile Gly Ser Glu Lys
        515                 520                 525

Ser Lys Asp Phe Ser Arg Asp Gln Leu Gln Lys Asp Leu Arg Lys Glu
    530                 535                 540

Ser Gln Leu Ser Gly Arg Ile Val Gln Glu Asn Glu Ser Glu Ile Gln
545                 550                 555                 560

Ser Gln Thr Ser Leu Gly Phe Ser Leu Ser Gly Thr Trp Ala Leu Ser
                565                 570                 575

Val Asp Asn Thr Phe Gln Leu Val Asp Met Arg Lys Pro Gly Ser Pro
            580                 585                 590

Pro Ser Tyr Glu Glu Ala Ile Tyr Tyr Gln Thr Ser Gly Leu Thr Ala
        595                 600                 605

Tyr Gly Gly Gln Thr Val Gly Ser Met Arg Ser Arg Met Phe Lys Pro
    610                 615                 620

Ser Thr Ala Val Pro Pro Val Pro Ser His His Gly Gly Asp Leu Ser
625                 630                 635                 640

Glu Gly Thr Pro Gly Gly His Arg Leu Ser Ser Val Thr Glu His Trp
                645                 650                 655

Thr His Ser Gln Thr Val His Val Ser Ile Glu Thr Gln Gly Arg Ser
            660                 665                 670

Glu Leu His Gln Leu Arg Thr Val Ser Glu Ser Met Gln Lys Ala Lys
        675                 680                 685

Leu Asp Cys Leu Gly Pro Gln His Ser His Leu Val Phe Glu Ala Asp
    690                 695                 700

Gln Leu Cys Cys Ala Arg Glu Ser Tyr Ile
705                 710

<210>  19
<211>  442
<212>  PRT
<213>  Tagap1 t1 mouse

<400>  19

Met Lys Leu Ile Ser Ser Leu Asp Gly Ser Lys Thr Leu Asn Ala Asn
1               5                   10                  15

Asn Met Glu Thr Leu Ile Glu Cys Gln Ser Glu Gly Asp Ile Lys Val
            20                  25                  30

Pro Pro Leu Leu Thr Ser Cys Glu Ser Glu Asp Ser Ile Cys Gln Leu
        35                  40                  45

Ile Glu Ile Lys Lys Arg Lys Lys Val Leu Ser Trp Pro Ser Leu Met
    50                  55                  60

Arg Lys Leu Ser Pro Ser Ser Asp Phe Ser Gly Ser Leu Glu Pro Glu
65                  70                  75                  80

Leu Lys Val Ser Leu Phe Asp Gln Pro Leu Ser Ile Ile Cys Gly Glu
                85                  90                  95

Asn Asp Thr Leu Pro Arg Pro Ile Gln Asp Ile Leu Thr Ile Leu Cys
            100                 105                 110

Leu Lys Gly Pro Ser Thr Glu Gly Ile Phe Arg Lys Ala Ala Ser Glu
        115                 120                 125

Lys Ala Arg Lys Glu Leu Lys Glu Gly Leu Asn Cys Gly Val Ser Leu
    130                 135                 140

Asn Leu Lys Gln Leu Pro Val His Leu Leu Ala Val Val Phe Lys Asp
145                 150                 155                 160

Phe Phe Arg Gly Ile Pro Leu Lys Leu Leu Ser Cys Asp Leu Phe Glu
                165                 170                 175

Asp Trp Met Gly Ala Leu Glu Lys Pro Thr Glu Glu Asp Arg Ile Glu
            180                 185                 190

Ala Leu Lys Gln Val Ala Gly Gly Leu Pro Arg Pro Asn Leu Leu Leu
        195                 200                 205

Leu Arg His Leu Leu Tyr Val Leu His Leu Ile Ser Lys Asn Ala Glu
    210                 215                 220

Val Asn Lys Met Asp Ser Ser Asn Leu Ala Ile Cys Ile Gly Pro Asn
225                 230                 235                 240

Met Leu Thr Leu Lys Asn Asp Gln Ser Leu Ser Phe Gln Ala Gln Lys
                245                 250                 255

Asp Leu Asn Asn Lys Val Lys Ile Leu Val Glu Phe Leu Ile Asp Asn
            260                 265                 270

Cys Phe Glu Ile Phe Gly Glu Asn Ile Arg Thr Arg Ser Arg Ile Thr
        275                 280                 285

Ser Asp Asp Ser Leu Glu His Thr Asp Ser Ser Asp Val Ser Thr Leu
    290                 295                 300

Gln Asn Asp Ser Ala Tyr Asp Ser Asn Asp Pro Asp Val Glu Pro Thr
305                 310                 315                 320

Ser Gly Thr Ala Ser Pro Asn Arg Gln Leu Glu Gly Pro Thr Pro Thr
                325                 330                 335

Met Ala Gly Leu Asp Thr Arg Gly His Arg Asp Thr Cys Glu Ser Ser
            340                 345                 350

Ser Glu Ser Ser Val Ser Met Val Val Arg Leu Lys Ser Ser Ile Val
        355                 360                 365

Gln Gln Asp Arg Arg Phe Ser Glu Pro Asn Met Ser Pro Ser Arg Glu
    370                 375                 380

Cys Leu Val Gly Pro Thr Ser Lys Gln Lys Leu Thr Arg Ser Glu Asp
385                 390                 395                 400

Ser Phe Thr Leu Ser Gln Asp Ala Ser Cys Ser Glu Gly Asp Glu Ala
                405                 410                 415

Glu Asp Pro Phe Thr Glu Glu Val Phe Pro Ala Val Asp Ser Lys Pro
            420                 425                 430

Lys Arg Pro Val Asp Leu Lys Ile Lys Asn
        435                 440

<210>  20
<211>  707
<212>  PRT
<213>  Tagapl t2 mouse

<400>  20

Met Lys Leu Ile Ser Ser Leu Asp Gly Ser Lys Thr Leu Asn Ala Asn
1               5                   10                  15

Asn Met Glu Thr Leu Ile Glu Cys Gln Ser Glu Gly Asp Ile Lys Val
            20                  25                  30

Pro Pro Leu Leu Thr Ser Cys Glu Ser Glu Asp Ser Ile Cys Gln Leu
        35                  40                  45

Ile Glu Ile Lys Lys Arg Lys Lys Val Leu Ser Trp Pro Ser Leu Met
    50                  55                  60

Arg Lys Leu Ser Pro Ser Ser Asp Phe Ser Gly Ser Leu Glu Pro Glu
65                  70                  75                  80

Leu Lys Val Ser Leu Phe Asp Gln Pro Leu Ser Ile Ile Cys Gly Glu
                85                  90                  95

Asn Asp Thr Leu Pro Arg Pro Ile Gln Asp Ile Leu Thr Ile Leu Cys
            100                 105                 110

Leu Lys Gly Pro Ser Thr Glu Gly Ile Phe Arg Lys Ala Ala Ser Glu
        115                 120                 125

Lys Ala Arg Lys Glu Leu Lys Glu Glu Leu Asn Cys Gly Val Ser Val
    130                 135                 140

Asn Leu Lys Gln Leu Pro Val His Leu Leu Ala Val Val Phe Lys Asp
145                 150                 155                 160

Phe Leu Arg Gly Ile Pro Leu Lys Leu Leu Ser Cys Asp Leu Phe Glu
                165                 170                 175

Asp Trp Met Gly Ala Leu Glu Lys Pro Thr Glu Ala Asp Arg Ile Glu
            180                 185                 190

Ala Leu Lys Gln Val Ala Gly Gly Leu Pro Arg Pro Asn Leu Phe Leu
        195                 200                 205

Leu Arg His Leu Leu Tyr Val Leu His Leu Ile Ser Lys Asn Ala Glu
    210                 215                 220

Val Asn Lys Met Asp Ser Ser Asn Leu Ala Ile Cys Ile Gly Pro Asn
225                 230                 235                 240

Met Leu Thr Leu Lys Asn Asp Gln Ser Leu Ser Phe Gln Ala Gln Lys
                245                 250                 255

Asp Leu Asn Asn Lys Val Lys Ile Leu Val Glu Phe Leu Ile Asp Asn
            260                 265                 270

Cys Phe Glu Ile Phe Gly Glu Asn Ile Arg Thr Arg Ser Arg Ile Thr
        275                 280                 285

Ser Asp Asp Ser Leu Glu His Thr Asp Ser Ser Asp Met Ser Thr Leu
    290                 295                 300

Gln Asn Asp Ser Ala Tyr Asp Ser Asn Asp Pro Asp Val Glu Pro Thr
305                 310                 315                 320

Ser Gly Thr Ala Ser Pro Asn Arg Gln Leu Glu Asp Pro Thr Pro Thr
                325                 330                 335

Met Ala Gly Leu Asp Thr Arg Gly His Arg Asp Thr Cys Glu Ser Ser
            340                 345                 350

Ser Glu Ser Ser Val Ser Met Val Val Arg Leu Lys Ser Ser Ile Val
        355                 360                 365

Gln Gln Asp Arg Arg Phe Ser Glu Pro Asn Met Ser Pro Ser Arg Glu
    370                 375                 380

Cys Leu Val Gly Pro Thr Ser Lys Gln Lys Leu Thr Arg Ser Glu Asp
385                 390                 395                 400

Ser Phe Thr Leu Ser Gln Asp Glu Ala Glu Asp Pro Phe Thr Glu Glu
                405                 410                 415

Val Phe Pro Ala Val Asp Ser Lys Pro Lys Arg Pro Val Asp Leu Lys
            420                 425                 430

Met Lys Asn Trp Thr Gln Gly Leu Ala Ser Pro Gln Gly His Ile Thr
        435                 440                 445

Lys Ala Phe Ser Arg Ser Ser Pro Gly Glu Ser Leu Gly Ser Ser Leu
    450                 455                 460

Val Pro Ser Pro Ser Cys Pro Lys Arg Asn Phe Phe Thr Arg His Gln
465                 470                 475                 480

Ser Phe Thr Thr Lys Thr Asp Lys Thr Lys Pro Gln Arg Glu Ile Arg
                485                 490                 495

Lys His Ser Met Ser Phe Ser Phe Ala Ser His Lys Lys Val Leu Pro
            500                 505                 510

Arg Thr Ser Ser Ile Gly Ser Glu Lys Ser Lys Asp Phe Ser Arg Asp
        515                 520                 525

Gln Leu Gln Lys Asp Leu Arg Lys Glu Ser Gln Leu Ser Gly Arg Ile
    530                 535                 540

Ile Gln Glu Asn Glu Ser Glu Ile Gln Ser Gln Thr Ser Leu Gly Phe
545                 550                 555                 560

Ser Leu Ser Gly Thr Trp Ala Leu Ser Val Asp Asn Thr Phe Gln Leu
                565                 570                 575

Val Asp Met Arg Lys Pro Gly Ser Pro Pro Ser Tyr Lys Glu Ala Ile
            580                 585                 590

Tyr Tyr Gln Thr Ser Gly Leu Arg Ala Tyr Ser Gly Gln Thr Val Gly
        595                 600                 605

Ser Met Arg Ser Arg Met Phe Lys Pro Ser Thr Ala Val Pro Pro Val
    610                 615                 620

Pro Ser His His Gly Gly Asp Leu Ser Glu Gly Thr Pro Gly Gly His
625                 630                 635                 640

Arg Leu Ser Ser Val Thr Glu His Trp Thr His Ser Gln Thr Val His
                645                 650                 655

Val Ser Ile Glu Thr Gln Gly Arg Ser Glu Leu His Gln Leu Arg Thr
            660                 665                 670

Val Ser Glu Ser Met Gln Lys Ala Lys Leu Asp Cys Leu Gly Pro Gln
        675                 680                 685

His Ser His Leu Val Phe Glu Val Asp Gln Leu Cys Cys Ala Arg Glu
    690                 695                 700

Ser Tyr Ile
705

<210>  21
<211>  707
<212>  PRT
<213>  Tagapl t3 mouse

<400>  21

Met Lys Leu Ile Ser Ser Leu Asp Gly Ser Lys Thr Leu Asn Ala Asn
1               5                   10                  15

Asn Met Glu Thr Leu Ile Glu Cys Gln Ser Glu Gly Asp Ile Lys Val
            20                  25                  30

Pro Pro Leu Leu Thr Ser Cys Glu Ser Glu Asp Ser Ile Cys Gln Leu
        35                  40                  45

Ile Glu Ile Lys Lys Gly Lys Lys Val Leu Ser Trp Pro Ser Leu Met
    50                  55                  60

Arg Lys Leu Ser Pro Ser Ser Asp Phe Ser Gly Ser Leu Glu Pro Glu
65                  70                  75                  80

Leu Lys Val Ser Leu Phe Asp Gln Pro Leu Ser Ile Ile Cys Gly Glu
                85                  90                  95

Asn Asp Thr Leu Pro Arg Pro Ile Gln Asp Ile Leu Thr Ile Leu Cys
            100                 105                 110

Leu Lys Gly Pro Ser Thr Glu Gly Ile Phe Arg Lys Ala Ala Ser Glu
        115                 120                 125

Lys Ala Arg Lys Glu Leu Lys Glu Glu Leu Asn Cys Gly Val Ser Val
    130                 135                 140

Asn Leu Lys Gln Leu Pro Val His Leu Leu Ala Val Val Phe Lys Asp
145                 150                 155                 160

Phe Leu Arg Gly Ile Pro Leu Lys Leu Leu Ser Cys Gly Leu Phe Glu
                165                 170                 175

Asp Trp Met Gly Ala Leu Glu Lys Pro Thr Glu Glu Asp Arg Ile Glu
            180                 185                 190

Ala Leu Lys Gln Val Ala Gly Gly Leu Pro Arg Pro Asn Leu Phe Leu
        195                 200                 205

Leu Arg His Leu Leu Tyr Val Leu His Leu Ile Ser Lys Asn Ala Glu
    210                 215                 220

Val Asn Lys Met Asp Ser Ser Asn Leu Ala Ile Cys Ile Gly Pro Asn
225                 230                 235                 240

Met Leu Thr Leu Lys Asn Asp Gln Ser Leu Ser Phe Gln Ala Gln Lys
                245                 250                 255

Asp Leu Asn Asn Lys Val Lys Ile Leu Val Glu Phe Leu Ile Asp Asn
            260                 265                 270

Cys Phe Glu Ile Phe Gly Glu Asn Ile Arg Thr Arg Ser Arg Ile Thr
        275                 280                 285

Ser Asp Asp Ser Leu Glu His Thr Asp Ser Ser Asp Met Ser Thr Leu
    290                 295                 300

Gln Asn Asp Ser Ala Tyr Asp Ser Asn Asp Pro Asp Val Glu Pro Thr
305                 310                 315                 320

Ser Gly Thr Ala Ser Pro Asn Arg Gln Leu Glu Asp Pro Thr Pro Thr
                325                 330                 335

Met Ala Gly Leu Asp Thr Arg Gly His Arg Asp Thr Cys Glu Ser Ser
            340                 345                 350

Ser Glu Ser Ser Val Ser Met Val Val Arg Leu Lys Ser Ser Ile Val
        355                 360                 365

Gln Gln Asp Arg Arg Phe Ser Glu Pro Asn Met Ser Pro Ser Arg Glu
    370                 375                 380

Cys Leu Val Gly Pro Thr Ser Lys Gln Lys Leu Thr Arg Ser Glu Asp
385                 390                 395                 400

Ser Phe Thr Leu Ser Gln Asp Glu Ala Glu Asp Pro Phe Thr Glu Glu
                405                 410                 415

Val Phe Pro Ala Val Asp Ser Lys Pro Lys Arg Pro Val Asp Leu Lys
            420                 425                 430

Met Lys Asn Trp Thr Gln Gly Leu Ala Ser Pro Gln Gly His Ile Thr
        435                 440                 445

Lys Ala Phe Ser Arg Ser Ser Pro Gly Glu Ser Leu Gly Ser Ser Leu
    450                 455                 460

Val Pro Ser Pro Ser Cys Pro Lys Arg Asn Phe Phe Thr Arg His Gln
465                 470                 475                 480

Ser Phe Thr Thr Lys Thr Asp Lys Thr Lys Pro Gln Arg Glu Ile Arg
                485                 490                 495

Lys His Ser Met Ser Phe Ser Phe Ala Ser His Lys Lys Val Leu Pro
            500                 505                 510

Arg Thr Ser Ser Ile Gly Ser Glu Lys Ser Lys Asp Phe Ser Arg Asp
        515                 520                 525

Gln Leu Gln Lys Asp Leu Arg Lys Glu Ser Gln Leu Ser Gly Arg Ile
    530                 535                 540

Ile Gln Glu Asn Glu Ser Glu Ile Gln Ser Gln Thr Ser Leu Gly Phe
545                 550                 555                 560

Ser Leu Ser Gly Thr Trp Ala Leu Ser Val Asp Asn Thr Phe Gln Leu
                565                 570                 575

Val Asp Met Arg Lys Pro Gly Ser Pro Pro Ser Tyr Lys Glu Ala Ile
            580                 585                 590

Tyr Tyr Gln Thr Ser Gly Leu Arg Ala Tyr Ser Gly Gln Thr Val Gly
        595                 600                 605

Ser Met Arg Ser Arg Met Phe Lys Pro Ser Thr Ala Val Pro Pro Val
    610                 615                 620

Pro Ser His His Gly Gly Asp Leu Ser Glu Gly Thr Pro Gly Gly His
625                 630                 635                 640

Arg Leu Ser Ser Val Thr Glu His Trp Thr His Ser Gln Thr Val His
                645                 650                 655

Val Ser Ile Glu Thr Gln Gly Arg Ser Glu Leu His Gln Leu Arg Thr
            660                 665                 670

Val Ser Glu Ser Met Gln Lys Ala Lys Leu Asp Cys Leu Gly Pro Gln
        675                 680                 685

His Ser His Leu Val Phe Glu Val Asp Gln Leu Cys Cys Ala Arg Glu
    690                 695                 700

Ser Tyr Ile
705

<210> 22
<211> 707
<212> PRT
<213> Tagap1 t4 mouse

<400> 22

Met Lys Leu Ile Ser Ser Leu Asp Gly Ser Lys Thr Leu Asn Ala Asn
1               5                   10                  15

Asn Met Glu Thr Leu Ile Glu Cys Gln Ser Glu Gly Asp Ile Lys Val
            20                  25                  30

Pro Pro Leu Leu Thr Ser Cys Glu Ser Glu Asp Ser Ile Cys Gln Leu
        35                  40                  45

Ile Glu Ile Lys Lys Arg Lys Lys Val Leu Ser Trp Pro Ser Leu Met
    50                  55                  60

Arg Lys Leu Ser Pro Ser Ser Asp Phe Ser Gly Ser Leu Glu Pro Glu
65                  70                  75                  80

Leu Lys Val Ser Leu Phe Asp Gln Pro Leu Ser Ile Ile Cys Gly Glu

                85                  90                  95

Asn Asp Thr Leu Pro Arg Pro Ile Gln Asp Ile Leu Thr Ile Leu Cys
            100                 105                 110

Leu Lys Gly Pro Ser Thr Glu Gly Ile Phe Arg Lys Ala Ala Ser Glu
        115                 120                 125

Lys Ala Arg Lys Glu Leu Lys Glu Glu Leu Asn Cys Gly Val Ser Val
    130                 135                 140

Asn Leu Lys Gln Leu Pro Val His Leu Leu Ala Val Val Phe Lys Asp
145                 150                 155                 160

Phe Leu Arg Gly Ile Pro Leu Lys Leu Leu Ser Cys Asp Leu Phe Glu
                165                 170                 175

Asp Trp Met Gly Ala Leu Glu Lys Pro Thr Glu Glu Asp Arg Ile Glu
            180                 185                 190

Ala Leu Lys Gln Val Ala Gly Gly Leu Pro Arg Pro Asn Leu Phe Leu
        195                 200                 205

Leu Arg His Leu Leu Tyr Val Leu His Leu Ile Ser Lys Asn Ala Glu
    210                 215                 220

Val Asn Lys Met Asp Ser Ser Asn Leu Ala Ile Cys Ile Gly Pro Asn
225                 230                 235                 240

Met Leu Thr Leu Lys Asn Asp Gln Ser Leu Ser Phe Gln Ala Gln Lys
                245                 250                 255

Asp Leu Asn Asn Lys Val Lys Ile Leu Val Glu Phe Leu Ile Asp Asn
            260                 265                 270

Cys Phe Glu Ile Phe Gly Glu Asn Ile Arg Thr Arg Ser Arg Ile Thr
        275                 280                 285

Ser Asp Asp Ser Leu Glu His Thr Asp Ser Ser Asp Met Ser Thr Leu
    290                 295                 300

Gln Asn Asp Ser Ala Tyr Asp Ser Asn Asp Pro Asp Val Glu Pro Thr
305                 310                 315                 320

Ser Gly Thr Ala Ser Pro Asn Arg Gln Leu Glu Asp Pro Thr Pro Thr
                325                 330                 335

Met Ala Gly Leu Asp Thr Arg Gly His Arg Asp Thr Cys Glu Ser Ser

340                 345                 350

Ser Glu Ser Ser Val Ser Met Val Val Arg Leu Lys Ser Ser Ile Val
        355                 360                 365

Gln Gln Asp Arg Arg Phe Ser Glu Pro Asn Met Ser Pro Ser Arg Glu
    370                 375                 380

Cys Leu Val Gly Pro Thr Ser Lys Gln Lys Leu Thr Arg Ser Glu Asp
385                 390                 395                 400

Ser Phe Thr Leu Ser Gln Asp Glu Ala Glu Asp Pro Phe Thr Glu Glu
                405                 410                 415

Val Phe Pro Ala Val Asp Ser Lys Pro Lys Arg Pro Val Asp Leu Lys
            420                 425                 430

Met Lys Asn Trp Thr Gln Gly Leu Ala Ser Pro Gln Gly His Ile Thr
        435                 440                 445

Lys Ala Phe Ser Arg Ser Ser Pro Gly Glu Ser Leu Gly Ser Ser Leu
    450                 455                 460

Val Pro Ser Pro Ser Cys Pro Lys Arg Asn Phe Phe Thr Arg His Gln
465                 470                 475                 480

Ser Phe Thr Thr Lys Thr Asp Lys Thr Lys Pro Gln Arg Glu Ile Arg
                485                 490                 495

Lys His Ser Met Ser Phe Ser Phe Ala Ser His Lys Lys Val Leu Pro
            500                 505                 510

Arg Thr Ser Ser Ile Gly Ser Glu Lys Ser Glu Asp Phe Ser Arg Asp
        515                 520                 525

Gln Leu Gln Lys Asp Leu Arg Lys Glu Ser Gln Leu Ser Gly Arg Ile
    530                 535                 540

Ile Gln Glu Asn Glu Ser Glu Ile Gln Ser Gln Thr Ser Leu Gly Phe
545                 550                 555                 560

Ser Leu Ser Gly Thr Arg Ala Leu Ser Val Asp Asn Thr Phe Gln Leu
                565                 570                 575

Val Asp Met Arg Lys Pro Gly Ser Pro Pro Ser Tyr Lys Glu Ala Ile
            580                 585                 590

Tyr Tyr Gln Thr Ser Gly Leu Arg Ala Tyr Ser Gly Gln Thr Val Gly

595                 600                 605

Ser Met Arg Ser Arg Met Phe Lys Pro Ser Thr Ala Val Pro Pro Val
    610                 615                 620

Pro Ser His His Gly Gly Asp Leu Ser Glu Gly Thr Pro Gly Gly His
625                 630                 635                 640

Arg Leu Ser Ser Val Thr Glu His Trp Thr His Ser Gln Thr Val His
                645                 650                 655

Val Ser Ile Glu Thr Gln Gly Arg Ser Glu Leu His Gln Leu Arg Thr
            660                 665                 670

Val Ser Glu Ser Met Gln Lys Ala Lys Leu Asp Cys Leu Gly Pro Gln
        675                 680                 685

His Ser His Leu Val Phe Glu Val Asp Gln Leu Cys Cys Ala Arg Glu
    690                 695                 700

Ser Tyr Ile
705

<210>  23
<211>  731
<212>  PRT
<213>  Tagap homo sapiens

<400>  23

Met Lys Leu Arg Ser Ser His Asn Ala Ser Lys Thr Leu Asn Ala Asn
1               5                   10                  15

Asn Met Glu Thr Leu Ile Glu Cys Gln Ser Glu Gly Asp Ile Lys Glu
            20                  25                  30

His Pro Leu Leu Ala Ser Cys Glu Ser Glu Asp Ser Ile Cys Gln Leu
        35                  40                  45

Ile Glu Val Lys Lys Arg Lys Lys Val Leu Ser Trp Pro Phe Leu Met
    50                  55                  60

Arg Arg Leu Ser Pro Ala Ser Asp Phe Ser Gly Ala Leu Glu Thr Asp
65                  70                  75                  80

Leu Lys Ala Ser Leu Phe Asp Gln Pro Leu Ser Ile Ile Cys Gly Asp
                85                  90                  95

Ser Asp Thr Leu Pro Arg Pro Ile Gln Asp Ile Leu Thr Ile Leu Cys
            100                 105                 110

Leu Lys Gly Pro Ser Thr Glu Gly Ile Phe Arg Arg Ala Ala Asn Glu
        115                 120                 125

Lys Ala Arg Lys Glu Leu Lys Glu Glu Leu Asn Ser Gly Asp Ala Val
    130                 135                 140

Asp Leu Glu Arg Leu Pro Val His Leu Leu Ala Val Val Phe Lys Asp
145                 150                 155                 160

Phe Leu Arg Ser Ile Pro Arg Lys Leu Leu Ser Ser Asp Leu Phe Glu
                165                 170                 175

Glu Trp Met Gly Ala Leu Glu Met Gln Asp Glu Glu Asp Arg Ile Glu
            180                 185                 190

Ala Leu Lys Gln Val Ala Asp Lys Leu Pro Arg Pro Asn Leu Leu Leu
        195                 200                 205

Leu Lys His Leu Val Tyr Val Leu His Leu Ile Ser Lys Asn Ser Glu
    210                 215                 220

Val Asn Arg Met Asp Ser Ser Asn Leu Ala Ile Cys Ile Gly Pro Asn
225                 230                 235                 240

Met Leu Thr Leu Glu Asn Asp Gln Ser Leu Ser Phe Glu Ala Gln Lys
                245                 250                 255

Asp Leu Asn Asn Lys Val Lys Thr Leu Val Glu Phe Leu Ile Asp Asn
            260                 265                 270

Cys Phe Glu Ile Phe Gly Glu Asn Ile Pro Val His Ser Ser Ile Thr
        275                 280                 285

Ser Asp Asp Ser Leu Glu His Thr Asp Ser Ser Asp Val Ser Thr Leu
    290                 295                 300

Gln Asn Asp Ser Ala Tyr Asp Ser Asn Asp Pro Asp Val Glu Ser Asn
305                 310                 315                 320

Ser Ser Ser Gly Ile Ser Ser Pro Ser Arg Gln Pro Gln Val Pro Met
                325                 330                 335

Ala Thr Ala Ala Gly Leu Asp Ser Ala Gly Pro Gln Asp Ala Arg Glu
            340                 345                 350

Val Ser Pro Glu Pro Ile Val Ser Thr Val Ala Arg Leu Lys Ser Ser
        355                 360                 365

Leu Ala Gln Pro Asp Arg Arg Tyr Ser Glu Pro Ser Met Pro Ser Ser
    370                 375                 380

Gln Glu Cys Leu Glu Ser Arg Val Thr Asn Gln Thr Leu Thr Lys Ser
385                 390                 395                 400

Glu Gly Asp Phe Pro Val Pro Arg Val Gly Ser Arg Leu Glu Ser Glu
                405                 410                 415

Glu Ala Glu Asp Pro Phe Pro Glu Glu Val Phe Pro Ala Val Gln Gly
            420                 425                 430

Lys Thr Lys Arg Pro Val Asp Leu Lys Ile Lys Asn Leu Ala Pro Gly
        435                 440                 445

Ser Val Leu Pro Arg Ala Leu Val Leu Lys Ala Phe Ser Ser Ser Ser
    450                 455                 460

Leu Asp Ala Ser Ser Asp Ser Ser Pro Val Ala Ser Pro Ser Ser Pro
465                 470                 475                 480

Lys Arg Asn Phe Phe Ser Arg His Gln Ser Phe Thr Thr Lys Thr Glu
                485                 490                 495

Lys Gly Lys Pro Ser Arg Glu Ile Lys Lys His Ser Met Ser Phe Thr
            500                 505                 510

Phe Ala Pro His Lys Lys Val Leu Thr Lys Asn Leu Ser Ala Gly Ser
        515                 520                 525

Gly Lys Ser Gln Asp Phe Thr Arg Asp His Val Pro Arg Gly Val Arg
    530                 535                 540

Lys Glu Ser Gln Leu Ala Gly Arg Ile Val Gln Glu Asn Gly Cys Glu
545                 550                 555                 560

Thr His Asn Gln Thr Ala Arg Gly Phe Cys Leu Arg Pro His Ala Leu
                565                 570                 575

Ser Val Asp Asp Val Phe Gln Gly Ala Asp Trp Glu Arg Pro Gly Ser
            580                 585                 590

Pro Pro Ser Tyr Glu Glu Ala Met Gln Gly Pro Ala Ala Arg Leu Val
        595                 600                 605

Ala Ser Glu Ser Gln Thr Val Gly Ser Met Thr Val Gly Ser Met Arg
    610                 615                 620

Ala Arg Met Leu Glu Ala His Cys Leu Leu Pro Pro Leu Pro Pro Ala
625                 630                 635                 640

His His Val Glu Asp Ser Arg His Arg Gly Ser Lys Glu Pro Leu Pro
                645                 650                 655

Gly His Gly Leu Ser Pro Leu Pro Glu Arg Trp Lys Gln Ser Arg Thr
            660                 665                 670

Val His Ala Ser Gly Asp Ser Leu Gly His Val Ser Gly Pro Gly Arg
        675                 680                 685

Pro Glu Leu Leu Pro Leu Arg Thr Val Ser Glu Ser Val Gln Arg Asn
    690                 695                 700

Lys Arg Asp Cys Leu Val Arg Arg Cys Ser Gln Pro Val Phe Glu Ala
705                 710                 715                 720

Asp Gln Phe Gln Tyr Ala Lys Glu Ser Tyr Ile
                725                 730

<210>  24
<211>  1008
<212>  PRT
<213>  Tagap Rattus

<400>  24

Met Glu Leu Leu Ser Ala Arg Leu Pro Arg Ala Gly Glu Thr Phe Val
1               5                   10                  15

Pro Ile Ser Glu His Gln His Trp Lys Pro Glu Asn Ala His Asn Asn
            20                  25                  30

Gly Ala Gln Cys Phe Pro Glu Leu Thr Leu Gly Arg Thr His Gln Gly
        35                  40                  45

Asn Lys Val Leu Asp Ser Glu Trp Val Thr Gln Met Leu Arg Thr Thr
    50                  55                  60

Leu Ala Phe Arg Arg Ala Leu Tyr Ile Lys Leu Thr Ala Ser Ile Leu
65                  70                  75                  80

Glu Leu Gln Asn Arg Lys Asn Gly Thr Phe His Val Leu Arg Gly Glu
                85                  90                  95

Asn Tyr Lys Phe Ser Ser Val Glu His Ser Ser Leu Ala Gly Ile Gln
            100                 105                 110

Gln Val Phe Ser Lys His Ser Gln Ser Val Gln Asp Cys Ser Asp Val
        115                 120                 125

Glu Lys Val Val Ala Ile Val Val Ala Glu Lys Pro Gly Ile Ser Phe
    130                 135                 140

Lys Asn Pro Pro Pro Ala Leu Met Ala Gly Pro Arg Asp Leu Ala Ala
145                 150                 155                 160

Ala Glu Leu Glu Arg Gly Glu Ala Lys Arg Pro Pro Glu Glu Ile Gln
                165                 170                 175

Cys Asp Gly Val Ser Gln Ile Thr Ala Pro Leu Glu Trp Arg Glu Ser
            180                 185                 190

Arg Phe Tyr Glu Asn Ser Val Ser Asp Lys Thr Asp Glu Phe Leu Cys
        195                 200                 205

Pro Gln Ile Arg Tyr Lys Ser Ser Thr Arg Gly Arg Cys Arg Arg His
    210                 215                 220

Ala Gly Gly Arg Tyr Met Arg Leu Ala Arg Pro Leu Pro Leu Asp Asn
225                 230                 235                 240

Asn His Leu Glu Thr His Arg Val Arg Thr Val Leu Val Ser Ile Leu
                245                 250                 255

Ser Arg Pro Leu Ser His Pro Val Arg Asp Ala Ala Ser Leu Gly Leu
            260                 265                 270

Thr Gln Thr Gln Asn Gln Ser Leu Glu Val Glu Lys Ile Pro Leu Gly
        275                 280                 285

Leu Ser Ala Pro Asn Pro Met Lys Leu Ile Ser Ser Leu Asp Gly Ala
    290                 295                 300

Lys Thr Leu Asn Ala Asn Asn Met Asp Thr Leu Ile Glu Cys Gln Ser
305                 310                 315                 320

Glu Gly Asp Ile Lys Val Leu Pro Leu Leu Thr Ser Cys Glu Ser Glu
                325                 330                 335

Asp Ser Ile Cys Gln Leu Ile Glu Val Lys Lys Arg Lys Lys Val Leu
            340                 345                 350

Ser Trp Pro Ser Leu Met Arg Lys Leu Ser Pro Ser Pro Asp Phe Ser
        355                 360                 365

Gly Ser Leu Glu Pro Glu Leu Lys Val Ser Leu Phe Asp Gln Pro Leu
    370                 375                 380

Ser Ile Ile Cys Lys Glu Asn Asp Thr Leu Pro Arg Pro Ile Gln Asp
385                 390                 395                 400

Ile Leu Thr Ile Leu Cys Leu Lys Gly Pro Ser Thr Glu Gly Ile Phe
                405                 410                 415

Arg Lys Ala Ala Ser Glu Lys Ala Arg Lys Glu Leu Lys Glu Glu Leu
            420                 425                 430

Asn Cys Gly Gly Ser Val Asn Leu Asn Gln Leu Pro Val His Leu Leu
        435                 440                 445

Ala Val Val Phe Lys Asp Phe Leu Arg Gly Ile Pro Leu Lys Leu Leu
    450                 455                 460

Ser Cys Asp Leu Phe Glu Asp Trp Met Gly Ala Leu Glu Lys Pro Ser
465                 470                 475                 480

Glu Glu Asp Arg Ile Glu Ala Leu Lys Gln Val Ala Asp Arg Leu Pro
                485                 490                 495

Arg Pro Asn Leu Leu Leu Leu Arg Thr Leu Val Tyr Val Leu His Leu
            500                 505                 510

Ile Ser Lys Asn Ala Glu Val Asn Lys Met Asp Ser Ser Asn Leu Ala
        515                 520                 525

Ile Cys Ile Gly Pro Asn Met Leu Thr Leu Lys Asn Asp Gln Ser Leu
    530                 535                 540

Ser Phe Gln Ala Gln Arg Asp Leu Asn Asn Lys Val Lys Ile Leu Val
545                 550                 555                 560

Glu Phe Leu Ile Asp Asn Cys Lcu Glu Ilc Phe Gly Glu Asn Ile Pro
                565                 570                 575

Thr His Pro Arg Ile Thr Ser Asp Asp Ser Leu Glu His Thr Asp Ser
            580                 585                 590

Ser Asp Val Ser Thr Leu Gln Asn Asp Ser Ala Tyr Asp Ser Asn Asp
        595                 600                 605

Pro Asp Val Glu Pro Ala Asn Gly Val Ala Ser Pro Cys Arg Gln Leu
    610                 615                 620

Glu Gly Pro Ser Thr Thr Val Ala Gly Met Asp Thr Arg Gly Pro Arg
625                 630                 635                 640

Asn Thr Cys Glu Ser Ser Ser Glu Ser Ser Val Ser Met Val Ala Arg
                645                 650                 655

Leu Lys Ser Ser Ile Gly Gln Gln Asp Arg Arg Phe Ser Glu Pro Asn
            660                 665                 670

Met Pro Pro Ser Arg Glu Cys Phe Val Gly Pro Ile Thr Lys Gln Lys
        675                 680                 685

Leu Thr Arg Ser Glu Asp Ser Phe Val Leu Pro Gln Glu Ala Ser Cys
    690                 695                 700

Ser Glu Gly Asn Glu Ala Glu Asp Pro Phe Thr Glu Glu Val Phe Pro
705                 710                 715                 720

Ala Val Glu Gly Lys Pro Met Arg Pro Val Asp Leu Lys Ile Lys Asn
                725                 730                 735

Leu Thr Gln Gly Leu Ala Ser Pro Gln Gly Pro Ala Asn Lys Ala Phe
            740                 745                 750

Ser Ser Phe Ser Ala Gly Glu Ser Leu Asp Ser Ser Pro Val Pro Ser
        755                 760                 765

Pro Ser Cys Pro Lys Arg Asn Phe Phe Thr Arg His Gln Ser Phe Thr
    770                 775                 780

Thr Lys Thr Asp Lys Thr Lys Pro Gln Arg Glu Ile Arg Lys His Ser
785                 790                 795                 800

Met Ser Phe Ser Phe Ala Ser His Lys Lys Val Leu Pro Arg Thr Ser
                805                 810                 815

Ser Ile Gly Ser Glu Lys Ser Lys Asp Phe Ser Arg Asp Gln Leu Gln
            820                 825                 830

Lys Asp Leu Arg Lys Glu Ser Gln Leu Ala Gly Arg Ile Ile Arg Glu
        835                 840                 845

Asn Glu Ser Glu Ile Gln Ser Gln Thr Asn Leu Gly Ser Ser Leu Ser
    850                 855                 860

Gly Thr Trp Ala Leu Ser Val Asp Asn Thr Phe Gln Phe Ile Asp Val
865                 870                 875                 880

Arg Lys Pro Gly Ser Pro Pro Ser Tyr Glu Glu Ala Ile Tyr Tyr His
                885                 890                 895

Thr Ser Gly Leu Thr Ala Tyr Ser Gly Gln Thr Val Gly Ser Met Arg
            900                 905                 910

Ala Arg Met Leu Lys Gln Ser Met Ala Val Pro Pro Val Pro Ser His
        915                 920                 925

His Glu Gly Glu Leu Ser Glu Gly Ile Pro Gly Gly His Arg Ser Ser
    930                 935                 940

Ser Val Thr Glu His Trp Thr Gln Ser Gln Thr Val His Val Ser Val
945                 950                 955                 960

Glu Thr Arg Gly Arg Ser Glu Leu His Arg Leu Arg Thr Val Ser Glu
                965                 970                 975

Ser Val Gln Arg Ala Lys Leu Asp Tyr Leu Gly Gln Gln Arg Ser His
            980                 985                 990

Leu Val Phe Glu Val Asp Gln Leu  Arg Cys Ala Lys Glu  Ser Tyr Ile
        995                 1000                1005

<210>  25
<211>  727
<212>  PRT
<213>  FGD2 mouse wildtype

<400>  25

Met Glu Arg Ala Cys Glu Lys Gln Asp Ser Val Cys Asn Leu Val Ala
1               5                   10                  15

Val Phe Glu Asn Asn Arg Thr Pro Gly Glu Ala Pro Gly Ser His Ser
            20                  25                  30

Leu Glu Asp Gln Pro His Ile Pro Glu His Gln Leu Ser Leu Ser Pro
        35                  40                  45

Glu Pro Trp Glu Ala Pro Pro Val Lys Glu Ala Leu Lys Ser Glu Phe
    50                  55                  60

Arg Pro Val Ser Arg Thr Tyr Leu Ser Ser Leu Lys Asn Lys Leu Ser
65                  70                  75                  80

Ser Gly Ala Trp Arg Arg Ser Cys Gln Pro Gly Val Ser Pro Gly Pro
                85                  90                  95

Glu Thr Gln Glu Pro Glu Glu Lys Arg Val Val Arg Glu Leu Leu Glu
            100                 105                 110

Thr Glu Gln Ala Tyr Val Ala Arg Leu His Leu Leu Asp Gln Val Phe
        115                 120                 125

Phe Gln Glu Leu Leu Arg Glu Ala Gly Arg Ser Lys Ala Phe Pro Glu
    130                 135                 140

Asp Val Val Lys Leu Ile Phe Ser Asn Ile Ser Ser Ile Tyr Arg Phe
145                 150                 155                 160

His Ala Gln Phe Phe Leu Pro Glu Leu Gln Arg Arg Val Asp Asp Trp
                165                 170                 175

Ala Ala Thr Pro Arg Ile Gly Asp Val Ile Gln Lys Leu Ala Pro Phe
            180                 185                 190

Leu Lys Met Tyr Ser Glu Tyr Val Lys Asn Phe Glu Arg Ala Ala Glu
        195                 200                 205

Leu Leu Ala Thr Trp Met Asp Lys Ser Gln Pro Phe Gln Glu Val Val
    210                 215                 220

Thr Arg Ile Gln Cys Ser Glu Ala Ser Ser Ser Leu Thr Leu Gln His
225                 230                 235                 240

His Met Leu Glu Pro Val Gln Arg Ile Pro Arg Tyr Glu Leu Leu Leu
                245                 250                 255

Lys Glu Tyr Val Gln Lys Leu Pro Ala Gln Ala Pro Asp Leu Glu Asp
            260                 265                 270

Ala Gln Arg Ala Leu Asp Met Ile Phe Ser Ala Ala Gln His Ser Asn
        275                 280                 285

Ala Ala Ile Ala Glu Met Glu Arg Leu Gln Gly Leu Trp Asp Val Tyr
    290                 295                 300

Gln Arg Leu Gly Leu Glu Asp Asp Ile Val Asp Pro Ser Asn Thr Leu
305                 310                 315                 320

Leu Arg Glu Gly Pro Val Leu Lys Ile Ser Phe Arg Arg Ser Asp Pro
                325                 330                 335

Met Glu Arg Tyr Leu Val Leu Phe Asn Asn Met Leu Leu Tyr Cys Val
            340                 345                 350

Pro Arg Val Leu Gln Val Gly Ala Gln Phe Gln Val Arg Thr Arg Ile
        355                 360                 365

Asp Val Ala Gly Met Lys Val Arg Glu Leu Thr Asp Ala Glu Phe Pro
    370                 375                 380

His Ser Phe Leu Val Ser Gly Lys Gln Arg Thr Leu Glu Leu Gln Ala
385                 390                 395                 400

Arg Ser Arg Asp Glu Met Val Ser Trp Met Gln Ala Cys Gln Ala Ala
                405                 410                 415

Ile Asp Gln Val Glu Lys Arg Ser Glu Thr Phe Lys Ala Ala Val Gln
            420                 425                 430

Gly Pro Gln Gly Asp Thr Gln Glu Pro Lys Pro Gln Val Glu Glu Leu
        435                 440                 445

Gly Leu Arg Ala Pro Gln Trp Val Arg Asp Lys Met Val Thr Met Cys
    450                 455                 460

Met Arg Cys Gln Glu Pro Phe Asn Ala Leu Thr Arg Arg Arg His His
465                 470                 475                 480

Cys Arg Ala Cys Gly Tyr Val Val Cys Ala Lys Cys Ser Asp Tyr Arg
                485                 490                 495

Ala Glu Leu Lys Tyr Asp Ser Asn Arg Pro Asn Arg Val Cys Leu Thr
            500                 505                 510

Cys Tyr Thr Phe Leu Thr Gly Asn Val Leu Pro Gln Gly Lys Glu Asp
        515                 520                 525

Lys Arg Arg Gly Ile Leu Glu Lys Glu Ala Ser Ala Ala Pro Glu Gln
    530                 535                 540

Ser Leu Val Cys Ser Phe Leu Gln Leu Ile Gly Asp Lys Cys Ser Arg
545                 550                 555                 560

Ser Leu Pro Arg Ser Trp Cys Val Ile Pro Arg Asp Asp Pro Leu Val
                565                 570                 575

Leu Tyr Val Tyr Ala Ala Pro Gln Asp Thr Lys Ala His Thr Ser Ile
            580                 585                 590

Pro Leu Leu Gly Tyr Gln Val Ile Ser Gly Pro Gln Gly Thr Ser Gly
        595                 600                 605

Phe Pro Ala Ala Thr Val Arg Arg Ala Val His Leu Gln Gly Arg Val
    610                 615                 620

Cys Gly Ala Ala Gly Pro Leu Gly Asp Ser Tyr Gln Ala Cys Ala Ser
625                 630                 635                 640

Gly Thr Pro Glu Asp Leu Thr Lys Lys Met Cys Leu Thr Glu Pro Ala
                645                 650                 655

Ala Ser Cys Ser Ser Arg Val His Asp Ser Leu Pro Arg Pro Thr Pro
            660                 665                 670

Trp Phe Tyr His Phe Thr Pro Ser Trp Ala Thr Pro Asp Pro Pro Asp
        675                 680                 685

Val Tyr Thr Glu Thr Ile His Pro Asp Ser Val Ser Ser Arg His Arg
    690                 695                 700

Pro Phe Pro Ser Gly Arg Tyr His Gln Val Ser Gln Leu Ala Gly Glu
705                 710                 715                 720

Gly Ala Asp Ile Pro Gly Ser
                725

<210>  26
<211>  655
<212>  PRT
<213>  FGD2 mouse wildtype isoform 1

<400>  26

Met Glu Arg Ala Cys Glu Lys Gln Asp Ser Val Cys Asn Leu Val Ala
1               5                   10                  15

Val Phe Glu Asn Asn Arg Thr Pro Gly Glu Ala Pro Gly Ser His Ser
            20                  25                  30

Leu Glu Asp Gln Pro His Ser Pro Glu His Gln Leu Ser Leu Ser Pro
        35                  40                  45

Glu Pro Trp Glu Ala Pro Pro Val Lys Glu Ala Leu Lys Ser Glu Phe
    50                  55                  60

Arg Pro Val Ser Arg Thr Tyr Leu Ser Ser Leu Lys Asn Lys Leu Ser
65                  70                  75                  80

Ser Gly Ala Trp Arg Arg Ser Cys Gln Pro Gly Val Ser Pro Gly Pro
                85                  90                  95

Glu Thr Gln Glu Pro Glu Glu Lys Arg Val Val Arg Glu Leu Leu Glu

            100                 105                 110

Thr Glu Gln Ala Tyr Val Ala Arg Leu His Leu Leu Asp Gln Val Phe
        115                 120                 125

Phe Gln Glu Leu Leu Arg Glu Ala Gly Arg Ser Lys Ala Phe Pro Glu
    130                 135                 140

Asp Val Val Lys Leu Ile Phe Ser Asn Ile Ser Ser Ile Tyr Arg Phe
145                 150                 155                 160

His Ala Gln Phe Phe Leu Pro Glu Leu Gln Arg Arg Val Asp Asp Trp
                165                 170                 175

Ala Ala Thr Pro Arg Ile Gly Asp Val Ile Gln Lys Leu Ala Pro Phe
            180                 185                 190

Leu Lys Met Tyr Ser Glu Tyr Val Lys Asn Phe Glu Arg Ala Ala Glu
        195                 200                 205

Leu Leu Ala Thr Trp Met Asp Lys Ser Gln Pro Phe Gln Glu Val Val
    210                 215                 220

Thr Arg Ile Gln Cys Ser Glu Ala Ser Ser Ser Leu Thr Leu Gln His
225                 230                 235                 240

His Met Leu Glu Pro Val Gln Arg Ile Pro Arg Tyr Glu Leu Leu Leu
                245                 250                 255

Lys Glu Tyr Val Gln Lys Leu Pro Ala Gln Ala Pro Asp Leu Glu Asp
            260                 265                 270

Ala Gln Arg Ala Leu Asp Met Ile Phe Ser Ala Ala Gln His Ser Asn
        275                 280                 285

Ala Ala Ile Ala Glu Met Glu Arg Leu Gln Gly Leu Trp Asp Val Tyr
    290                 295                 300

Gln Arg Leu Gly Leu Glu Asp Asp Ile Val Asp Pro Ser Asn Thr Leu
305                 310                 315                 320

Leu Arg Glu Gly Pro Val Leu Lys Ile Ser Phe Arg Arg Ser Asp Pro
                325                 330                 335

Met Glu Arg Tyr Leu Val Leu Phe Asn Asn Met Leu Leu Tyr Cys Val
            340                 345                 350

Pro Arg Val Leu Gln Val Gly Ala Gln Phe Gln Val Arg Thr Arg Ile

355                 360                 365

Asp Val Ala Gly Met Lys Val Arg Glu Leu Thr Asp Ala Glu Phe Pro
    370                 375                 380

His Ser Phe Leu Val Ser Gly Lys Gln Arg Thr Leu Glu Leu Gln Ala
385                 390                 395                 400

Arg Ser Arg Asp Glu Met Val Ser Trp Met Gln Ala Cys Gln Ala Ala
                405                 410                 415

Ile Asp Gln Val Glu Lys Arg Ser Glu Thr Phe Lys Ala Ala Val Gln
            420                 425                 430

Gly Pro Gln Gly Asp Thr Gln Glu Pro Lys Pro Gln Val Glu Glu Leu
        435                 440                 445

Gly Leu Arg Ala Pro Gln Trp Val Arg Asp Lys Met Val Thr Met Cys
    450                 455                 460

Met Arg Cys Gln Glu Pro Phe Asn Ala Leu Thr Arg Arg Arg His His
465                 470                 475                 480

Cys Arg Ala Cys Gly Tyr Val Val Cys Ala Lys Cys Ser Asp Tyr Arg
                485                 490                 495

Ala Glu Leu Lys Tyr Asp Ser Asn Arg Pro Asn Arg Val Cys Leu Thr
            500                 505                 510

Cys Tyr Thr Phe Leu Thr Gly Asn Val Leu Pro Gln Gly Lys Glu Asp
        515                 520                 525

Lys Arg Arg Gly Ile Leu Glu Lys Glu Ala Ser Ala Ala Pro Glu Gln
    530                 535                 540

Ser Leu Val Cys Ser Phe Leu Gln Leu Ile Gly Asp Lys Cys Ser Arg
545                 550                 555                 560

Ser Leu Pro Arg Ser Trp Cys Val Ile Pro Arg Asp Asp Pro Leu Val
                565                 570                 575

Leu Tyr Val Tyr Ala Ala Pro Gln Asp Thr Lys Ala His Thr Ser Ile
            580                 585                 590

Pro Leu Leu Gly Tyr Gln Val Ile Ser Gly Pro Gln Gly Asp Pro Arg
        595                 600                 605

Val Phe Gln Leu Gln Gln Ser Gly Gln Gln Tyr Thr Phe Lys Ala Glu

610                 615                 620

Ser Val Glu Leu Gln Gly Arg Trp Val Thr Ala Ile Lys Arg Ala Ala
625                 630                 635                 640

Ser Gly Arg Thr Pro Glu Gly Pro Asp Glu Glu Asp Val Ser Asp
                645                 650                 655

<210>  27
<211>  461
<212>  PRT
<213>  FGD2 mouse wildtype isoform 2

<400>  27

Met Tyr Ser Glu Tyr Val Lys Asn Phe Glu Arg Ala Ala Glu Leu Leu
1               5                   10                  15

Ala Thr Trp Met Asp Lys Ser Gln Pro Phe Gln Glu Val Val Thr Arg
            20                  25                  30

Ile Gln Cys Ser Glu Ala Ser Ser Ser Leu Thr Leu Gln His His Met
        35                  40                  45

Leu Glu Pro Val Gln Arg Ile Pro Arg Tyr Glu Leu Leu Leu Lys Glu
    50                  55                  60

Tyr Val Gln Lys Leu Pro Ala Gln Ala Pro Asp Leu Glu Asp Ala Gln
65                  70                  75                  80

Arg Ala Leu Asp Met Ile Phe Ser Ala Ala Gln His Ser Asn Ala Ala
                85                  90                  95

Ile Ala Glu Met Glu Arg Leu Gln Gly Leu Trp Asp Val Tyr Gln Arg
            100                 105                 110

Leu Gly Leu Glu Asp Asp Ile Val Asp Pro Ser Asn Thr Leu Leu Arg
        115                 120                 125

Glu Gly Pro Val Leu Lys Ile Ser Phe Arg Arg Ser Asp Pro Met Glu
    130                 135                 140

Arg Tyr Leu Val Leu Phe Asn Asn Met Leu Leu Tyr Cys Val Pro Arg
145                 150                 155                 160

Val Leu Gln Val Gly Ala Gln Phe Gln Val Arg Thr Arg Ile Asp Val
                165                 170                 175

Ala Gly Met Lys Val Arg Glu Leu Thr Asp Ala Glu Phe Pro His Ser
            180                 185                 190

Phe Leu Val Ser Gly Lys Gln Arg Thr Leu Glu Leu Gln Ala Arg Ser
        195                 200                 205

Arg Asp Glu Met Val Ser Trp Met Gln Ala Cys Gln Ala Ala Ile Asp
    210                 215                 220

Gln Val Glu Lys Arg Ser Glu Thr Phe Lys Ala Ala Val Gln Gly Pro
225                 230                 235                 240

Gln Gly Asp Thr Gln Glu Pro Lys Pro Gln Val Glu Glu Leu Gly Leu
                245                 250                 255

Arg Ala Pro Gln Trp Val Arg Asp Lys Met Val Thr Met Cys Met Arg
            260                 265                 270

Cys Gln Glu Pro Phe Asn Ala Leu Thr Arg Arg Arg His His Cys Arg
        275                 280                 285

Ala Cys Gly Tyr Val Val Cys Ala Lys Cys Ser Asp Tyr Arg Ala Glu
    290                 295                 300

Leu Lys Tyr Asp Ser Asn Arg Pro Asn Arg Val Cys Leu Thr Cys Tyr
305                 310                 315                 320

Thr Phe Leu Thr Gly Asn Val Leu Pro Gln Gly Lys Glu Asp Lys Arg
                325                 330                 335

Arg Gly Ile Leu Glu Lys Glu Ala Ser Ala Ala Pro Glu Gln Ser Leu
            340                 345                 350

Val Cys Ser Phe Leu Gln Leu Ile Gly Asp Lys Cys Ser Arg Ser Leu
        355                 360                 365

Pro Arg Ser Trp Cys Val Ile Pro Arg Asp Asp Pro Leu Val Leu Tyr
    370                 375                 380

Val Tyr Ala Ala Pro Gln Asp Thr Lys Ala His Thr Ser Ile Pro Leu
385                 390                 395                 400

Leu Gly Tyr Gln Val Ile Ser Gly Pro Gln Gly Asp Pro Arg Val Phe
                405                 410                 415

Gln Leu Gln Gln Ser Gly Gln Gln Tyr Thr Phe Lys Ala Glu Ser Val
            420                 425                 430

Glu Leu Gln Gly Arg Trp Val Thr Ala Ile Lys Arg Ala Ala Ser Gly
        435                 440                 445

Arg Thr Pro Glu Gly Pro Asp Glu Glu Asp Val Ser Asp
    450                 455                 460

<210>  28
<211>  656
<212>  PRT
<213>  FGD2 mouse isoform 1 t6/tw5

<400>  28

Met Glu Arg Ala Cys Glu Lys Gln Asp Ser Val Cys Asn Leu Val Ala
1               5                   10                  15

Val Phe Glu Asn Asn Ser Arg Thr Pro Gly Glu Ala Pro Gly Ser His
            20                  25                  30

Ser Leu Glu Asp Gln Leu His Ser Pro Glu His Gln Leu Ser Leu Ser
        35                  40                  45

Pro Glu Pro Trp Glu Ala Pro Pro Val Lys Glu Ala Leu Lys Ser Glu
    50                  55                  60

Phe Arg Pro Val Ser Arg Thr Tyr Leu Ser Ser Leu Lys Asn Lys Leu
65                  70                  75                  80

Ser Ser Gly Ala Trp Arg Arg Ser Cys Gln Pro Gly Val Ser Pro Gly
                85                  90                  95

Pro Glu Thr Gln Glu Pro Glu Glu Lys Arg Val Val Arg Glu Leu Leu
            100                 105                 110

Glu Thr Glu Gln Ala Tyr Val Ala Arg Leu His Leu Leu Asp Gln Val
        115                 120                 125

Phe Phe Gln Glu Leu Leu Arg Glu Ala Gly Arg Ser Lys Ala Phe Pro
    130                 135                 140

Glu Asp Val Val Lys Leu Ile Phe Ser Asn Ile Ser Ser Ile Tyr Arg
145                 150                 155                 160

Phe His Ala Gln Phe Phe Leu Pro Glu Leu Gln Arg Arg Val Asp Asp
                165                 170                 175

Trp Ala Ala Thr Pro Arg Ile Gly Asp Val Ile Gln Lys Leu Ala Pro
            180                 185                 190

Phe Leu Lys Met Tyr Ser Glu Tyr Val Lys Asn Phe Glu Arg Ala Ala
        195                 200                 205

Glu Leu Leu Ala Thr Trp Met Asp Lys Ser Gln Pro Phe Gln Glu Val
    210                 215                 220

Val Thr Arg Ile Gln Cys Ser Glu Ala Ser Gly Ser Leu Thr Leu Gln
225                 230                 235                 240

His His Met Leu Glu Pro Val Gln Arg Ile Pro Arg Tyr Glu Leu Leu
                245                 250                 255

Leu Lys Glu Tyr Val Gln Lys Leu Pro Ala Gln Ala Pro Asp Leu Glu
            260                 265                 270

Asp Ala Gln Arg Ala Leu Asp Met Ile Phe Ser Ala Ala Gln His Ser
        275                 280                 285

Asn Ala Ala Ile Ala Glu Met Glu Arg Leu Gln Gly Leu Trp Asp Val
    290                 295                 300

Tyr Gln Arg Leu Gly Leu Glu Asp Asp Ile Val Asp Pro Ser Asn Thr
305                 310                 315                 320

Leu Leu Arg Glu Gly Pro Val Leu Lys Ile Ser Phe Arg Arg Ser Asp
                325                 330                 335

Pro Met Glu Arg Tyr Leu Val Leu Phe Asn Asn Met Leu Leu Tyr Cys
            340                 345                 350

Val Pro Arg Val Leu Gln Val Gly Ala Gln Phe Gln Val Arg Thr Arg
        355                 360                 365

Ile Asp Val Ala Gly Met Lys Val Arg Glu Leu Thr Asp Ala Glu Phe
    370                 375                 380

Pro His Ser Phe Leu Val Ser Gly Lys Gln Arg Thr Leu Glu Leu Gln
385                 390                 395                 400

Ala Arg Ser Arg Asp Glu Met Val Ser Trp Met Gln Ala Cys Gln Ala
                405                 410                 415

Ala Ile Asp Gln Val Glu Lys Arg Ser Glu Thr Phe Lys Ala Ala Val
            420                 425                 430

Gln Gly Pro Gln Gly Asp Thr Gln Glu Pro Lys Pro Gln Val Glu Glu
        435                 440                 445

Leu Gly Leu Arg Ala Pro Gln Trp Val Arg Asp Lys Met Val Thr Met
    450                 455                 460

Cys Met Arg Cys Gln Glu Pro Phe Asn Ala Leu Thr Arg Arg Arg His
465                 470                 475                 480

His Cys Arg Ala Cys Gly Tyr Val Val Cys Ala Lys Cys Ser Asp Tyr
                485                 490                 495

Arg Ala Glu Leu Lys Tyr Asp Ser Asn Arg Pro Asn Arg Val Cys Leu
            500                 505                 510

Thr Cys Tyr Thr Phe Leu Thr Gly Asn Val Leu Pro Gln Gly Lys Glu
        515                 520                 525

Asp Lys Arg Arg Gly Ile Leu Glu Lys Glu Ala Ser Ala Ala Pro Glu
    530                 535                 540

Gln Ser Leu Val Cys Ser Phe Leu Gln Leu Ile Gly Asp Lys Cys Ser
545                 550                 555                 560

Arg Ser Leu Pro Arg Ser Trp Cys Val Ile Pro Arg Asp Asp Pro Leu
                565                 570                 575

Val Leu Tyr Val Tyr Ala Ala Pro Gln Asp Thr Lys Ala His Thr Ser
            580                 585                 590

Ile Pro Leu Leu Gly Tyr Gln Val Ile Ser Gly Pro Gln Gly Asp Pro
        595                 600                 605

Arg Val Phe Gln Leu Gln Gln Ser Gly Gln Gln Tyr Thr Phe Lys Ala
    610                 615                 620

Glu Ser Val Glu Leu Gln Gly Arg Trp Val Thr Ala Ile Lys Arg Ala
625                 630                 635                 640

Ala Ser Gly Arg Thr Pro Glu Gly Pro Asp Glu Glu Asp Val Ser Asp
                645                 650                 655

<210>  29
<211>  1714
<212>  PRT
<213>  Tiam2 mouse wildtype

<400>  29

Met Gly Asn Ser Glu Ser Gln Tyr Thr Phe Gln Gly Ser Lys Asn His
1               5                   10                  15

Ser Asn Thr Val Thr Gly Ala Lys Gln Lys Pro Cys Ser Leu Lys Ile
            20                  25                  30

Arg Ser Val His Ala Lys Asp Glu Lys Ser Leu His Gly Trp Thr His
        35                  40                  45

Gly Ser Ser Gly Ala Gly Tyr Lys Ser Arg Ser Leu Ala Arg Ser Cys
    50                  55                  60

Leu Ser His Phe Lys Asn His Gln Pro Tyr Ala Thr Arg Leu Ser Gly
65                  70                  75                  80

Pro Thr Cys Lys Val Ser Lys Gly Thr Thr Tyr Ser Lys His Arg Ala
                85                  90                  95

Asn Thr Pro Gly Asn Asp Phe Gln Gly Asn Ser Gly Ala Phe Leu Pro
            100                 105                 110

Glu Asn Gly Phe His Tyr Val Asp Arg Glu Ser Glu Glu Ser His Ile
        115                 120                 125

Thr Ser Asn Gly His Leu Leu Thr Cys Tyr Gly Arg Lys Glu Ser Leu
    130                 135                 140

Ala Ser Thr Pro Pro Gly Glu Asp His Arg Ser Pro Arg Val Leu Ile
145                 150                 155                 160

Lys Thr Leu Gly Lys Leu Asp Gly Cys Leu Arg Val Glu Phe His Asn
                165                 170                 175

Gly Gly Asn Pro His Lys Gly Thr Ser Glu Asp Pro Ser Gly Pro Val
            180                 185                 190

Arg Leu Leu Arg Tyr Ser Pro Thr Leu Ala Ser Glu Thr Cys Pro Val
        195                 200                 205

Arg Glu Thr Arg Arg His Ser Ala Ala Gly Ser Pro Ser Ser Gln Arg
    210                 215                 220

Pro Ser Pro Thr Asp Ser Arg Leu Arg Ser Ser Lys Gly Ser Ser Leu
225                 230                 235                 240

Ser Ser Glu Ser Ser Trp Tyr Asp Ser Pro Trp Gly Asn Ala Gly Glu
                245                 250                 255

Val Ser Glu Val Glu Gly Ser Phe Leu Ala Pro Ser Thr Pro Asp Pro
            260                 265                 270

Ser Leu Pro Ser Ser Phe Pro Pro Ser Asp Thr Lys Lys Pro Phe Asn
        275                 280                 285

Gln Ser Ser Ser Leu Ser Ser Leu Arg Glu Leu Tyr Lys Asp Pro Asn
    290                 295                 300

Leu Gly Cys Arg Ser Pro Ser Gly Thr Cys Leu Ser Ser Asn Glu Tyr
305                 310                 315                 320

Ile Ser Ser Gln Val Ser Leu Asn Asn Arg Val Ser Phe Ala Ser Asp
                325                 330                 335

Met Asp Val Pro Ser Arg Val Asp His Arg Asp Pro Leu His Tyr Ser
            340                 345                 350

Ser Phe Thr Leu Pro Cys Arg Lys Ser Lys Ala Leu Thr Glu Asp Ala
        355                 360                 365

Ala Lys Lys Asp Thr Leu Lys Ala Arg Met Arg Arg Phe Ser Asp Trp
    370                 375                 380

Thr Gly Ser Leu Ser Arg Lys Lys Arg Lys Leu Gln Glu Pro Arg Ser
385                 390                 395                 400

Met Glu Gly Ser Glu Tyr Phe Asp Ser His Ser Asp Gly Leu Asn Ala
                405                 410                 415

Glu Gly Gln Val Pro Ala Gln Thr Ser Ser Leu Leu Trp Ser Gly Gly
            420                 425                 430

Ser Ala Gln Thr Leu Pro His Arg Ser Glu Ser Thr His Ala Ile Ser
        435                 440                 445

Val Asp Pro Leu Arg Gln Asn Ile Tyr Glu Asn Phe Met Arg Glu Leu
    450                 455                 460

Glu Met Ser Arg Ser Asn Thr Glu His Val Glu Thr Ser Thr Glu Thr
465                 470                 475                 480

Met Glu Ser Ser Ser Glu Ser Val Ser Ser Leu Glu Gln Leu Asp Leu
                485                 490                 495

Leu Phe Glu Lys Glu Gln Gly Val Val Arg Lys Ala Gly Trp Leu Phe
            500                 505                 510

Phe Lys Pro Leu Val Thr Leu Gln Lys Glu Arg Lys Leu Glu Leu Val
        515                 520                 525

Ala Arg Arg Lys Trp Lys Gln Tyr Trp Val Thr Leu Lys Gly Cys Thr
    530                 535                 540

Leu Leu Phe Tyr Glu Thr Tyr Gly Lys Asn Ser Thr Glu Gln Asn Ser
545                 550                 555                 560

Ala Pro Arg Cys Ala Leu Phe Ala Glu Asp Ser Ile Val Gln Ser Val
                565                 570                 575

Pro Glu His Pro Lys Lys Glu His Val Phe Cys Leu Ser Asn Ser Cys
            580                 585                 590

Gly Asp Val Tyr Leu Phe Gln Ala Thr Ser Gln Thr Asp Leu Glu Asn
        595                 600                 605

Trp Val Thr Ala Ile His Ser Cys Ala Ser Leu Phe Ala Lys Lys His
    610                 615                 620

Gly Lys Glu Asp Thr Val Arg Leu Leu Lys Ser Gln Thr Arg Ser Leu
625                 630                 635                 640

Leu Gln Lys Ile Asp Met Asp Ser Lys Met Lys Lys Met Ala Glu Leu
                645                 650                 655

Gln Leu Ser Val Val Ser Asp Pro Lys Asn Arg Lys Ala Ile Glu Asn
            660                 665                 670

Gln Ile Arg Gln Trp Glu Gln Asn Leu Glu Lys Phe His Met Asp Leu
        675                 680                 685

Phe Arg Met Arg Cys Tyr Leu Ala Ser Leu Gln Gly Gly Glu Leu Pro
    690                 695                 700

Asn Pro Lys Ser Leu Leu Ala Ala Thr Ser Arg Pro Ser Lys Leu Ala
705                 710                 715                 720

Leu Gly Arg Leu Gly Val Leu Ser Val Ser Ser Phe His Ala Leu Val
                725                 730                 735

Cys Ser Arg Asp Asp Ser Thr Leu Arg Lys Arg Thr Leu Ser Leu Thr
            740                 745                 750

Gln Arg Gly Lys Ser Lys Lys Gly Ile Phe Ser Ser Leu Lys Gly Leu
        755                 760                 765

Asp Thr Leu Ala Arg Lys Gly Arg Glu Lys Arg Ala Ser Ile Thr Gln
    770                 775                 780

Met Phe Asp Ser Ser His Ser His Gly Phe Leu Gly Thr Gln Leu Pro
785                 790                 795                 800

Gln Lys Ser Thr Asn Ser Asn Lys Ala His Asp Leu His Leu Tyr Gly
                805                 810                 815

Ser Ala Val Asp Ser Ala Leu Arg Asp Ser Met Trp Glu Val Gln Thr
            820                 825                 830

Tyr Val His Phe Gln Asp Asn Glu Gly Val Thr Val Thr Ile Lys Pro
        835                 840                 845

Glu His Arg Val Glu Asp Val Leu Ala Leu Val Cys Lys Met Arg Gln
    850                 855                 860

Leu Glu Pro Thr His Tyr Gly Leu Gln Leu Arg Lys Val Val Asp Lys
865                 870                 875                 880

Ser Val Glu Trp Cys Val Pro Ala Leu Tyr Glu Tyr Met Gln Glu Gln
                885                 890                 895

Val Tyr Asp Glu Ile Glu Val Phe Pro Leu Ser Val Tyr Asp Val Gln
            900                 905                 910

Leu Thr Lys Thr Gly Asp Met Thr Asp Phe Gly Phe Ala Val Thr Val
        915                 920                 925

Gln Val Asp Glu His Gln His Leu Asn Arg Ile Phe Ile Ser Asp Val
    930                 935                 940

Leu Pro Asp Ser Leu Ala Tyr Gly Gly Gly Leu Arg Lys Gly Asn Glu
945                 950                 955                 960

Ile Thr Ser Leu Asn Gly Glu Pro Val Ser Asp Leu Asp Ile Gln Gln
                965                 970                 975

Met Glu Ala Leu Phe Ser Glu Lys Ser Val Gly Leu Thr Leu Val Ala
            980                 985                 990

Arg Pro Val Thr Thr Arg Arg Thr Leu Cys Ala Ser Trp Ser Asp Ser
        995                 1000                1005

Asp Leu Phe Ser Arg Asp Gln Lys Ser Leu Pro Pro Ser Pro Asn
    1010                1015                1020

Gln Ser Gln Leu Leu Glu Glu Phe Leu Asp Asn Phe Arg Lys Thr
    1025                1030                1035

Ala Thr Ser Asp Phe Ser Asn Val Pro Glu Ile Thr Thr Gly Leu
    1040                1045                1050

Lys Arg Ser Gln Thr Glu Gly Thr Leu Asp Gln Val Pro His Arg
    1055                1060                1065

Glu Lys Met Glu Gln Thr Phe Leu Ser Ala Asp Gln Ile Ala Glu
    1070                1075                1080

Leu Cys Arg Asp Leu Asn Asn Thr His Thr Asn Ser Met Glu Ala
    1085                1090                1095

Pro Thr Glu Ser His Asp Pro Pro Pro Arg Pro Leu Ala Arg His
    1100                1105                1110

Leu Ser Asp Ala Asp Arg Leu Arg Lys Val Ile Gln Glu Leu Val
    1115                1120                1125

Asp Thr Glu Lys Ser Tyr Val Lys Asp Leu Ser Cys Leu Phe Glu
    1130                1135                1140

Leu Tyr Leu Glu Pro Leu Gln Asn Glu Thr Phe Leu Thr Gln Asp
    1145                1150                1155

Glu Met Glu Ser Leu Phe Gly Ser Leu Pro Glu Met Leu Glu Phe
    1160                1165                1170

Gln Lys Val Phe Leu Glu Thr Leu Glu Asp Ala Ile Ser Ala Ser
    1175                1180                1185

Ser Asp Phe Ser Val Leu Glu Thr Pro Ser Gln Phe Arg Lys Leu
    1190                1195                1200

Leu Phe Ser Leu Gly Gly Ser Phe Leu Tyr Tyr Ala Asp His Phe
    1205                1210                1215

Lys Leu Tyr Ser Gly Phe Cys Ala Asn His Ile Lys Val Gln Arg
    1220                1225                1230

Val Leu Glu Arg Ala Lys Thr Asp Lys Ala Phe Lys Ala Phe Leu
    1235                1240                1245

Asp Ala Arg Asn Pro Thr Lys Gln His Ser Ser Thr Leu Glu Ser
    1250                1255                1260

Tyr Leu Ile Lys Pro Val Gln Arg Val Leu Lys Tyr Pro Leu Leu
    1265                1270                1275

Leu Lys Glu Leu Val Ser Leu Thr Asp His Glu Ser Glu Glu His
    1280                1285                1290

Tyr His  Leu Thr Glu Ala Leu  Lys Ala Met Glu Lys  Val Ala Ser
    1295                 1300                 1305

His Ile  Asn Glu Met Gln Lys  Ile Tyr Glu Asp Tyr  Gly Met Val
    1310                 1315                 1320

Phe Asp  Gln Leu Val Ala Glu  Gln Ser Gly Thr Glu  Lys Glu Val
    1325                 1330                 1335

Thr Glu  Leu Ser Met Gly Glu  Leu Leu Met His Ser  Thr Val Ser
    1340                 1345                 1350

Trp Leu  Asn Pro Phe Leu Ser  Leu Gly Lys Ala Arg  Lys Asp Ile
    1355                 1360                 1365

Glu Leu  Thr Val Phe Val Phe  Lys Arg Ala Val Ile  Leu Val Tyr
    1370                 1375                 1380

Lys Glu  Asn Cys Lys Leu Lys  Lys Lys Leu Pro Ser  Asn Ser Arg
    1385                 1390                 1395

Pro Ala  His Asn Ser Ala Asp  Leu Asp Pro Phe Lys  Phe Arg Trp
    1400                 1405                 1410

Leu Ile  Pro Ile Ser Ala Leu  Gln Val Arg Leu Gly  Asn Thr Ala
    1415                 1420                 1425

Gly Thr  Glu Asn Asn Ser Thr  Trp Glu Leu Ile His  Thr Lys Ser
    1430                 1435                 1440

Glu Ile  Glu Gly Arg Pro Glu  Thr Ile Phe Gln Leu  Cys Cys Ser
    1445                 1450                 1455

Asp Ser  Glu Asn Lys Thr Ser  Ile Val Lys Val Ile  Arg Ser Ile
    1460                 1465                 1470

Leu Arg  Glu Asn Phe Arg Arg  His Ile Lys Cys Glu  Leu Pro Leu
    1475                 1480                 1485

Glu Lys  Thr Cys Lys Asp Arg  Leu Val Pro Leu Lys  Asn Arg Val
    1490                 1495                 1500

Pro Val  Ser Ala Lys Leu Ala  Ser Ser Arg Ser Leu  Lys Gly Leu
    1505                 1510                 1515

Arg Thr  Ser Ser Ser Ser Glu  Trp Pro Ser Glu Pro  Ser Lys Gly
    1520                 1525                 1530

Asn Ser Leu Asp Ser Asp Glu Cys Ser Leu Ser Ser Gly Thr Gln
    1535                1540                1545

Ser Ser Gly Cys Pro Val Ala Glu Ser Arg Arg Asp Ser Lys Ser
    1550                1555                1560

Thr Glu Leu Glu Lys Asp Ala Gln Glu Gly Leu Ala Glu Phe Pro
    1565                1570                1575

Asp Gly Leu Ile Lys Glu Ser Asp Ile Leu Ser Asp Glu Asp Glu
    1580                1585                1590

Asp Phe His His Pro Leu Lys Gln Gly Ser Pro Thr Lys Asp Ile
    1595                1600                1605

Glu Ile Gln Phe Gln Arg Leu Lys Ile Ser Glu Glu Ser Asp Val
    1610                1615                1620

His Pro Val Gly Gln Gln Pro Leu Thr Glu Ser Gly Glu Gln Pro
    1625                1630                1635

Lys Leu Val Arg Gly His Phe Cys Pro Ile Lys Arg Lys Ala Asn
    1640                1645                1650

Ser Thr Lys Arg Gly Arg Gly Thr Leu Leu Lys Ala Gln Thr Arg
    1655                1660                1665

His Gln Ser Leu Asp Ser His Pro Glu Thr Ala Ser Ile Asp Leu
    1670                1675                1680

Asn Leu Val Leu Glu Arg Glu Phe Ser Val Gln Ser Leu Thr Ser
    1685                1690                1695

Val Val Asn Glu Glu Gly Phe Tyr Glu Thr Gln Ser His Gly Lys
    1700                1705                1710

Ser

<210>  30
<211>  1701
<212>  PRT
<213>  Tiam2 homo sapiens

<400>  30

Met Gly Asn Ser Asp Ser Gln Tyr Thr Leu Gln Gly Ser Lys Asn His
1               5                   10                  15

Ser Asn Thr Ile Thr Gly Ala Lys Gln Ile Pro Cys Ser Leu Lys Ile

            20                  25                  30

Arg Gly Ile His Ala Lys Glu Glu Lys Ser Leu His Gly Trp Gly His
        35                  40                  45

Gly Ser Asn Gly Ala Gly Tyr Lys Ser Arg Ser Leu Ala Arg Ser Cys
    50                  55                  60

Leu Ser His Phe Lys Ser Asn Gln Pro Tyr Ala Ser Arg Leu Gly Gly
65                  70                  75                  80

Pro Thr Cys Lys Val Ser Arg Gly Val Ala Tyr Ser Thr His Arg Thr
                85                  90                  95

Asn Ala Pro Gly Lys Asp Phe Gln Gly Ile Ser Ala Ala Phe Ser Thr
            100                 105                 110

Glu Asn Gly Phe His Ser Val Gly His Glu Leu Ala Asp Asn His Ile
        115                 120                 125

Thr Ser Arg Asp Cys Asn Gly His Leu Leu Asn Cys Tyr Gly Arg Asn
    130                 135                 140

Glu Ser Ile Ala Ser Thr Pro Pro Gly Glu Asp Arg Lys Ser Pro Arg
145                 150                 155                 160

Val Leu Ile Lys Thr Leu Gly Lys Leu Asp Gly Cys Leu Arg Val Glu
                165                 170                 175

Phe His Asn Gly Gly Asn Pro Ser Lys Val Pro Ala Glu Asp Cys Ser
            180                 185                 190

Glu Pro Val Gln Leu Leu Arg Tyr Ser Pro Thr Leu Ala Ser Glu Thr
        195                 200                 205

Ser Pro Val Pro Glu Ala Arg Arg Gly Ser Ser Ala Asp Ser Leu Pro
    210                 215                 220

Ser His Arg Pro Ser Pro Thr Asp Ser Arg Leu Arg Ser Ser Lys Gly
225                 230                 235                 240

Ser Ser Leu Ser Ser Glu Ser Ser Trp Tyr Asp Ser Pro Trp Gly Asn
                245                 250                 255

Ala Gly Glu Leu Ser Glu Ala Glu Gly Ser Phe Leu Ala Pro Gly Met
            260                 265                 270

Pro Asp Pro Ser Leu His Ala Ser Phe Pro Pro Gly Asp Ala Lys Lys

275                 280                 285

Pro Phe Asn Gln Ser Ser Ser Leu Ser Ser Leu Arg Glu Leu Tyr Lys
    290                 295                 300

Asp Ala Asn Leu Gly Ser Leu Ser Pro Ser Gly Ile Arg Leu Ser Asp
305                 310                 315                 320

Glu Tyr Met Gly Thr His Ala Ser Leu Ser Asn Arg Val Ser Phe Ala
                325                 330                 335

Ser Asp Ile Asp Val Pro Ser Arg Val Ala His Gly Asp Pro Ile Gln
            340                 345                 350

Tyr Ser Ser Phe Thr Leu Pro Cys Arg Lys Pro Lys Ala Phe Val Glu
        355                 360                 365

Asp Thr Ala Lys Lys Asp Ser Leu Lys Ala Arg Met Arg Arg Ile Ser
    370                 375                 380

Asp Trp Thr Gly Ser Leu Ser Arg Lys Lys Arg Lys Leu Gln Glu Pro
385                 390                 395                 400

Arg Ser Lys Glu Gly Ser Asp Tyr Phe Asp Ser Arg Ser Asp Gly Leu
                405                 410                 415

Asn Thr Asp Val Gln Gly Ser Ser Gln Ala Ser Ala Phe Leu Trp Ser
            420                 425                 430

Gly Gly Ser Thr Gln Ile Leu Ser Gln Arg Ser Glu Ser Thr His Ala
        435                 440                 445

Ile Gly Ser Asp Pro Leu Arg Gln Asn Ile Tyr Glu Asn Phe Met Arg
    450                 455                 460

Glu Leu Glu Met Ser Arg Thr Asn Thr Glu Asn Ile Glu Thr Ser Thr
465                 470                 475                 480

Glu Thr Ala Glu Ser Ser Ser Glu Ser Leu Ser Ser Leu Glu Gln Leu
                485                 490                 495

Asp Leu Leu Phe Glu Lys Glu Gln Gly Val Val Arg Lys Ala Gly Trp
            500                 505                 510

Leu Phe Phe Lys Pro Leu Val Thr Val Gln Lys Glu Arg Lys Leu Glu
        515                 520                 525

Leu Val Ala Arg Arg Lys Trp Lys Gln Tyr Trp Val Thr Leu Lys Gly

530                 535                 540

Cys Thr Leu Leu Phe Tyr Glu Thr Tyr Gly Lys Asn Ser Met Asp Gln
545                 550                 555                 560

Ser Ser Ala Pro Arg Cys Ala Leu Phe Ala Glu Asp Ser Ile Val Gln
                565                 570                 575

Ser Val Pro Glu His Pro Lys Lys Glu Asn Val Phe Cys Leu Ser Asn
            580                 585                 590

Ser Phe Gly Asp Val Tyr Leu Phe Gln Ala Thr Ser Gln Thr Asp Leu
        595                 600                 605

Glu Asn Trp Val Thr Ala Val His Ser Ala Cys Ala Ser Leu Phe Ala
    610                 615                 620

Lys Lys His Gly Lys Glu Asp Thr Leu Arg Leu Leu Lys Asn Gln Thr
625                 630                 635                 640

Lys Asn Leu Leu Gln Lys Ile Asp Met Asp Ser Lys Met Lys Lys Met
                645                 650                 655

Ala Glu Leu Gln Leu Ser Val Val Ser Asp Pro Lys Asn Arg Lys Ala
            660                 665                 670

Ile Glu Asn Gln Ile Gln Gln Trp Glu Gln Asn Leu Glu Lys Phe His
        675                 680                 685

Met Asp Leu Phe Arg Met Arg Cys Tyr Leu Ala Ser Leu Gln Gly Gly
    690                 695                 700

Glu Leu Pro Asn Pro Lys Ser Leu Leu Ala Ala Ala Ser Arg Pro Ser
705                 710                 715                 720

Lys Leu Ala Leu Gly Arg Leu Gly Ile Leu Ser Val Ser Ser Phe His
                725                 730                 735

Ala Leu Val Cys Ser Arg Asp Asp Ser Ala Leu Arg Lys Arg Thr Leu
            740                 745                 750

Ser Leu Thr Gln Arg Gly Arg Asn Lys Lys Gly Ile Phe Ser Ser Leu
        755                 760                 765

Lys Gly Leu Asp Thr Leu Ala Arg Lys Gly Lys Glu Lys Arg Pro Ser
    770                 775                 780

Ile Thr Gln Val Asp Glu Leu Leu His Ile Tyr Gly Ser Thr Val Asp

785                 790                 795                 800

Gly Val Pro Arg Asp Asn Ala Trp Glu Ile Gln Thr Tyr Val His Phe
                805                 810                 815

Gln Asp Asn His Gly Val Thr Val Gly Ile Lys Pro Glu His Arg Val
            820                 825                 830

Glu Asp Ile Leu Thr Leu Ala Cys Lys Met Arg Gln Leu Glu Pro Ser
        835                 840                 845

His Tyr Gly Leu Gln Leu Arg Lys Leu Val Asp Asp Asn Val Glu Tyr
    850                 855                 860

Cys Ile Pro Ala Pro Tyr Glu Tyr Met Gln Gln Gln Val Tyr Asp Glu
865                 870                 875                 880

Ile Glu Val Phe Pro Leu Asn Val Tyr Asp Val Gln Leu Thr Lys Thr
                885                 890                 895

Gly Ser Val Cys Asp Phe Gly Phe Ala Val Thr Ala Gln Val Asp Glu
            900                 905                 910

Arg Gln His Leu Ser Arg Ile Phe Ile Ser Asp Val Leu Pro Asp Gly
        915                 920                 925

Leu Ala Tyr Gly Glu Gly Leu Arg Lys Gly Asn Glu Ile Met Thr Leu
    930                 935                 940

Asn Gly Glu Ala Val Ser Asp Leu Asp Leu Lys Gln Met Glu Ala Leu
945                 950                 955                 960

Phe Ser Glu Lys Ser Val Gly Leu Thr Leu Ile Ala Arg Pro Pro Asp
                965                 970                 975

Thr Lys Ala Thr Leu Cys Thr Ser Trp Ser Asp Ser Asp Leu Phe Ser
            980                 985                 990

Arg Asp Gln Lys Ser Leu Leu Pro  Pro Pro Asn Gln Ser  Gln Leu Leu
        995                 1000                 1005

Glu Glu  Phe Leu Asp Asn Phe  Lys Lys Asn Thr Ala  Asn Asp Phe
    1010                 1015                 1020

Ser Asn  Val Pro Asp Ile Thr  Thr Gly Leu Lys Arg  Ser Gln Thr
    1025                 1030                 1035

Asp Gly  Thr Leu Asp Gln Val  Ser His Arg Glu Lys  Met Glu Gln

1040                 1045                 1050

Thr Phe Arg Ser Ala Glu Gln Ile Thr Ala Leu Cys Arg Ser Phe
    1055                 1060                 1065

Asn Asp Ser Gln Ala Asn Gly Met Glu Gly Pro Arg Glu Asn Gln
    1070                 1075                 1080

Asp Pro Pro Pro Arg Pro Leu Ala Arg His Leu Ser Asp Ala Asp
    1085                 1090                 1095

Arg Leu Arg Lys Val Ile Gln Glu Leu Val Asp Thr Glu Lys Ser
    1100                 1105                 1110

Tyr Val Lys Asp Leu Ser Cys Leu Phe Glu Leu Tyr Leu Glu Pro
    1115                 1120                 1125

Leu Gln Asn Glu Thr Phe Leu Thr Gln Asp Glu Met Glu Ser Leu
    1130                 1135                 1140

Phe Gly Ser Leu Pro Glu Met Leu Glu Phe Gln Lys Val Phe Leu
    1145                 1150                 1155

Glu Thr Leu Glu Asp Gly Ile Ser Ala Ser Ser Asp Phe Asn Thr
    1160                 1165                 1170

Leu Glu Thr Pro Ser Gln Phe Arg Lys Leu Leu Phe Ser Leu Gly
    1175                 1180                 1185

Gly Ser Phe Leu Tyr Tyr Ala Asp His Phe Lys Leu Tyr Ser Gly
    1190                 1195                 1200

Phe Cys Ala Asn His Ile Lys Val Gln Lys Val Leu Glu Arg Ala
    1205                 1210                 1215

Lys Thr Asp Lys Ala Phe Lys Ala Phe Leu Asp Ala Arg Asn Pro
    1220                 1225                 1230

Thr Lys Gln His Ser Ser Thr Leu Glu Ser Tyr Leu Ile Lys Pro
    1235                 1240                 1245

Val Gln Arg Val Leu Lys Tyr Pro Leu Leu Leu Lys Glu Leu Val
    1250                 1255                 1260

Ser Leu Thr Asp Gln Glu Ser Glu Glu His Tyr His Leu Thr Glu
    1265                 1270                 1275

Ala Leu Lys Ala Met Glu Lys Val Ala Ser His Ile Asn Glu Met

1280                 1285                 1290

Gln Lys Ile Tyr Glu Asp Tyr Gly Thr Val Phe Asp Gln Leu Val
    1295                 1300                 1305

Ala Glu Gln Ser Gly Thr Glu Lys Glu Val Thr Glu Leu Ser Met
    1310                 1315                 1320

Gly Glu Leu Leu Met His Ser Thr Val Ser Trp Leu Asn Pro Phe
    1325                 1330                 1335

Leu Ser Leu Gly Lys Ala Arg Lys Asp Leu Glu Leu Thr Val Phe
    1340                 1345                 1350

Val Phe Lys Arg Ala Val Ile Leu Val Tyr Lys Glu Asn Cys Lys
    1355                 1360                 1365

Leu Lys Lys Lys Leu Pro Ser Asn Ser Arg Pro Ala His Asn Ser
    1370                 1375                 1380

Thr Asp Leu Asp Pro Phe Lys Phe Arg Trp Leu Ile Pro Ile Ser
    1385                 1390                 1395

Ala Leu Gln Val Arg Leu Gly Asn Pro Ala Gly Thr Glu Asn Asn
    1400                 1405                 1410

Ser Ile Trp Glu Leu Ile His Thr Lys Ser Glu Ile Glu Gly Arg
    1415                 1420                 1425

Pro Glu Thr Ile Phe Gln Leu Cys Cys Ser Asp Ser Glu Ser Lys
    1430                 1435                 1440

Thr Asn Ile Val Lys Val Ile Arg Ser Ile Leu Arg Glu Asn Phe
    1445                 1450                 1455

Arg Arg His Ile Lys Cys Glu Leu Pro Leu Glu Lys Thr Cys Lys
    1460                 1465                 1470

Asp Arg Leu Val Pro Leu Lys Asn Arg Val Pro Val Ser Ala Lys
    1475                 1480                 1485

Leu Ala Ser Ser Arg Ser Leu Lys Val Leu Lys Asn Ser Ser Ser
    1490                 1495                 1500

Asn Glu Trp Thr Gly Glu Thr Gly Lys Gly Thr Leu Leu Asp Ser
    1505                 1510                 1515

Asp Glu Gly Ser Leu Ser Ser Gly Thr Gln Ser Ser Gly Cys Pro

1520                    1525                    1530

Thr Ala Glu Gly Arg Gln Asp Ser Lys Ser Thr Ser Pro Gly Lys
    1535                    1540                    1545

Tyr Pro His Pro Gly Leu Ala Asp Phe Ala Asp Asn Leu Ile Lys
    1550                    1555                    1560

Glu Ser Asp Ile Leu Ser Asp Glu Asp Asp Asp His Arg Gln Thr
    1565                    1570                    1575

Val Lys Gln Gly Ser Pro Thr Lys Asp Ile Glu Ile Gln Phe Gln
    1580                    1585                    1590

Arg Leu Arg Ile Ser Glu Asp Pro Asp Val His Pro Glu Ala Glu
    1595                    1600                    1605

Gln Gln Pro Gly Pro Glu Ser Gly Glu Gly Gln Lys Gly Gly Glu
    1610                    1615                    1620

Gln Pro Lys Leu Val Arg Gly His Phe Cys Pro Ile Lys Arg Lys
    1625                    1630                    1635

Ala Asn Ser Thr Lys Arg Asp Arg Gly Thr Leu Leu Lys Ala Gln
    1640                    1645                    1650

Ile Arg His Gln Ser Leu Asp Ser Gln Ser Glu Asn Ala Thr Ile
    1655                    1660                    1665

Asp Leu Asn Ser Val Leu Glu Arg Glu Phe Ser Val Gln Ser Leu
    1670                    1675                    1680

Thr Ser Val Val Ser Glu Glu Cys Phe Tyr Glu Thr Glu Ser His
    1685                    1690                    1695

Gly Lys Ser
    1700